# EUROPEAN PATENT SPECIFICATION

(11) **EP 3 945 091 B1**
(45) Date of publication and mention of the grant of the patent: **03.01.2024**
(21) Application number: 20189036.5
(22) Date of filing: 31.07.2020
(51) Int. Cl.: C07D 295/13, C07D 295/15, C07D 243/08, C07D 401/04, C07D 471/04, C07D 487/04, C07D 487/10, C07D 498/10, A61P 3/10, C07D 471/10, C07D 519/00, A61K 31/495, A61K 31/537

(54) **NOVEL VDAC1 INHIBITORS**
NEUE VDAC1-INHIBITOREN
NOUVEAUX INHIBITEURS DE VDAC1

(43) Date of publication of application: 02.02.2022
(73) Proprietor: Abarceo AB, 20512 Malmö (SE)
(72) Inventor: Brown, William Dalby, 2860 Søborg (DK); Hansen, Kristian Tage Hansen, 3550 Slangerup (DK); Salehi, Sebastian Albert, 226 54 Lund (SE)
(74) Representative: Kjerrumgaard, Lars Bo

(56) References cited:
- WO-A1-2017/046794
- T Galuschka: "Accession No. 1234012946" In: "Enamine Advanced HTS Collection Catalog", 27 May 2020 (2020-05-27), Enamine LLC, XP055754341, * Compound with Registry Number 2419701-18-9, Order Number Z4343738734 *
- Aurora: "Extract from Aurora Building Blocks 1 Catalogue" In: "Extract from Aurora Building Blocks 1 Catalogue", 27 February 2020 (2020-02-27), San Diego, XP055767992, * Compounds with Registry Numbers 1536806-30-0, 1512557-14-0, 1538425-49-8, 1510317-46-0, 1520364-22-0, Order Numbers A10.072.210, A10.072.162, A10.072.146, A10.072.159, A.10.072.147 *

## Description

### Field of the Invention

The present invention relates to compounds of formula I and/or II, which are inhibitors of VDAC1 for treating prediabetes and diabetes, prevent diabetes disease progression (deterioration) and prevent progression of prediabetes to diabetes.

### Background Art

Diabetes is a severe metabolic disease that shortens life expectancy through cardiovascular and chronic kidney diseases, as well as causing stroke, peripheral nerve disease and blindness. Approximately 12% of global health costs are spent on diabetes. Diabetes is due to either the pancreas not producing enough insulin or the cells of the body not responding properly to the insulin produced. There are two main types of diabetes: Type 1 Diabetes Mellitus (T1D) and type 2 diabetes mellitus (T2D). T1D is one of the most common multifactorial endocrine and metabolic diseases in childhood resulting in persistent hyperglycemia, due to autoimmune destruction of the insulin-producing beta cellls in the pancreatic islets. T1D requires life-long insulin therapy. Several studies have reported that residual beta-cells (β cells) remain in the pancreatic islets of T1D patients. In fact, up to 40-50% of beta-cells may escape destruction, but insulin secretion is insufficient to maintain normal blood glucose regulation. In vitro culture of islets from T1D subjects at physiological glucose concentration has shown time-dependent improvement of glucose-stimulated insulin secretion (Lupi R, et al. 2004. Diabetes/metabolism research and reviews, 5 20(3), 246-251; Krogvold L et al. 2015. Diabetes 64:2506-2512; Brissova M. et al. 2018 Cell Rep. 22:2667-2676). These results suggest that the residual beta-cells are dysfunctional in vivo but can resume regulated insulin secretion after extraction from the harmful in vivo environment. Type 2 diabetes (T2D) is a world-wide health problem in the wake of the obesity epidemic. Approximately 400 million people world-wide suffer from T2D and 320 million are estimated to have pre-diabetes (Zimmet P and Alberti KG. 2016. Nature Rev Endo 10:616-622). Normoglycemia is maintained when obesity-associated insulin resistance is compensated by increased insulin secretion. T2D has a strong genetic component and most frequently occurs when members of diabetes-prone families cumulate risk factors, such as obesity, smoking, repeated pregnancies or shift work. It appears that the disease occurs as a sequel of obesity, when the insulin secretion from the pancreatic beta cells cannot adapt to the increased insulin requirements of the organs resistant to the hormone. T2D develops after years of increased fasting blood glucos (FBG) and/or impaired glucose tolerance (IGT), criteria defining prediabetes (Ligthart S, et al. 2016. Lancet Diabetes & 20 endocrinology 4:44-51). Elevated average blood glucose concentrations exert harmful effects on most cells in the body, including endothelial cells and beta cells, so called glucotoxicity (Hansen NW, et al. IUBMB Life, 69:148-161, 2017 ; Weir GC et al. 2004. Diabetes 53, Suppl 3: S16-21). Glucotoxicity contributes to β cell decompensation, a phenomenon which also exists in healthy subjects as glucose infusion attenuates glucose-stimulated insulin secretion (GSIS). The overt T2D with hyperglycemia, increased urine volume and thirst is preceded by a period of prediabetes, often lasting up to 7 years. Prediabetes is defined as elevated fasting blood glucose or impaired lowering of blood glucose after an oral glucose challenge. T2D is reversible early after onset of the disease by life-style interventions aiming at weight loss and implementing physical exercise (Al-Mrabeh et al., 2016. Diabetologia 59:1753-1759). Such interventions show, however, low patient compliance.

In healthy cells, Voltage-dependent anion channel (VDAC), a multi-functional protein, is positioned at the crossroad of metabolic and survival pathways considered a master-gatekeeper regulating the flux of metabolites and ions between mitochondria and the cytosol. Of the three VDAC isoforms, VDAC1 and VDAC2 mediate ADP/ATP exchange and calcium flux in mitochondria, while VDAC3 function is less clear (Shoshan-Barmatz V et al., 2015; Biochim. Biophys. Acta 1848:2547-2575; Shoshan-Barmatz V et al., 2010. Molecular aspects of medicine 31:227-285). VDAC has also been localized to cell compartments other than mitochondria, such as the plasma membrane of various cells, the sarcoplasmic reticulum of skeletal muscles, the endoplasmic reticulum (ER) of rat cerebellum, and in synaptosomes from the Torpedo electric organ (De Pinto V et al., FEBS Lett. 2010. 584:1793-1799). In endothelial cells, VDAC1 is expressed at the plasma membrane even under physiological conditions (Li L et al. J Biol Chem. 2014, 289:32628-38. ). The mechanism underlying plasma membrane localization of VDAC 1 in certain cell types remains to be elucidated. VDAC1 also plays a key role in apoptosis, participating in the release of apoptotic factors from mitochondria and interacting with anti-apoptotic regulators (Shoshan-Barmatz et al., 2015, ibid; Shoshan-Barmatz et al., 2010, ibid). Mitochondrial proteome analysis reveals altered expression of VDAC and multiple other proteins involved in nutrient metabolism, ATP synthesis, cellular defense, glycoprotein folding and mitochondrial DNA stability in rat clonal pancreatic β-cells exposed to high glucose (Ahmed, M. et al., Islets 2010, 2:283-292). Under glucotoxic condition, the expression of VDAC1 was upregulated while that of VDAC2 was downregulated. In islets of human T2D organ donors, VDAC1 mRNA and protein are upregulated, while VDAC2 levels are downregulated (Zhang E. et al. 2019 Cell Metabolism 29: 64-77). VDAC1 was mistargeted to the plasma membrane in the beta cells of T2D organ donours and such translocation was reproduced when islets from non-diabetic organ donors were cultured under glucotoxic conditions. Remarkably, the plasma membrane expression of VDAC1 resulted in loss of ATP, a coupling factor essential for glucose-stimulated insulin secretion (GSIS). Prevention of the loss of ATP by acute exposure of the T2D islets to an antibody against VDAC 1 or a chemical VDAC1 inhibitor, N-(4-chlorophenyl)-4-hydroxy-3-{4-[4-(trifluoromethoxy)phenyl]piperazin-1-yl}butanamide (CAS number: 2086257-77-2), restored GSIS. Inhibition of VDAC1 function in vitro does not affect insulin secretion, nor is glycemia altered in non-diabetic control mice (Zhang et al. Cell Metabolism ibid).

In WO2017046794 is described piperazine and piperidine derivatives interacting with Voltage-Dependent Anion Channel (VDAC), reducing its channel conductance and acting as inhibitors of VDAC oligomerization, associated with apoptosis induction, for the treatment of diseases associated with enhanced apoptosis.

### Summary of the invention

In a first aspect the present invention relates to a compound of general formula I 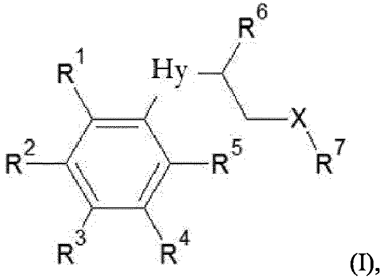 wherein
R¹-R⁵ are independently selected from the group consisting of hydrogen, halogen, CN, NO₂, CF₃, quaternary ammonium, COOH, COO-C₁₋₆ alkyl, OCF₃, SCF₃, SO₃-C₁₋₆ alkyl, provided that at least one of R¹-R⁵ is not hydrogen,
R⁶ is selected from C₁₋₃-R⁸, wherein R⁸ is selected from H, OH, SH, and NH₂,
R⁷ is selected from the group consisting of a) aryl substituted with one or more groups selected from hydrogen, halogen, CN, C₁₋₃ alkyl, NO₂, CONH₂, NHCO-C₁₋₃ alkyl, CF₃, tertiary ammonium, COOH, COO-C₁₋₆ alkyl, OC₁₋₃ alkyl, OCF₃, SCF₃, SO₂-C₁₋₃ alkyl, SO₃-C₁₋₃ alkyl, provided that at least one of said groups is not hydrogen; b) pyridyl optionally substituted with one or more groups selected from halogen, CN, C₁₋₃ alkyl, NO₂, CONH₂, NHCO-C₁₋₃ alkyl, CF₃, tertiary ammonium, COOH, COO-C₁₋₆ alkyl, OC₁₋₃ alkyl, OCF₃, SCF₃, SO₂-C₁₋₃ alkyl, SO₃-C₁₋₃ alkyl; c) pyrrolidinyl optionally substituted with one or more groups selected from halogen, CN, C₁₋₃ alkyl, NO₂, CONH₂, NHCO-C₁₋₃ alkyl, CF₃, tertiary ammonium, COOH, COO-C₁₋₆ alkyl, OC₁₋₃ alkyl, OCF₃, SCF₃, SO₂-C₁₋₃ alkyl, SO₃-C₁₋₃ alkyl; d) pyrazolyl optionally substituted with one or more groups selected from halogen, CN, C₁₋₃ alkyl, NO₂, CONH₂, NHCO-C₁₋₃ alkyl, CF₃, tertiary ammonium, COOH, COO-C₁₋₆ alkyl, OC₁₋₃ alkyl, OCF₃, SCF₃, SO₂-C₁₋₃ alkyl, SO₃-C₁₋₃ alkyl; e) thiazolyl optionally substituted with one or more groups selected from halogen, CN, C₁₋₃ alkyl, NO₂, CONH₂, NHCO-C₁₋₃ alkyl, CF₃, tertiary ammonium, COOH, COO-C₁₋₆ alkyl, OC₁₋₃ alkyl, OCF₃, SCF₃, SO₂-C₁₋₃ alkyl, SO₃-C₁₋₃ alkyl; f) C₅₋₇ cycloalkyl optionally substituted with one or more groups selected from halogen, CN, C₁₋₃ alkyl, NO₂, CONH₂, NHCO-C₁₋₃ alkyl, CF₃, tertiary ammonium, COOH, COO-C₁₋₆ alkyl, OC₁₋₃ alkyl, OCF₃, SCF₃, SO₂-C₁₋₃ alkyl, SO₃-C₁₋₃ alkyl; g) NHCONH₂; h) C₁₋₃ alkyl optionally substituted with a group selected from CONH₂, CON(CH₃)₂, CF₃, and C₂₋₄ alken;
X is -CONH- or -NHCO-, or
X-R⁷ taken together is CONR'R", wherein R' and R" together with the nitrogen forms a monoheterocyclic ring or a biheterocyclic group selected from a) a monoheterocyclic ring having 4-7 ring atoms wherein 1-2 is selected from nitrogens and 0-2 from oxygens and the remaining ring atoms are carbon, and optionally substituted with a group selected from oxo, OH, C₁₋₃ alkyl-CN, or b) a biheterocyclic group having 7-10 ring atoms wherein 1-4 is selected from nitrogens and the remaining ring atoms are carbon, and optionally substituted with a group selected from oxo, OH, C₁₋₃ alkyl-CN;
Hy is a non-aromatic heterocyclic system selected from b) a biheterocyclic group having a first and a second heterocyclic ring which are fused, the biheterocyclic group having from 6 to 12 ring atoms wherein 2-4 ring atoms are N, 0-2 ring atoms is O, 0-2 ring atoms is S, and the remaining ring atoms are carbon, wherein the first heterocyclic ring is attached to the phenyl ring having substituents R¹-R⁵ of formula I, and the second heterocyclic ring is attached to the carbon atom linked to R⁶ and CH₂-X-R⁷ of formula I, c) a spiroheterocyclic group having a first and a second heterocyclic ring which are connected and share one carbon atom, the spiroheterocyclic group having from 6 to 12 ring atoms wherein 2-4 ring atoms are N, 0-2 ring atoms is O, 0-2 ring atoms is S, and the remaining ring atoms are carbon, wherein the first heterocyclic ring is attached to the phenyl ring having substituents R¹-R⁵ of formula I, and the second heterocyclic ring is attached to the carbon atom linked to R⁶ and CH₂-X-R⁷ of formula I, or d) a first and a second monoheterocyclic ring connected by a bond, e.g. a *sp*² or *sp*³ hybridized bond, wherein each monoheterocyclic ring is independently selected from a monoheterocyclic ring having 4-7 ring atoms, wherein 1-3 ring atoms are N, 0-1 ring atoms is O and 0-1 ring atoms is S, and the remaining ring atoms are carbon, wherein the first monoheterocyclic ring is attached to the phenyl ring having substituents R¹-R⁵ of formula I, and the second monoheterocyclic ring is attached to the carbon atom linked to R⁶ and CH₂-X-R⁷ of formula I; disclaiming 3-(4-hydroxy-3-{2-[4-(trifluoromethoxy)phenyl]-6-oxa-2,9-diazaspiro[4.5]decan-9-yl}butanamido)pyrrolidine-1-carboxamide; or
a pharmaceutically acceptable salt or solvate thereof.

In an embodiment R¹ is selected from halogen, CN, NO₂, CF₃, quaternary ammonium, COOH, COO-C₁₋₆ alkyl, OCF₃, SCF₃, SO₃-C₁₋₆ alkyl; and R²-R⁵ are hydrogen.

In anonther embodiment R² is selected from halogen, CN, NO₂, CF₃, quaternary ammonium, COOH, COO-C₁₋₆ alkyl, OCF₃, SCF₃, SO₃-C₁₋₆ alkyl; and R¹, R³-R⁵ are hydrogen.

In a further embodiment R³ is selected from halogen, CN, NO₂, CF₃, quaternary ammonium, COOH, COO-C₁₋₆ alkyl, OCF₃, SCF₃, SO₃-C₁₋₆ alkyl; and R¹, R², R⁴-R⁵ are hydrogen; typically R³ is OCF₃.

In a still further embodiment R⁴ is selected from halogen, CN, NO₂, CF₃, quaternary ammonium, COOH, COO-C₁₋₆ alkyl, OCF₃, SCF₃, SO₃-C₁₋₆ alkyl; and R¹-R³, R⁵ are hydrogen.

In a further embodiment R⁵ is selected from halogen, CN, NO₂, CF₃, quaternary ammonium, COOH, COO-C₁₋₆ alkyl, OCF₃, SCF₃, SO₃-C₁₋₆ alkyl; and R¹-R⁴ are hydrogen.

In a still further embodiment R⁶ is selected from C₁₋₃-R⁸, wherein R⁸ is OH, typically CH₂OH.

In a further embodiment R⁷ is selected from the group consisting of a) a phenyl substituted with one or more groups selected from hydrogen, halogen, CN, C₁₋₃ alkyl, NO₂, CONH₂, NHCO-C₁₋₃ alkyl, CF₃, tertiary ammonium, COOH, COO-C₁₋₆ alkyl, OC₁₋₃ alkyl, OCF₃, SCF₃, SO₂-C₁₋₃ alkyl, SO₃-C₁₋₃ alkyl, provided that at least one of said groups is not hydrogen; typically, phenyl substituted with a group selected from halogen, CF₃, OCH₃, NHCOCH₃, CN, CH₃, and SO₂CH₃, b) a pyridyl optionally substituted with one or more halogen; c) a pyrrolidinyl substituted with one or more NHCO-C₁₋₃ alkyl; d) a pyrazolyl optionally substituted with one or more CONH₂; e) a thiazolyl; f) a cyclohexyl substituted with one or more CN alkyl; g) a NHCONH₂; and h) a C₁₋₂ alkyl substituted with a group selected from CON(CH₃)₂, CF₃, and C₂ alken.

In an alternative embodiment X-R⁷ taken together is CONR'R", wherein R' and R" together with the nitrogen forms a monoheterocyclic ring or a biheterocyclic group selected from a) a monoheterocyclic group having 5 ring atoms wherein 1-2 is selected from nitrogens and the remaining ring atoms are carbon, and optionally substituted with a group selected from oxo, OH, CH₂-CN, or b) a biheterocyclic group having 9-10 ring atoms wherein 1-3 is selected from nitrogens and the remaining ring atoms are carbon.

In a further embodiment X is -CONH-. In another embodiment X is -NHCO-.

In a further embodiment Hy is a non-aromatic cyclic system selected from b) wherein the biheterocyclic group has from 8 to 11 ring atoms wherein 1-2 is N, 0-1 is O and the remaining ring atoms are carbon.

In a still further embodiment Hy is a non-aromatic cyclic system selected from c) wherein the spiroheterocyclic group has from 8 to 11 ring atoms wherein 2-3 ring atoms are N, 0-1 ring atoms is O, and the remaining ring atoms are carbon.

In a further embodiment Hy is a non-aromatic cyclic system selected from d) wherein the first and second monoheterocyclic ring is connected by a bond, and the first monoheterocyclic ring has from 4-6 ring atoms, wherein 1-2 is N and 0-1 is O, and the remaining ring atoms are carbon, and the second monoheterocyclic ring has from 4-6 ring atoms, wherein 1-2 is N and 0-1 is O, and the remaining ring atoms are carbon.

In a still further embodiment, the compound of general formula I is a compound of formula II 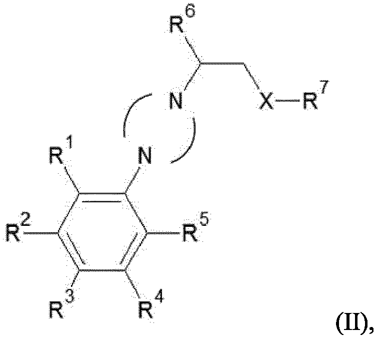 wherein
R¹-R⁵ are independently selected from the group consisting of hydrogen, halogen, CN, NO₂, CF₃, quaternary ammonium, COOH, COO-C₁₋₆ alkyl, OCF₃, SCF₃, SO₃-C₁₋₆ alkyl, provided that at least one of R¹-R⁵ is not hydrogen,
R⁶ is selected from C₁₋₃-R⁸, wherein R⁸ is selected from H, OH, SH, and NH₂,
R⁷ is selected from the group consisting of a) aryl substituted with one or more groups selected from hydrogen, halogen, CN, C₁₋₃ alkyl, NO₂, CONH₂, NHCO-C₁₋₃ alkyl, CF₃, tertiary ammonium, COOH, COO-C₁₋₆ alkyl, OC₁₋₃ alkyl, OCF₃, SCF₃, SO₂-C₁₋₃ alkyl, SO₃-C₁₋₃ alkyl, provided that at least one of said groups is not hydrogen; b) pyridyl optionally substituted with one or more groups selected from halogen, CN, C₁₋₃ alkyl, NO₂, CONH₂, NHCO-C₁₋₃ alkyl, CF₃, tertiary ammonium, COOH, COO-C₁₋₆ alkyl, OC₁₋₃ alkyl, OCF₃, SCF₃, SO₂-C₁₋₃ alkyl, SO₃-C₁₋₃ alkyl; c) pyrrolidinyl optionally substituted with one or more groups selected from halogen, CN, C₁₋₃ alkyl, NO₂, CONH₂, NHCO-C₁₋₃ alkyl, CF₃, tertiary ammonium, COOH, COO-C₁₋₆ alkyl, OC₁₋₃ alkyl, OCF₃, SCF₃, SO₂-C₁₋₃ alkyl, SO₃-C₁₋₃ alkyl; d) pyrazolyl optionally substituted with one or more groups selected from halogen, CN, C₁₋₃ alkyl, NO₂, CONH₂, NHCO-C₁₋₃ alkyl, CF₃, tertiary ammonium, COOH, COO-C₁₋₆ alkyl, OC₁₋₃ alkyl, OCF₃, SCF₃, SO₂-C₁₋₃ alkyl, SO₃-C₁₋₃ alkyl; e) thiazolyl optionally substituted with one or more groups selected from halogen, CN, C₁₋₃ alkyl, NO₂, CONH₂, NHCO-C₁₋₃ alkyl, CF₃, tertiary ammonium, COOH, COO-C₁₋₆ alkyl, OC₁₋₃ alkyl, OCF₃, SCF₃, SO₂-C₁₋₃ alkyl, SO₃-C₁₋₃ alkyl; f) C₅₋₇ cycloalkyl optionally substituted with one or more groups selected from halogen, CN, C₁₋₃ alkyl, NO₂, CONH₂, NHCO-C₁₋₃ alkyl, CF₃, tertiary ammonium, COOH, COO-C₁₋₆ alkyl, OC₁₋₃ alkyl, OCF₃, SCF₃, SO₂-C₁₋₃ alkyl, SO₃-C₁₋₃ alkyl; g) NHCONH₂; h) C₁₋₃ alkyl optionally substituted with a group selected from CONH₂, CON(CH₃)₂, CF₃, and C₂₋₄ alken;
X is -CONH- or -NHCO-, or
X-R⁷ taken together is CONR'R", wherein R' and R" together with the nitrogen forms a monoheterocyclic ring or a biheterocyclic group selected from a) a monoheterocyclic ring having 4-7 ring atoms wherein 1-2 is selected from nitrogens and 0-2 fromoxygens and the remaining ring atoms are carbon, and optionally substituted with a group selected from oxo, OH, C₁₋₃ alkyl-CN, or b) a biheterocyclic group having 7-10 ring atoms wherein 1-4 is selected from nitrogens and the remaining ring atoms are carbon, and optionally substituted with a group selected from oxo, OH, C₁₋₃ alkyl-CN; 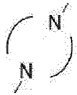
is a non-aromatic heterocyclic system having a first and a second nitrogen as attachment point selected from b) a biheterocyclic group having a first and a second heterocyclic ring which are fused, the biheterocyclic group having from 6 to 12 ring atoms wherein 2-4 ring atoms are N, 0-2 ring atoms is O, 0-2 ring atoms is S, and the remaining ring atoms are carbon, wherein the first nitrogen of the first heterocyclic ring is attached to the phenyl ring having substituents R¹-R⁵ of formula I and the second nitrogen of the second heterocyclic ring is attached to the carbon atom linked to R⁶ and CH₂-X-R⁷ of formula I, c) a spiroheterocyclic group having a first and a second heterocyclic ring which are connected and share one carbon atom, the spiroheterocyclic group having from 6 to 12 ring atoms wherein 2-4 ring atoms are N, 0-2 ring atoms is O, 0-2 ring atoms is S, and the remaining ring atoms are carbon, wherein the first nitrogen of the first heterocyclic ring is attached to the phenyl ring having substituents R¹-R⁵ of formula I and the second nitrogen of the second heterocyclic ring is attached to the carbon atom linked to R⁶ and CH₂-X-R⁷ of formula I, or d) a first and a second monoheterocyclic ring connected by a bond, wherein each monoheterocyclic ring is independently selected from a monoheterocyclic ring having 4-7 ring atoms, wherein 1-3 ring atoms are N, 0-1 ring atoms is O and 0-1 ring atoms is S, and the remaining ring atoms are carbon, wherein the first nitrogen of the first monoheterocyclic ring is attached to the phenyl ring having substituents R¹-R⁵ of formula I, and the second nitrogen of the second monoheterocyclic ring is attached to the carbon atom linked to R⁶ and CH₂-X-R⁷ of formula I; or
a pharmaceutically acceptable salt or solvate thereof.

In a further embodiment the compound of formula I is selected from any one of
*N*-(4-Chlorophenyl)-4-hydroxy-3-{5-[4-(trifluoromethoxy)phenyl]-1H,2H,3H,4H,SH,6H-pyrrolo[3,4-c]pyrrol-2-yl}butanamide,
*N*-(4-Chlorophenyl)-4-hydroxy-3-{2-[4-(trifluoromethoxy)phenyl]-6-oxa-2,9-diazaspiro[4.5]decan-9-yl}butanamide,
*N*-(4-Chlorophenyl)-4-hydroxy-3-(3-{1-[4-(trifluoromethoxy)phenyl]azetidin-3-yl}piperidin-1-yl)butanamide,
*N*-(4-Chlorophenyl)-4-hydroxy-3-{6-[4-(trifluoromethoxy)phenyl]-2,6-diazaspiro [3.4]octan-2-yl}butanamide,
*N*-(4-Chlorophenyl)-4-hydroxy-3-{1-[4-(trifluoromethoxy)phenyl]-octahydro-1H-pyrrolo[3,2-b]pyridin-4-yl}butanamide,
*N*-(4-Chlorophenyl)-4-hydroxy-3-{5-[4-(trifluoromethoxy)phenyl]-octahydropyrrolo[3,4-c]pyrrol-2-yl}butanamide,
*N*-(4-Chlorophenyl)-4-hydroxy-3-{8-[4-(trifluoromethoxy)phenyl]-2,8-diazaspiro[5.5]undecan-2-yl}butanamide,
*N*-[(Dimethylcarbamoyl)methyl]-4-hydroxy-3 - {2- [4-(trifluoromethoxy)phenyl] -6-oxa-2,9-diazaspiro[4.5]decan-9-yl}butanamide,
*N*-[2-(Dimethylcarbamoyl)ethyl] -4-hydroxy-3 - {2- [4-(trifluoromethoxy)phenyl]-6-oxa-2,9-diazaspiro[4.5]decan-9-yl}butanamide,
5-(4-Hydroxy-3- {2-[4-(trifluoromethoxy)phenyl]-6-oxa-2,9-diazaspiro[4.5]decan-9-yl}butanamido)-1H-pyrazole-3-carboxamide,
4-Hydroxy-*N*-(2,2,2-trifluoroethyl)-3-{2-[4-(trifluoromethoxy)phenyl]-6-oxa-2,9-diazaspiro[4.5]decan-9-yl}butanamide,
4-Hydroxy-3 - {2-[4-(trifluoromethoxy)phenyl]-6-oxa-2,9-diazaspiro[4.5]decan-9-yl} - N-[4-(trifluoromethyl)phenyl]butanamide,
*N*-(5-Chloropyridin-2-yl)-4-hydroxy-3-{2-[4-(trifluoromethoxy)phenyl]-6-oxa-2,9-diazaspiro[4.5]decan-9-yl}butanamide,
4-Hydroxy-*N*-(3-methoxyphenyl)-3-{2-[4-(trifluoromethoxy)phenyl]-6-oxa-2,9-diazaspiro[4.5]decan-9-yl}butanamide,
*N*-(4-Acetamidophenyl)-4-hydroxy-3-{2-[4-(trifluoromethoxy)phenyl]-6-oxa-2,9-diazaspiro[4.5]decan-9-yl}butanamide,
*N*-(3-Acetamidophenyl)-4-hydroxy-3-{2-[4-(trifluoromethoxy)phenyl]-6-oxa-2,9-diazaspiro[4.5]decan-9-yl}butanamide,
*N*-(4-Cyanophenyl)-4-hydroxy-3-{2-[4-(trifluoromethoxy)phenyl]-6-oxa-2,9-diazaspiro[4.5]decan-9-yl}butanamide,
*N*-(2-Chlorophenyl)-4-hydroxy-3-{2-[4-(trifluoromethoxy)phenyl]-6-oxa-2,9-diazaspiro[4.5]decan-9-yl}butanamide,
*N*-(3-Chlorophenyl)-4-hydroxy-3-{2-[4-(trifluoromethoxy)phenyl]-6-oxa-2,9-diazaspiro[4.5]decan-9-yl}butanamide,
*N*-(4-Fluorophenyl)-4-hydroxy-3-{2-[4-(trifluoromethoxy)phenyl]-6-oxa-2,9-diazaspiro[4.5]decan-9-yl}butanamide,
1-(4-Hydroxy-3- f 2-[4-(trifluoromethoxy)phenyl]-6-oxa-2,9-diazaspiro[4.5]decan-9-yl}butanoyl)imidazolidin-4-one,
4-Hydroxy-*N*-(prop-2-en-1-yl)-3-{2-[4-(trifluoromethoxy)phenyl]-6-oxa-2,9-diazaspiro[4.5]decan-9-yl}butanamide,
4-Hydroxy-1-(pyrazolidin-1-yl)-3- {2-[4-(trifluoromethoxy)phenyl] -6-oxa-2,9-diazaspiro[4.5]decan-9-yl}butan-1-one,
4-Hydroxy-1-(3-hydroxypyrrolidin-1-yl)-3-{2-[4-(trifluoromethoxy)phenyl]-6-oxa-2,9-diazaspiro[4.5]decan-9-yl}butan-1-one,
2-[1-(4-Hydroxy-3-{5-[4-(trifluoromethoxy)phenyl]-1H,2H,3H,4H,5H,6H-pyrrolo[3,4-c]pyrrol-2-yl}butanoyl)pyrrolidin-3-yl]acetonitrile,
*N*-(3-chlorophenyl)-4-hydroxy-3-{5-[4-(trifluoromethoxy)phenyl]- 1H,2H,3H,4H,5H,6H-pyrrolo[3,4-c]pyrrol-2-yl}butanamide,
4-Hydroxy-*N*-(pyridin-3-yl)-3-{5-[4-(trifluoromethoxy)phenyl]-1H,2H,3H,4H,5H,6H-pyrrolo[3,4-c]pyrrol-2-yl}butanamide,
4-Hydroxy-*N*-(1,2-thiazol-5-yl)-3-{5-[4-(trifluoromethoxy)phenyl]-1H,2H,3H,4H,5H,6H-pyrrolo[3,4-c]pyrrol-2-yl}butanamide,
*N*-[(Dimethylcarbamoyl)methyl] -4-hydroxy-3 -{5- [4-(trifluoromethoxy)phenyl] - 1H,2H,3H,4H,5H,6H-pyrrolo[3,4-c]pyrrol-2-yl}butanamide,
7V-(2-Chlorophenyl)-4-hydroxy-3-{5-[4-(trifluoromethoxy)phenyl]-1H,2H,3H,4H,5H,6H-pyrrolo[3,4-c]pyrrol-2-yl}butanamide,
4-Hydroxy-*N*-(1H-pyrazol-1-yl)-3- {2- [4-(trifluoromethoxy)phenyl] -6-oxa-2,9-diazaspiro[4.5]decan-9-yl}butanamide,
*N*-(4-Cyanophenyl)-4-hydroxy-3-{5-[4-(trifluoromethoxy)phenyl]- 1H,2H,3H,4H,5H,6H-pyrrolo[3,4-c]pyrrol-2-yl}butanamide,
4-Hydroxy-1-{1H,4H,5H,6H,7H-pyrazolo[4,3-c]pyridin-5-yl}-3-{5-[4-(trifluoromethoxy)phenyl]-1H,2H,3H,4H,5H,6H-pyrrolo[3,4-c]pyrrol-2-yl}butan-1-one,
4-Hydroxy-*N*-[(1r,4r)-4-cyanocyclohexyl]-3-{2-[4-(trifluoromethoxy)phenyl]-6-oxa-2,9-diazaspiro[4.5]decan-9-yl}butanamide,
*N*-(Carbamoylamino)-4-hydroxy-3-{2-[4-(trifluoromethoxy)phenyl]-6-oxa-2,9-diazaspiro[4.5]decan-9-yl}butanamide,
4-Hydroxy-*N*-(pyridin-2-yl)-3-{2-[4-(trifluoromethoxy)phenyl]-6-oxa-2,9-diazaspiro[4.5]decan-9-yl}butanamide,
4-Hydroxy-*N*-(pyridin-3-yl)-3-{2-[4-(trifluoromethoxy)phenyl]-6-oxa-2,9-diazaspiro[4.5]decan-9-yl}butanamide,
4-Hydroxy-3-{5-[4-(trifluoromethoxy)phenyl]-1H,2H,3H,4H,5H,6H-pyrrolo[3,4-c]pyrrol-2-yl}-*N*-(2,4,6-trimethylphenyl)butanamide,
4-Hydroxy-N (4-methanesulfonylphenyl)-3- f 2-[4-(trifluoromethoxy)phenyl]-6-oxa-2,9-diazaspiro[4.5]decan-9-yl}butanamide,
4-Hydroxy-1-{1H,4H,5H,6H,7H-pyrrolo[3,2-c]pyridin-5-yl}-3-{5-[4-(trifluoromethoxy)phenyl]-1H,2H,3H,4H,5H,6H-pyrrolo[3,4-c]pyrrol-2-yl}butan-1-one; or a pharmaceutically acceptable salt or solvate thereof. Each of the above listed compounds or a pharmaceutically acceptable salt or solvate thereof are individual embodiments and may be subject to one or more independent claims.

The compounds presented herein are intended to include all diastereomeric, enantiomeric, atropisomers, and epimeric forms as well as the appropriate mixtures thereof.

In a second aspect the present invention relates to a compound of any one of the above aspect or embodiments for use in a method for treating diabetes or pre-diabetes, in a subject in need thereof. Typically, the diabetes is type 2.

To be sufficiently capable of treating diabetes or pre-diabetes in a human subject the compound of formula I or II should have a high affinity to VDAC1 and inhibit VDAC1 function. In this respect, the specific binding to VDAC1 is a Kd value of less than 15 µM (wherein µM means micromolar), such as less than 10 µM such as less than 5 µM, and in a preferred embodiment less than 1 µM, as measured in a Microscale Thermophoresis (MST) binding assay, typically in the MST assay as described herein.

Further objects and advantages of the present invention will appear from the following description, and claims.

### Brief description of figures

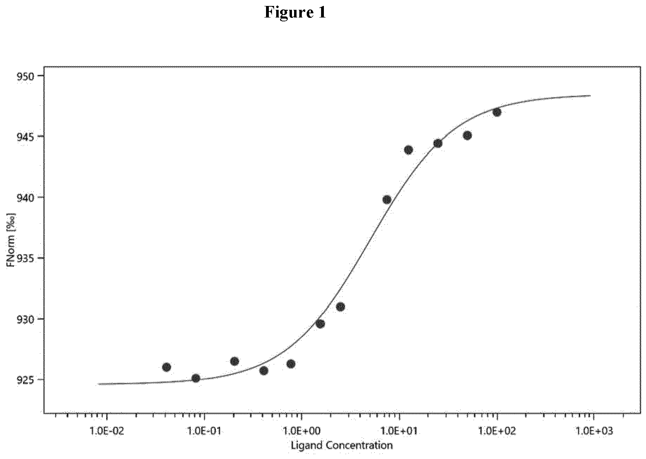

Figure 1 shows an MST plot of the compound of example 4.

### Description of the invention

The present invention relates to the use of compound of formula I binding to the Voltage-Dependent Anion Channel Type 1 (VDAC1) protein for the treatment of diabetes or pre-diabetes.

Compounds binding VDAC1 can reduce the VDAC1 channel conductance and inhibits VDAC1-mediated metabolite transport, particularly inhibiting VDAC1 translocated to the plasma membrane of pancreatic β-cells.

In a first aspect the present invention relates to a compound of general formula I 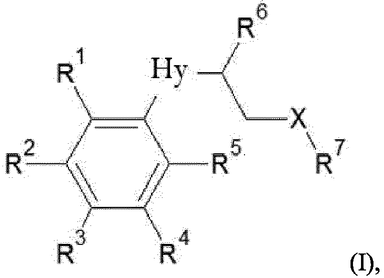 wherein
R¹-R⁷, Hy and X are as defined in claim 1.

In a preferred embodiment R³ is selected from halogen, CN, NO₂, CF₃, quaternary ammonium, COOH, COO-C₁₋₆ alkyl, OCF₃, SCF₃, SO₃-C₁₋₆ alkyl; and R¹, R², R⁴-R⁵ are hydrogen. In a further preferred embodiment R¹, R², R⁴-R⁵ are hydrogen and R³ is OCF₃.

In a further embodiment R⁶ is selected from C₁₋₃-R⁸, wherein R⁸ is OH. In a preferred embodiment R⁶ is selected from C₁₋₂-R⁸, wherein R⁸ is OH. Typically, R⁶ is CH₂CH₂OH or CH₂OH.

In a further embodiment R⁷ is a phenyl substituted with one, two or three groups selected from halogen, CN, C₁₋₃ alkyl, NO₂, CONH₂, NHCO-C₁₋₃ alkyl, CF₃, tertiary ammonium, COOH, COO-C₁₋₆ alkyl, OC₁₋₃ alkyl, OCF₃, SCF₃, SO₂-C₁₋₃ alkyl, SO₃-C₁₋₃ alkyl, and the remaining substituents are hydrogen. Typically, R⁷ is phenyl substituted with one or two groups, such as one group, selected from halogen, CF₃, OCH₃, NHCOCH₃, CN, CH₃, and SO₂CH₃.

In a still further embodiment R⁷ is a pyridyl. In another embodiment R⁷ is a pyridyl substituted with one or more halogen, such as phenyl substituted with one halogen, e.g. one Cl.

In a further embodiment R⁷ is a pyrrolidinyl substituted with one or more CONH₂. In a typical embodiment R⁷ is a pyrrolidinyl substituted with one CONH₂.

In a further embodiment R⁷ is a pyrazolyl. In a further embodiment R⁷ is a pyrazolyl substituted with one or two CONH₂, such as one CONH₂.

In a further embodiment R⁷ is a thiazolyl.

In a further embodiment R⁷ is a cyclohexyl substituted with one or two CN, such as one CN.

In a further embodiment R⁷ is a NHCONH₂.

In a further embodiment R⁷ is a C₁₋₂ alkyl substituted with one group selected from CON(CH₃)₂, CF₃, and C₂ alken. In a further embodiment R⁷ is a CH₂ substituted with one group selected from CON(CH₃)₂, CF₃, and C₂ alken. In another embodiment R⁷ is a CH₂CH₂ substituted with one group selected from CON(CH₃)₂.

In one alternative embodiment X-R⁷ taken together is CONR'R", wherein R' and R" together with the nitrogen forms a monoheterocyclic group having 5 ring atoms wherein 1-2 is selected from nitrogens and the remaining ring atoms are carbon, and optionally substituted with a group selected from oxo, OH, CH₂-CN. In a further embodiment R' and R" together with the nitrogen forms a monoheterocyclic group having 5 ring atoms wherein 1-2 is selected from nitrogens and the remaining ring atoms are carbon, such as imidazolidinyl, pyrazolidinyl, pyrrolidinyl. In a still further embodiment R' and R" together with the nitrogen forms a monoheterocyclic group having 5 ring atoms wherein 1-2 is selected from nitrogens and the remaining ring atoms are carbon, substituted with one or two groups, such as one group, selected from oxo, OH, CH₂-CN, typically imidazolidinyl substituted with one or two groups, such as one group, selected from oxo, or pyrrolidinyl substituted with one or two groups, such as one group, selected from OH and CH₂-CN,

In another alternative embodiment X-R⁷ taken together is CONR'R", wherein R' and R" together with the nitrogen forms a biheterocyclic group having 9 ring atoms wherein 1-3 is selected from nitrogens and the remaining ring atoms are carbon. In a still further embodiment R' and R" together with the nitrogen forms a biheterocyclic group having 9 ring atoms wherein 3 are selected from nitrogens and the remaining ring atoms are carbon; typically the biheterocyclic group is a 1H,4H,5H,6H,7H-pyrazolo[4,3-c]pyridinyl, such as a 1H,4H,5H,6H,7H-pyrazolo[4,3-c]pyridine-5-yl.

In a further embodiment X is -CONH-. In another embodiment X is -NHCO-.

In a further embodiment Hy is a non-aromatic cyclic system selected from b) wherein the biheterocyclic group has 8 ring atoms wherein 2 is N and the remaining ring atoms are carbon, such as 1H,2H,3H,4H,5H,6H-pyrrolo[3,4-c]pyrrol or octahydropyrrolo[3,4-c]pyrrol.

In a still further embodiment Hy is a non-aromatic cyclic system selected from b) wherein the biheterocyclic group has 9 ring atoms wherein 2 is N and the remaining ring atoms are carbon, such as octahydro-1H-pyrrolo[3,2-b]pyridinyl.

In a further embodiment Hy is a non-aromatic cyclic system selected from c) wherein the spiroheterocyclic group has 8 ring atoms wherein 2 ring atoms are N and the remaining ring atoms are carbon, such as 2,6-diazaspiro[3.4]octanyl.

In a still further embodiment Hy is a non-aromatic cyclic system selected from c) wherein the spiroheterocyclic group has 10 ring atoms wherein 2 ring atoms are N and 1 ring atom is O and the remaining ring atoms are carbon, such as 6-oxa-2,9-diazaspiro[4.5]decanyl.

In a still further embodiment Hy is a non-aromatic cyclic system selected from c) wherein the spiroheterocyclic group has 11 ring atoms wherein 2 ring atoms are N and the remaining ring atoms are carbon, such as 2,8-diazaspiro[5.5]undecanyl.

In a further embodiment Hy is a non-aromatic cyclic system selected from d) wherein the first and second monoheterocyclic ring is connected by a bond, and the first monoheterocyclic ring has 4 ring atoms, wherein 1 is N and the remaining ring atoms are carbon, and the second monoheterocyclic ring has 6 ring atoms, wherein 1 is N and the remaining ring atoms are carbon, such as azetidin-3-yl-piperidinyl.

In a still further embodiment the compound of general formula I is a compound of formula II 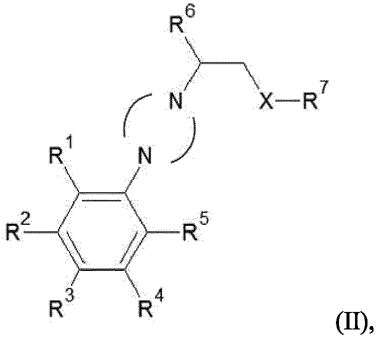 wherein
R¹-R⁷ and X are are as defined in claim 1. All embodiments described above in connection with ifrst aspect relating to the compound of formula I are also embodiments of the compound of formula II.

In formula II the 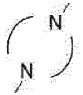 is a particular embodiment of Hy as defined above and in claim 1, wherein there are two nitrogen atoms which are termed a first nitrogen and a second nitrogen. The first nitrogen is defined as the nitrogen attached to the phenyl ring having substituents R¹-R⁵ of formula II and the second nitrogen is defined as the nitrogen attached to the carbon atom linked to R⁶ and CH₂-X-R⁷ of formula II. Consequently, in this particular embodiment of Hy one nitrogen of either the biheterocyclic group, the spiroheterocyclic group or the first and second monoheterocyclic ring connected by a linker, such as a bond, is attached to the phenyl ring having substituents R¹-R⁵ of formula II and the second nitrogen of either the monoheterocyclic ring, the biheterocyclic group, the spiroheterocyclic group or the first and second monoheterocyclic ring connected by a bond, is attached to the carbon atom linked to R⁶ and CH₂-X-R⁷ of formula II, and the first and second nitrogen cannot be the same obviously, however, the terminology first and second nitrogen should not be construed to mean that the above mentioned heterocyclic rings cannot contain additional nitrogens (N), oxygens (O), and/or sulphurs (S).

The following table shows the IUPAC name of each compound and the corresponding chemical structure:

| Name | Structure |
|---|---|
| ***N*-(4-chlorophenyl)-4-hydroxy-3-{5-[4-(trifluoromethoxy)phenyl]-1H,2H,3H,4H,5H,6H-pyrrolo[3,4-c]pyrrol-2-yl}butanamide** | 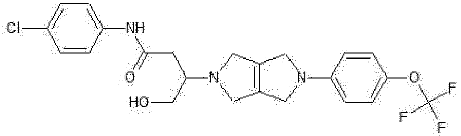 |
| **N-(4-chlorophenyl)-4-hydroxy-3-{2-[4-(trifluoromethoxy)phenyl]-6-oxa-2,9-diazaspiro[4.5]decan-9-yl}butanamide** | 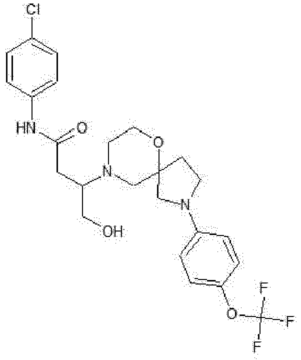 |
| ***N*-(4-chlorophenyl)-4-hydroxy-3-(3-{1-[4-(trifluoromethoxy)phenyl]azetidi n-3-yl}piperidin-1-yl)butanamide** | 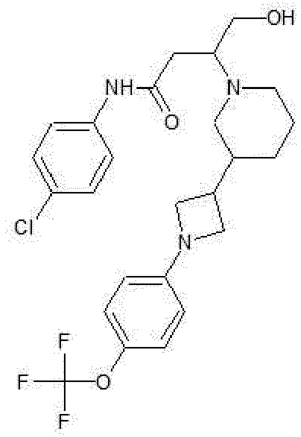 |
| ***N*-(4-chlorophenyl)-4-hydroxy-3-{6-[4-(trifluoromethoxy)phenyl]-2,6-diazaspiro[3.4]octan-2-yl}butanamide** | 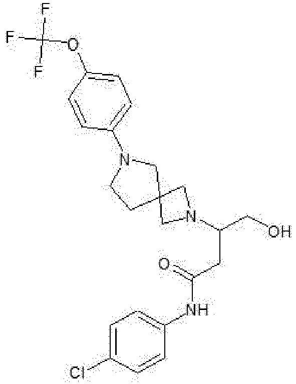 |
| ***N*-(4-chlorophenyl)-4-hydroxy-3-{1-[4-(trifluoromethoxy)phenyl]-octahydro-1H-pyrrolo [3,2-b]pyridin-4-yl}butanamide** | 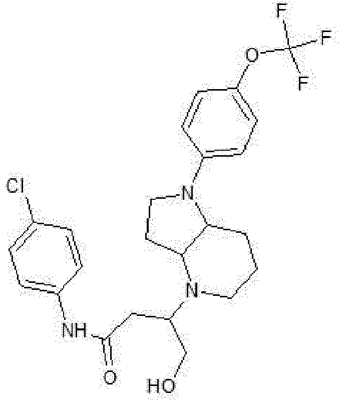 |
| ***N*-(4-chlorophenyl)-4-hydroxy-3-{5-[4-(trifluoromethoxy)phenyl]-octahydropyrrolo[3,4-c]pyrrol-2-yl}butanamide** | 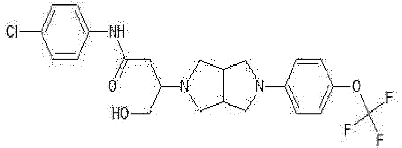 |
| ***N*-(4-chlorophenyl)-4-hydroxy-3-{8-[4-(trifluoromethoxy)phenyl]-2,8-diazaspiro[5.5]undecan-2-yl}butanamide** | 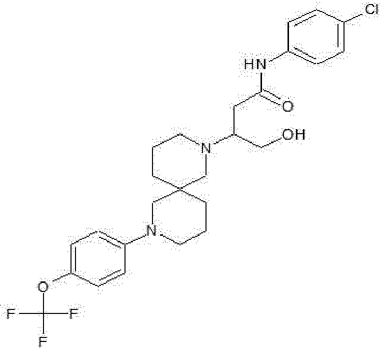 |
| **N-[(dimethylcarbamoyl)methyl]-4-hydroxy-3-{2-[4-(trifluoromethoxy)phenyl]-6-oxa-2,9-diazaspiro[4.5]decan-9-yl}butanamide** | 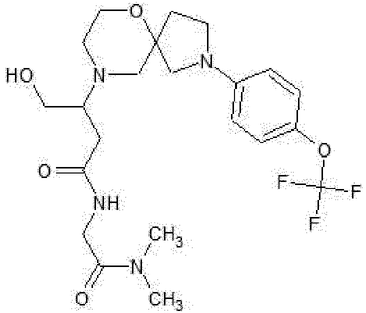 |
| **3-(4-hydroxy-3-{2-[4-(trifluoromethoxy)phenyl]-6-oxa-2,9-diazaspiro[4.5]decan-9-yl}butanamido)pyrrolidine-1-carboxamide** | 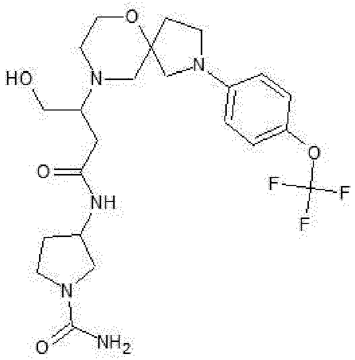 |
| ***N*-[2-(dimethylcarbamoyl)ethyl]-4-hydroxy-3-{2-[4-(trifluoromethoxy)phenyl]-6-oxa-2,9-diazaspiro[4.5]decan-9-yl}butanamide** | 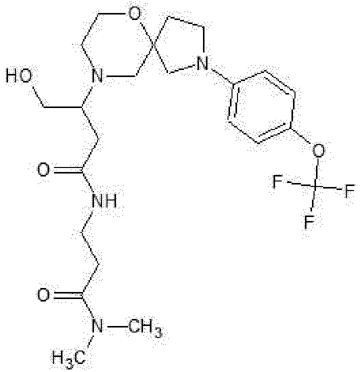 |
| **5-(4-hydroxy-3-{2-[4-(trifluoromethoxy)phenyl]-6-oxa-2,9-diazaspiro[4.5]decan-9-yl}butanamido)-1H-pyrazole-3-carboxamide** | 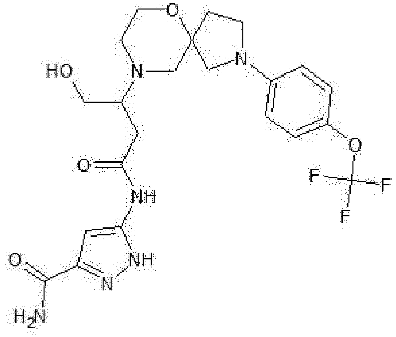 |
| **4-hydroxy-N-(2,2,2-trifluoroethyl)-3-{2-[4-(trifluoromethoxy)phenyl]-6-oxa-2,9-diazaspiro[4.5]decan-9-yl}butanamide** | 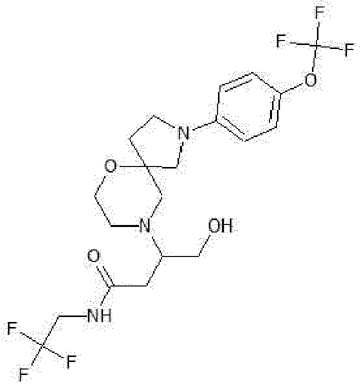 |
| **4-hydroxy-3-{2-[4-(trifluoromethoxy)phenyl]-6-oxa-2,9-diazaspiro[4.5]decan-9-yl}-N-[4-(trifluoromethyl)phenyl]butanam ide** | 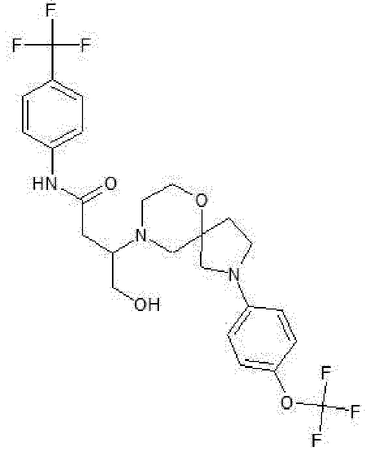 |
| ***N*-(5-chloropyridin-2-yl)-4-hydroxy-3-{2-[4-(trifluoromethoxy)phenyl]-6-oxa-2,9-diazaspiro[4.5]decan-9-yl}butanamide** | 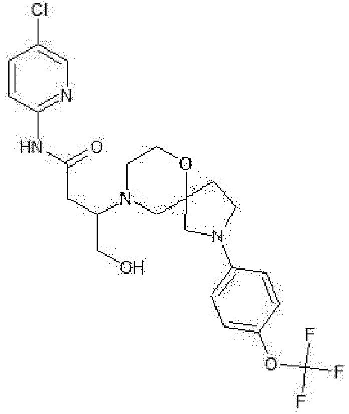 |
| **4-hydroxy-N-(3-methoxyphenyl)-3-{2-[4-(trifluoromethoxy)phenyl]-6-oxa-2,9-diazaspiro[4.5]decan-9-yl}butanamide** | 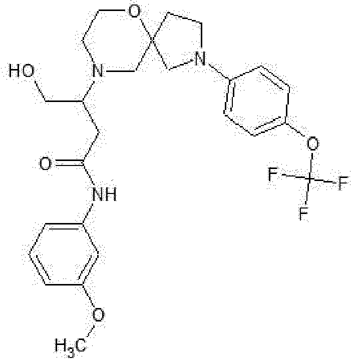 |
| ***N*-{4-acetamidophenyl)-4-hydroxy-3-{2-[4-(trifluoromethoxy)phenyl]-6-oxa-2,9-diazaspiro[4.5]decan-9-yl}butanamide** | 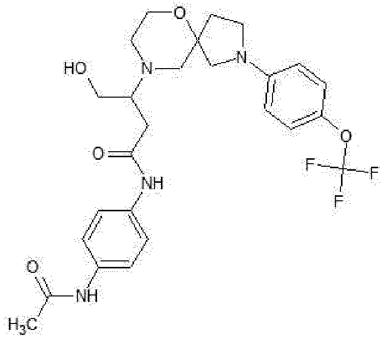 |
| **N-(3-acetamidophenyl)-4-hydroxy-3-{2-[4-(trifluoromethoxy)phenyl]-6-oxa-2,9-diazaspiro[4.5]decan-9-yl}butanamide** | 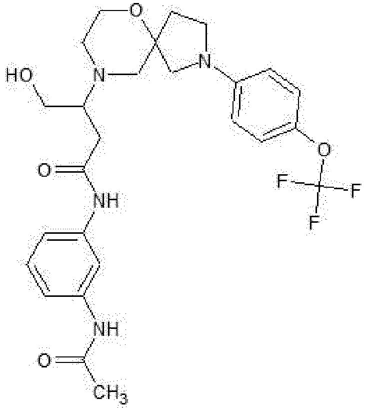 |
| **N-(4-cyanophenyl)-4-hydroxy-3-{2-[4-(trifluoromethoxy)phenyl]-6-oxa-2,9-diazaspiro[4.5]decan-9-yl}butanamide** | 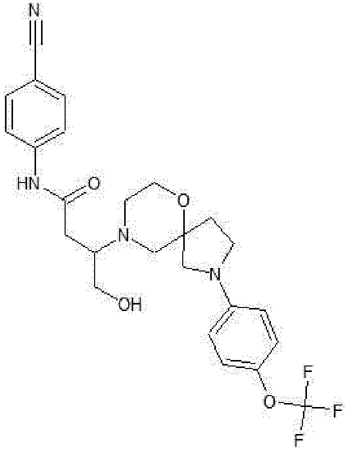 |
| **N-(2-chlorophenyl)-4-hydroxy-3-{2-[4-(trifluoromethoxy)phenyl]-6-oxa-2,9-diazaspiro[4.5]decan-9-yl}butanamide** | 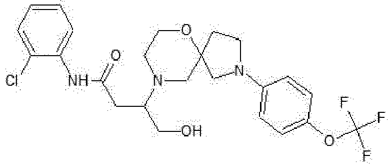 |
| ***N*-(3-chlorophenyl)-4-hydroxy-3-{2-[4-(trifluoromethoxy)phenyl]-6-oxa-2,9-diazaspiro[4.5]decan-9-yl}butanamide** | 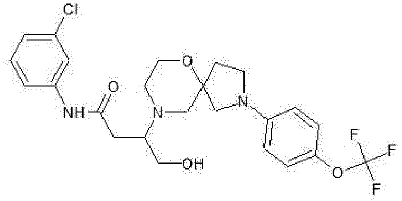 |
| ***N*-(4-fluorophenyl)-4-hydroxy-3-{2-[4-(trifluoromethoxy)phenyl]-6-oxa-2,9-diazaspiro[4.5]decan-9-yl}butanamide** | 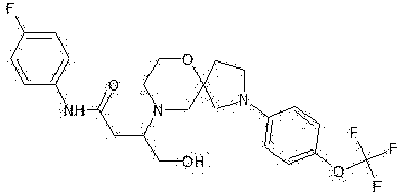 |
| **1-(4-hydroxy-3-{2-[4-(trifluoromethoxy)phenyl]-6-oxa-2,9-diazaspiro[4.5]decan-9-yl}butanoyl)imidazolidin-4-one** | 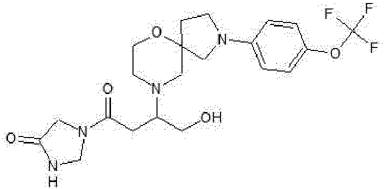 |
| **4-hydroxy-N-(prop-2-en-1-yl)-3-{2-[4-(trifluoromethoxy)phenyl]-6-oxa-2,9-diazaspiro[4.5]decan-9-yl}butanamide** | 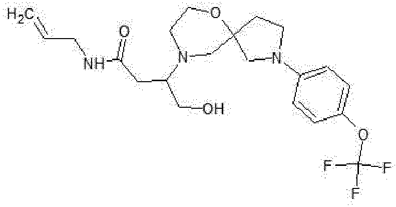 |
| **4-hydroxy-1-(pyrazolidin-1-yl)-3-{2-[4-(trifluoromethoxy)phenyl]-6-oxa-2,9-diazaspiro[4.5]decan-9-yl}butan-1-one** | 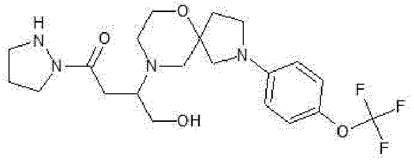 |
| **4-hydroxy-1-(3-hydroxypyrrolidin-1-yl)-3-{2-[4-(trifluoromethoxy)phenyl]-6-oxa-2,9-diazaspiro[4.5]decan-9-yl}butan-1-one** | 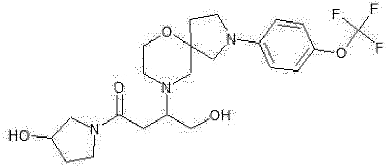 |
| **2-[1-(4-hydroxy-3-{5-[4-(trifluoromethoxy)phenyl]-1H,2H,3H,4H,5H,6H-pyrrolo[3,4-c]pyrrol-2-yl}butanoyl)pyrrolidin-3-yl]acetonitrile** | 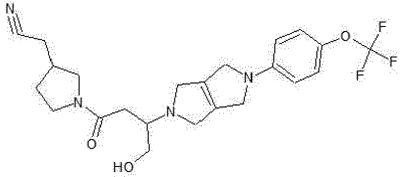 |
| **N-(3-chlorophenyl)-4-hydroxy-3-{5-[4-(trifluoromethoxy)phenyl]-1H,2H,3H,4H,5H,6H-pyrrolo[3,4-c]pyrrol-2-yl}butanamide** | 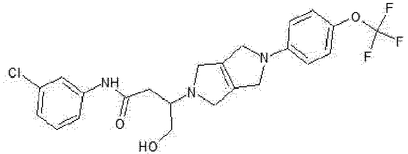 |
| **4-hydroxy-N-(pyridin-3-yl)-3-{5-[4-(trifluoromethoxy)phenyl]-1H,2H,3H,4H,5H,6H-pyrrolo[3,4-c]pyrrol-2-yl}butanamide** | 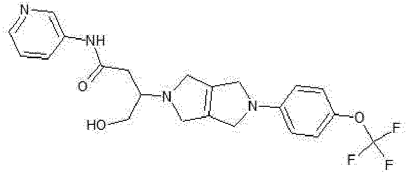 |
| **4-hydroxy-N-(1,2-thiazol-5-yl)-3-{5-[4-(trifluoromethoxy)phenyl]-1H,2H,3H,4H,5H,6H-pyrrolo[3,4-c]pyrrol-2-yl}butanamide** | 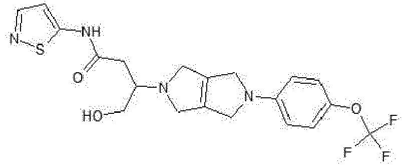 |
| **N-[(dimethylcarbamoyl)methyl]-4-hydroxy-3-{5-[4-(trifluoromethoxy)phenyl]-1H,2H,3H,4H,5H,6H-pyrrolo[3,4-c]pyrrol-2-yl}butanamide** | 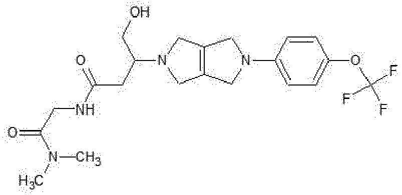 |
| **N-(2-chlorophenyl)-4-hydroxy-3-{5-[4-(trifluoromethoxy)phenyl]-1H,2H,3H,4H,5H,6H-pyrrolo[3,4-c]pyrrol-2-yl}butanamide** | 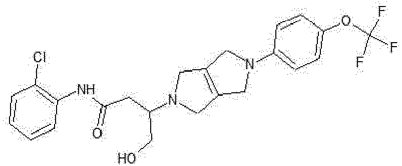 |
| **4-hydroxy-N-(1H-pyrazol-1-yl)-3-{2-[4-(trifluoromethoxy)phenyl]-6-oxa-2,9-diazaspiro[4.5]decan-9-yl}butanamide** | 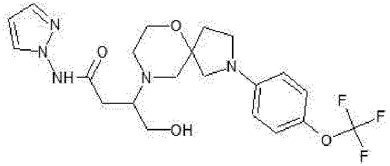 |
| **N-(4-cyanophenyl)-4-hydroxy-3-{5-[4-(trifluoromethoxy)phenyl]-1H,2H,3H,4H,5H,6H-pyrrolo[3,4-c]pyrrol-2-yl}butanamide** | 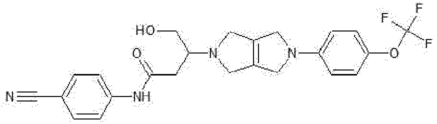 |
| **4-hydroxy-1-{1H,4H,SH,6H,7H-pyrazolo[4,3-c]pyridin-5-yl}-3-{5-[4-(trifluoromethoxy)phenyl]-1H,2H,3H,4H,5H,6H-pyrrolo[3,4-c]pyrrol-2-yl}butan-1-one** | 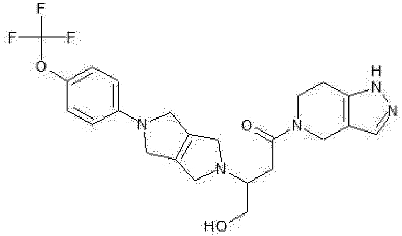 |
| **4-hydroxy-N-[(1r,4r)-4-cyanocyclohexyl]-3-{2-[4-(trifluoromethoxy)phenyl]-6-oxa-2,9-diazaspiro[4.5]decan-9-yl}butanamide** | 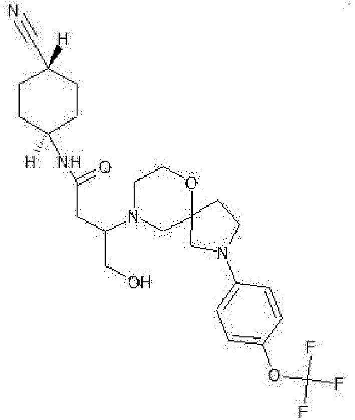 |
| **N-(carbamoylamino)-4-hydroxy-3-{2-[4-(trifluoromethoxy)phenyl]-6-oxa-2,9-diazaspiro[4.5]decan-9-yl}butanamide** | 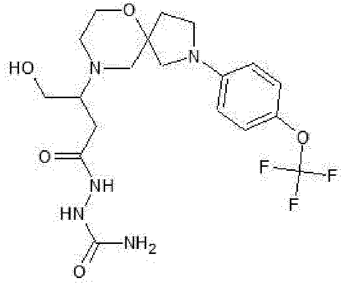 |
| **4-hydroxy-N-(pyridin-2-yl)-3-{2-[4-(trifluoromethoxy)phenyl]-6-oxa-2,9-diazaspiro[4.5]decan-9-yl}butanamide** | 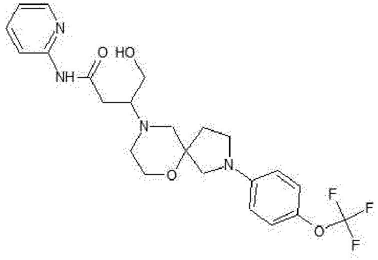 |
| **4-hydroxy-N-(pyridin-3-yl)-3-{2-[4-(trifluoromethoxy)phenyl]-6-oxa-2,9-diazaspiro[4.5]decan-9-yl}butanamide** | 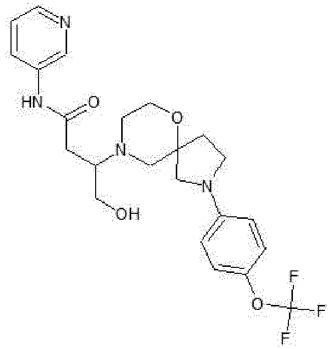 |
| **4-hydroxy-3-{5-[4-(trifluoromethoxy)phenyl]-1H,2H,3H,4H,5H,6H-pyrrolo[3,4-c]pyrrol-2-yl}-N-(2,4,6-trimethylphenyl)butanamide** | 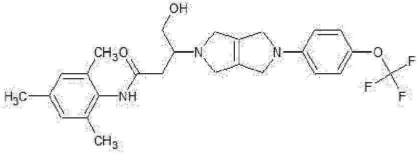 |
| **4-hydroxy-N-(4-methanesulfonylphenyl)-3-{2-[4-(trifluoromethoxy)phenyl]-6-oxa-2,9-diazaspiro[4.5]decan-9-yl}butanamide** | 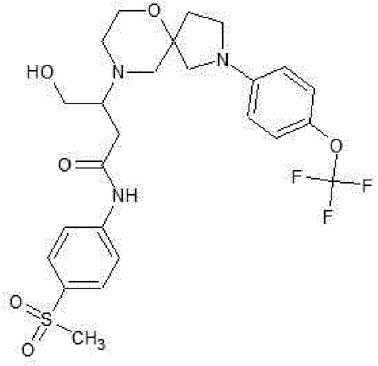 |
| **4-hydroxy-1-{1H,4H,5H,6H,7H-pyrrolo[3,2-c]pyridin-5-yl}-3-{5-[4-(trifluoromethoxy)phenyl]-1H,2H,3H,4H,5H,6H-pyrrolo[3,4-c]pyrrol-2-yl}butan-1-one** | 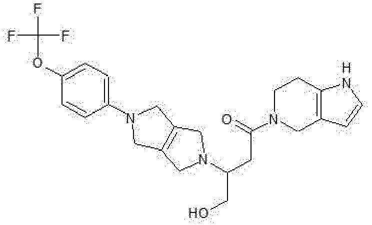 |

In a second aspect the present invention relates to a compound of any one of the above aspect or embodiments for use in a method for treating diabetes or pre-diabetes, in a subject in need thereof. Typically, the diabetes is type 2. In a preferred embodiment the compound of formula I or II binds to VDAC1 in an MST assay with a Kd value of less than 15 µM, or less than 10 µM, such as less than 5 µM, such as less than 1 µM.

The term "halogen" as used herein means Cl, F, I or Br.

The term "quaternary ammonium" as used herein means a nitrogen having four valence bonds, such as NH₄⁺ or N(CH₃)₄⁺.

The term "C₁₋ₓ alkyl" as used herein means a linear alkyl group containing 1-x carbon atoms, e.g. C₁₋₃ or C₁₋₆, such as methyl, ethyl, propyl, butyl, pentyl or hexyl.

The term "C₅₋₇ cycloalkyl" as used herein means a cyclic alkyl group containing 5-7 carbon atoms, such as cyclopentyl, cyclohexyl, or cycloheptyl.

The term "C₂₋₄ alkenyl" as used herein means means a linear alkenyl having from 2-4 carbonatoms and comprising one double bond, such as ethenyl.

The term "Oxo" as used herein means an oxygen atom with double bonds, also indicated as =O.

The term "COO-C₁₋₆ alkyl, SO₃-C₁₋₆ alkyl, NHCO-C₁₋₃ alkyl, OC₁₋₃ alkyl, SO₂-C₁₋₃ alkyl" as used herein means a group selected from COO, SO₃, NHCO, O, or SO₂ to which is attached a C₁₋ₓ alkyl as defined above.

The term "C₁₋₃ alkyl-CN" as used herein means a C₁₋ₓ alkyl as defined above having a CN attached to one of the carbon atoms.

The term "aryl" as used herein means a mono or bicyclic aromatic ring system containing 6-12 carbon atoms, such as phenyl or naphthyl.

The term "a monoheterocyclic ring" as used herein means one ring comprising carbon atoms and at least one hetero atom, such as N, O or S.

The term "a biheterocyclic group" as used herein means two monoheterocyclic rings fused together and sharing two ring atoms, and each comprising carbon atoms and at least one hetero atom, such as N, O or S.

The term "a non-aromatic heterocyclic system" as used herein in connection with a monoheterocyclic ring, a biheterocyclic group, a spiroheterocyclic group or a first and a second monoheterocyclic ring connected by a linker, such as a bond, means a saturated or partly unsaturated system, provide that none of the heterocyclic rings are aromatic.

The term "VDAC" as used herein refers to Voltage-Dependent Anion Channel protein of a highly conserved family of mitochondrial porins. Three VDAC isoforms, VDAC Type 1 (VDAC1), VDAC Type 2 (VDAC2) and VDAC Type 3 (VDAC3), encoded by three genes, are known to date. As used herein the term "VDAC1" as mean mammalian VDAC 1 and in particular human VDAC 1 comprising 283 amino acids (NP_003365) (Shoshan-Barmatz V et al., 2010. Molecular aspects of medicine 31:227-285).

When the compound of formula I or II and pharmaceutical compositions herein disclosed are used for the above treatment, a therapeutically effective amount of at least one compound is administered to a mammal in need of said treatment.

The term "treatment" and "treating" as used herein means the management and care of a patient for the purpose of combating a condition, such as a disease or a disorder. The term is intended to include the full spectrum of treatments for a given condition from which the patient is suffering, such as administration of the active compound to alleviate the symptoms or complications, to delay the progression of the disease, disorder or condition, to alleviate or relief the symptoms and complications, and/or to cure or eliminate the disease, disorder or condition as well as to prevent the condition, wherein prevention is to be understood as the management and care of a patient for the purpose of combating the disease, condition, or disorder and includes the administration of the active compounds to prevent the onset of the symptoms or complications. The treatment may either be performed in an acute or in a chronic way. The patient to be treated is preferably a mammal; in particular, a human being, but it may also include animals, such as dogs, cats, cows, sheep and pigs.

The term "a therapeutically effective amount" of the compound for use of the present invention as used herein means an amount sufficient to cure, alleviate or partially arrest the clinical manifestations of a given disease and its complications. An amount adequate to accomplish this is defined as "therapeutically effective amount". Effective amounts for each purpose will depend on the severity of the disease or injury as well as the weight and general state of the subject. It will be understood that determining an appropriate dosage may be achieved using routine experimentation, by constructing a matrix of values and testing different points in the matrix, which is all within the ordinary skills of a trained physician or veterinary.

In a still further aspect the present invention relates to a pharmaceutical composition comprising the compound of formula I or II for use and optionally a pharmaceutically acceptable additive, such as a carrier or an excipient.

As used herein "pharmaceutically acceptable additive" is intended without limitation to include carriers, excipients, diluents, adjuvant, colorings, aroma, preservatives etc. that the skilled person would consider using when formulating a compound of the present invention in order to make a pharmaceutical composition.

The adjuvants, diluents, excipients and/or carriers that may be used in the composition of the invention must be pharmaceutically acceptable in the sense of being compatible with the compound of formula I or II for use and the other ingredients of the pharmaceutical composition, and not deleterious to the recipient thereof. It is preferred that the compositions shall not contain any material that may cause an adverse reaction, such as an allergic reaction. The adjuvants, diluents, excipients and carriers that may be used in the pharmaceutical composition of the invention are well known to a person skilled within the art.

As mentioned above, the compositions and particularly pharmaceutical compositions as herein disclosed may, in addition to the compounds of formula I or II herein disclosed, further comprise at least one pharmaceutically acceptable adjuvant, diluent, excipient and/or carrier. In some embodiments, the pharmaceutical compositions comprise from 1 to 99 % by weight of said at least one pharmaceutically acceptable adjuvant, diluent, excipient and/or carrier and from 1 to 99 % by weight of a compound of formula I or II as herein disclosed. The combined amount of the active ingredient and of the pharmaceutically acceptable adjuvant, diluent, excipient and/or carrier may not constitute more than 100% by weight of the composition, particularly the pharmaceutical composition.

In some embodiments, only one compound of formula I or II as herein disclosed is used for the purposes discussed above.

In some embodiments, two or more of the compounds of formula I or II as herein disclosed are used in combination for the purposes discussed above.

The composition, particularly pharmaceutical composition comprising a compound set forth herein may be adapted for oral, intravenous, topical, intraperitoneal, nasal, buccal, sublingual, or subcutaneous administration, or for administration via the respiratory tract in the form of, for example, an aerosol or an air-suspended fine powder. Therefore, the pharmaceutical composition may be in the form of, for example, tablets, capsules, powders, nanoparticles, crystals, amorphous substances, solutions, transdermal patches or suppositories.

Further embodiments of the process are described in the experimental section herein, and each individual process as well as each starting material constitutes embodiments that may form part of embodiments.

The above embodiments should be seen as referring to any one of the aspects (such as 'method for treatment', 'pharmaceutical composition', 'compound of formula I or II for use as a medicament', or 'compound of formula I or II for use in a method') described herein as well as any one of the embodiments described herein unless it is specified that an embodiment relates to a certain aspect or aspects of the present invention.

All headings and sub-headings are used herein for convenience only and should not be construed as limiting the invention in any way.

Any combination of the above-described elements in all possible variations thereof is encompassed by the invention unless otherwise indicated herein or otherwise clearly contradicted by context.

The terms "a" and "an" and "the" and similar referents as used in the context of describing the invention are to be construed to cover both the singular and the plural, unless otherwise indicated herein or clearly contradicted by context.

The term "and/or" as used herein is intended to mean both alternatives as well as each of the alternatives individually. For instance, expression "xxx and/or yyy" means "the xxx and yyy; the xxx; or the yyy", all three alternatives are subject to individual embodiments.

Recitation of ranges of values herein are merely intended to serve as a shorthand method of referring individually to each separate value falling within the range, unless other-wise indicated herein, and each separate value is incorporated into the specification as if it were individually recited herein. Unless otherwise stated, all exact values provided herein are representative of corresponding approximate values (e.g., all exact exemplary values provided with respect to a particular factor or measurement can be considered to also pro-vide a corresponding approximate measurement, modified by "about," where appropriate).

All methods described herein can be performed in any suitable order unless otherwise indicated herein or otherwise clearly contradicted by context.

The use of any and all examples, or exemplary language (e.g., "such as") provided herein, is intended merely to better illuminate the invention and does not pose a limitation on the scope of the invention unless otherwise indicated. No language in the specification should be construed as indicating any element is essential to the practice of the invention unless as much is explicitly stated.

The citation and incorporation of patent documents herein is done for convenience only and does not reflect any view of the validity, patentability and/or enforceability of such patent documents.

The description herein of any aspect or embodiment of the invention using terms such as "comprising", "having", "including" or "containing" with reference to an element or elements is intended to provide support for a similar aspect or embodiment of the invention that "consists of", "consists essentially of', or "substantially comprises" that particular element or elements, unless otherwise stated or clearly contradicted by context (*e*.*g*., a composition described herein as comprising a particular element should be understood as also describing a composition consisting of that element, unless otherwise stated or clearly contradicted by context). This invention includes all modifications and equivalents of the subject matter recited in the aspects or claims presented herein to the maximum extent permitted by applicable law.

The present invention is further illustrated by the following examples that, however, are not to be construed as limiting the scope of protection. The features disclosed in the foregoing description and in the following examples may, both separately and in any combination thereof, be material for realizing the invention in diverse forms thereof.

### MST assay and data

MicroScale Thermophoresis (MST) is a biophysical technique that measures the strength of interaction (affinity) between two molecules by detecting variations in fluorescence signal as a result of an IR-laser induced temperature change. The range of the variation in the fluorescence signal correlates with the binding of a ligand to a fluorescent target. By that it allows for quantitative analysis of molecular interactions in solution on the microliter scale with high sensitivity.

The MST signal is composed of two major factors: The TRIC effect and the thermophoresis. TRIC stands for Temperature-Related Intensity-Change and describes how the fluorescence intensity of a fluorophore depends on the local temperature of the solution. For the vast majority of fluorophores, the fluorescence intensity decreases with increasing temperature. Most importantly, however, the extent of the temperature dependence is strongly related to the chemical environment of the fluorophore. This environment is sensitively affected by the binding of a ligand molecule to the fluorescently labeled target molecule (via conformational changes or ligand-dye proximity). The second component of the MST-signal, the thermophoresis describes the movement of molecules along temperature gradients which results in a quantifiable change of the local concentration of the target molecules. As these target molecules are fluorescently labeled in MST, such concentration changes can be monitored simply by fluorescence measurements. This directed movement of molecules depends on their molecular size, charge, and hydration shell. Binding of a ligand to a target molecule leads to the change of at least one of these parameters and therefore to an altered thermophoretic movement of the target-ligand complex compared to the single molecules alone. In sum, both TRIC and thermophoresis contribute to the overall MST-signal, which is detected by fluorescence measurement. Due to this robust physical principle and direct monitoring, MST can be used for determining the affinity and binding strength of almost any kind of molecular interaction or modification of small molecules, proteins, peptides, DNA, sugars, or molecular complexes.

For an in-depth description of the physical principles behind MST, reference is made to the following (Jerabek-Willemsen M, André T, Wanner A, Roth HM, Duhr S, Baaske P, Breitsprecher D (2014). "MicroScale Thermophoresis: Interaction analysis and beyond". Journal of Molecular Structure. 1077: 101-113 and Jerabek-Willemsen M, Wienken CJ, Braun D, Baaske P, Duhr S (2011). "Molecular interaction studies using microscale thermophoresis". Assay and Drug Development Technologies. 9 (4): 342-53.

### Recombinant VDAC1 labeling

Reconstituted N-terminal His-GST tagged E. coli recombinant human VDAC1 (hVDAC1) was buffer exchanged into protein labeling buffer due to the primary amine of the Tris lyophilization buffer interferes with NHS-based labeling. The labeling buffer was kept as close to the storage buffer of the target protein as given on the protein data sheet (LSBio LS-G25810). Tris was replaced by HEPES. Pluronic F-127 was added to 0.05% in order to enhance protein recovery in the final dye removal step. The total volume of the labeling batch was 100 µL. The labeling time was 30 minutes. The labeling reaction was performed at room temperature. Any unbound or unreacted dye was separated from the labeled protein on a gravity-flow gel-filtration column (GE Healthcare, PD Minitrap G-25, GE28-9180-07). The final target buffer was kept as close to the original protein storage buffer as possible. It contained Tris again instead of HEPES.

### Assay validation

The first step of the technical assay establishment was a so-called "noise test". In this test, the intrinsic MST-noise of the labeled target protein was tested. Subsequently, a reference compound or positive control (CAS number: 2086257-77-2) was characterized to confirm previously reported VDAC1 binding (Zhang et al (2019)). Preserving Insulin Secretion in Diabetes by Inhibiting VDAC1 Overexpression and Surface Translocation in β Cells". Cell Metabolism 29, 64-77.). In short, serial dilutions of the compound having CAS number: 2086257-77-2 was prepared in a way to match the final buffer conditions in the reaction mix (assay buffer). The highest concentration of ligand was 10.0 mM and the lowest 4.88 µM. A total of 12 dilution steps were prepared. The serial dilution was prepared in a total volume of 10 µL for each step with 0.1 µl of each dilution step mixed with 9.9 µl of the fluorescent target molecule (labeled VDAC1). The final reaction mixture, which was filled into premium-coated MST capillaries, contained a respective amount of ligand (max. conc. 100 µM, min. cone. 41 nM) and constant 5 nM fluorescent target molecule. The samples were analyzed on a NT.Automated instrument (Nanotemper Technologies, Munich) at 25°C, with 10% LED power and 40% Laser power.

To test the reproducibility of the established positive control interaction was tested using labeled VDAC1 at 4 °C for 0, 2, 4, 6 hours before preparation of assay samples.

### Screening assay

Compound serial dilutions were prepared using a Labcyte Echo 550 acoustic nano-liter dispenser. 10 mM DMSO compound stocks were transferred to LabCyte LP-0200 384-well plates ("source plates"). In 12 adjacent wells (e.g. A1-A12) of Greiner 784201 384-well plates ("destination plates"), a serially reduced volume series of the compounds were dispensed: 100, 50, 25, 12.5, 7.5, 2.5, 1.56, 0.78, 0.41, 0.20, 0.08, 0.04 nL (volumes below 1 nL were dispensed from intermediate DMSO dilutions of the compounds). After that, a DMSO-backfill was performed by filling each well up to a total volume of 100 nL. The destination plates were sealed with a heat-seal and stored at room temperature until use.

In addition to the compound serial dilutions, DMSO references were prepared in the same manner by dispensing a serially reduced volume series of DMSO (same volumes as above) and back-filling to 100 nL with DMSO.

The high-throughput compound screening was performed as follows. 100 µL labeled VDAC1 stock were thawed and centrifuged (15 min, 21000 x g, 4°C) and 90 µl supernatant was used. The thawed and centrifuged labeled VDAC1 stock was stored on ice for a maximum of 6 hours. After 6 hours, fresh labeled stock was thawed and centrifuged as described above. A positive control test with CAS number: 2086257-77-2 was prepared as escribed above. A positive control for assay validation was performed every 6 hours and after thawing a fresh labeled VDAC1 stock. A positive control was also performed after the full compound set had been measured. For compound **Example 1-10** screening, the labeled VDAC1 stock was diluted to 5.05 nM in assay buffer (see **Table 1)** and added to the pre-dispensed compound plates. 10 µL diluted VDAC1 stock were added per well using an Opentrons OT-2 liquid handling robot. Four 24-capillary chips (enough capillaries for 8 ligands in 12-data-point serial dilution) were filled with the sample solutions at a time (premium coated capillaries, Nanotemper Technologies, Munich, Germany). Filled capillaries were incubated for 10 minutes at room temperature. Four capillary chips (total of 8 ligands in serial dilution) were loaded into an NT.Automated MST instrument (Nanotemper Technologies, Munich, Germany) and measured with the settings listed in **Table 1**. The 12 p-data-point DMSO references were analyzed similarly.

**Table 1: HTS-MST assay parameters**

| **Target protein** | **VDAC1, labeled via NHS-chemistry** |
|---|---|
| **Target protein storage buffer** | 20 mM Tris-HCl pH 8.0, 150 mM NaCl, 1 mM DTT, 0.05% Pluronic F-127 |
| **Target protein stock concentration** | 370 nM |
| **Target protein assay concentration** | 5 nM |
| **Target protein storage** | Short term-storage at 4°C. Storage of frozen, labeled target protein at -80°C. |
| **Ligand compounds** | 10 mM DMSO stocks |
| **Ligand dilution** | 12-point, contact-less, direct serial dilution (Labcyte Echo 550) |
| **Ligand compound assay concentrations** | 100, 50, 25, 12.5, 7.5, 2.5, 1.56, 0.78, 0.41, 0.21, 0.08, 0.04 µM |
| **Ligand compound storage** | Long-term storage at -80 °C; Thawing of the required ligand compounds |
| **Assay buffer** | 20 mM Hepes pH 7.0, 150 mM NaCl, 1 mM DTT, 0.05% Pluronic F-127, 1% DMSO |
| **Fluorescence channel** | Red channel (647 nm) |
| **LED-power** | 10 |
| **MST-power** | 40% ("medium" setting) |
| **MST run time** | 3 seconds pre-run, 10 seconds laser-on time, 1 second post-run |
| **Assay temperature** | 25°C (thermostat controlled) |
| **Control compound** | CAS number: 2086257-77-2 |
| **Control measurements** | Four control measurements across the compound set. |

### Data analysis

The tested ligand compounds were classified into the following categories; binder, weak binder and non-binder. Data were analyzed with MO.AffinityAnalysis (version 2.2.6.5385, Nanotemper Technologies, Munich, Germany) and MO.ScreeningAnalysis (version 1.0.2.8057, Nanotemper Technologies, Munich, Germany). MST traces (fluorescence vs. time) were analyzed at the time intervals -3 to 0 seconds (laser off, "cold" time interval) and +1.5 to +2.5 seconds (laser on, "hot" time interval). Fnorm data were fitted to the KD model.

For classifying the analyzed ligands into the pre-defined categories, a 12-point DMSO blank-control was included in the screen. The noise of this blank control was 1.35 Fnorm units (2·*STDEV*(*Fnorm*))*.* Thus, for a classification as binder or weak binder, a minimum Fnorm signal amplitude of >(2·*noise*) (> 2.7) was required.

A typical MST plot of Example 4 is shown in Figure 1. (The MST plot is of the compound of example 4; K_{D} 5.1 µM (S/N 20.1; dFnorm 23.9).

| Table 2: VDAC1 affinity (K_{D}; µM) Example | K_{D} |
|---|---|
| 1 (not a compound of the present invention) | 12 |
| 2 (not a compound of the present invention) | 8.4 |
| 3 (not a compound of the present invention) | 5.2 |
| 4 | 5.1 |
| 5 | 3.8 |
| 6 | 9.8 |
| 7 | 13.1 |
| 8 | 2.8 |
| 9 | 11.4 |
| 10 | 1.9 |
| CAS: 2086257-77-2 | 14 |

### EXAMPLES

Abbreviatons used:
- DCM: Dichloromethane
- TFA: Trifluoroacetic acid
- CDI: Carbonyldiimidazole
- MeCN: Acetonitrile
- HOAc: Acetic acid
- MeOH: Methanol
- NaBH₃CN: Sodium cyanoborohydride
- NBS: 1-Bromo-2,5-pyrrolidinedione
- K₂CO₃: Potassium carbonate
- DIPEA: N,N-Diisopropylethylamine
- HATU: 1-[Bis(dimethylamino)methylene]-1H-1,2,3-triazolo[4,5-b]pyridinium 3-oxide hexafluorophosphate
- DMF: Dimethylformamide
- NaOH: Sodium hydroxide
- THF: Tetrahydrofuran
- LiHMDS: Lithium bis(trimethylsilyl)amide
- DIAD: Diisopropyl azodicarboxylate
- PPh₃: Triphenylphosphine
- r.t.: Room temperature

### 1. HPLC purification

Purification was performed by HPLC (H₂O - MeOH; Agilent 1260 Infinity systems equipped with DAD and mass-detectors. Waters Sunfire C18 OBD Prep Column, 100Å, 5 µm, 19 mm × 100 mm with SunFire C18 Prep Guard Cartridge, 100Å, 10 µm, 19 mm × 10 mm). The material was dissolved in 0.7 mL DMSO. Flow: 30mL/min. Purity of the obtained fractions was checked via the analytical LCMS. Spectra were recorded for each fraction as it was obtained straight after chromatography in the solution form. The solvent was evaporated in the flow of N₂ at 80°C. On the basis of post-chromatography LCMS analysis fractions were united. Solid fractions were dissolved in 0.5 mL MeOH and transferred into a pre-weighted marked vial. Obtained solutions were again evaporated in the flow of N₂ at 80°C. After drying, products were finally characterized by LCMS and ¹H NMR.

### 2. Analytical Methods

### NMR

### Instrument specifications:

Bruker AVANCE DRX 500
Varian UNITY plus 400

### LC/MS

### Instrument specifications:

Agilent 1100 Series LC/MSD system with DAD\ELSD and Agilent LC\MSD VL (G1956A), SL (G1956B) mass-spectrometer.
Agilent 1200 Series LC/MSD system with DAD\ELSD and Agilent LC\MSD SL (G6130A), SL (G6140A) mass-spectrometer.
All the LC/MS data were obtained using positive/negative mode switching.
Column Zorbax SB-C18 1.8 µm 4.6x15mm Rapid Resolution cartridge (PN 821975-932)
Mobile phase A - acetonitrile, 0.1% formic acid
B - water (0.1% formic acid)
Flow rate 3ml/min
Gradient 0 min - 100% B
0.01 min - 100% B
1.5 min - 0% B
1.8 min - 0% B
1.81 min - 100% B
Injection volume 1µl
Ionization mode atmospheric pressure chemical ionization (APCI)
Scan range m/z 80-1000

### 3. Experimental

All compounds were isolated as racemates if not otherwise stated.

### Synthesis of 4-chloro-N-(3-hydroxy-2-{4-[4-(trifluoromethoxy)phenyl]piperazin-1-yl}propyl)benzamide Example 1 (not a compound of the present invention)

### Step 1: Synthesis of 4-chloro-N-(prop-2-en-1-yl)benzamide

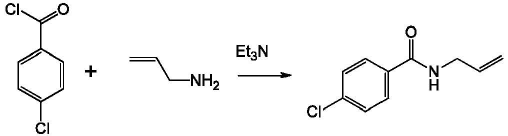

Prop-2-en-1-amine (1.48 g, 25.97 mmol) was dissolved in CH₂Cl₂ (30 mL). 4-Chlorobenzoyl chloride (5.0 g, 28.57 mmol) and triethylamine (5.78 g, 57.13 mmol) were added and the mixture was stirred for 2 days at room temperature under nitrogen. Then, aqueous NaOH solution (1 M, 30 mL) was added, and the mixture was extracted with CH₂Cl₂ (2 × 30 mL). The organic layers were combined and dried over anhydrous sodium sulfate, filtered and concentrated under vacuum. The residue was purified by flash chromatography (5:1 hexane/EtOAc, R_{f} = 0.10) to provide 4-chloro-N-(prop-2-en-1-yl)benzamide (4.46 g, 22.8 mmol, 87.8% yield) as a yellow solid.

### Step 2: Synthesis of 4-chloro-N-(2,3-dihydroxypropyl)benzamide

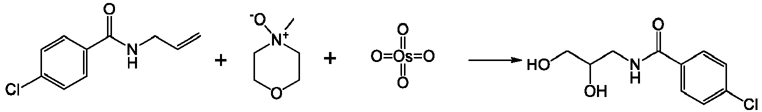

To a stirred solution of the 4-chloro-N-(prop-2-en-1-yl)benzamide (4.4 g, 22.49 mmol) in 2:1 acetonitrile/water solution (100 mL:50 mL) 4-methylmorpholin-4-ium-4-olate (5.27 g, 44.98 mmol) was added at 0°C followed by tetraoxoosmium (114.35 mg, 449.79 µmol). The mixture was stirred overnight at room temperature; The reaction was quenched by the addition of sodium sulfite (25 g) and diluted with water (60 mL). The aqueous solution was then extracted with EtOAc (3 × 90 mL), the combined organic extracts were dried over sodium sulfate and concentrated in vacuo to afford 4-chloro-N-(2,3-dihydroxypropyl)benzamide (4.45 g, 19.38 mmol, 86.2% yield) as crystalline solid.

### Step 3: The synthesis of N-{3-[(tert-butyldimethylsilyl)oxy]-2-hydroxypropyl}-4-chlorobenzamide

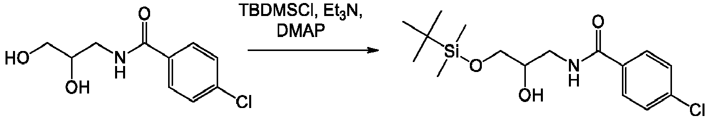

4-Chloro-N-(2,3-dihydroxypropyl)benzamide (3.92 g, 17.07 mmol) was suspended in CH₂Cl₂ (600 mL). Tert-butyl(chloro)dimethylsilane (2.86 g, 18.95 mmol) in CH₂Cl₂ (10 mL) was added under stirring, followed by triethylamine (2.07 g, 20.48 mmol) and N,N-dimethylpyridin-4-amine (83.41 mg, 682.71 µmol). The mixture was stirred for 36 hours and was then shaken with water (100 mL). The aqueous phase was washed with CH₂Cl₂ (70 mL), and the combined organic phases was combined, washed with brine (2 × 50 mL) and then dried over Na₂SO₄. The solution was concentrated under reduced pressure, and the oily residue was purified by chromatography on silica gel (hexane/EtOAc 1/1, R_{f} = 0.42) to afford N-3-[(tertbutyldimethylsilyl)oxy]-2-hydroxypropyl-4-chlorobenzamide (2.61 g, 7.59 mmol, 44.5% yield) as colorless oil.

### Step 4: Synthesis of N-{3-[(tert-butyldimethylsilyl)oxy]-2-oxopropyl}-4-chlorobenzamide

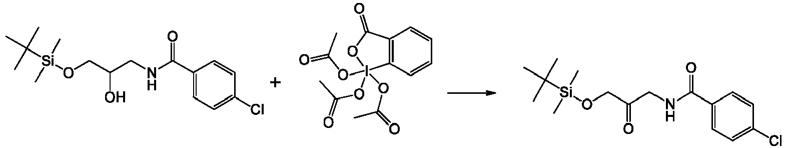

To a solution of 1,1,1-tris(acetoxy)-1,1-dihydro-1,2-benziodoxol-3(1H)-one (493.73 mg, 1.16 mmol) in anhydrous dichloromethane (5 mL) at room temperature was added a solution N-3-[(tert-butyldimethylsilyl)oxy]-2-hydroxypropyl-4-chlorobenzamide (400.35 mg, 1.16 mmol) in anhydrous dichloromethane (5 mL). The resulting mixture was stirred at room temperature for 12 h, then evaporated *in vacuo* to a third of the initial volume, and applied to a column packed with silica. Elution with ethyl acetate-petroleum ether solvent mixture (111) afforded N-3-[(tertbutyldimethylsilyl)oxy]-2-oxopropyl-4-chlorobenzamide (400.0 mg, 1.17 mmol, 100.5% yield) as colorless oil.

### Step 5: Synthesis of 4-chloro-N-(3-hydroxy-2-{4-[4-(trifluoromethoxy)phenyl]piperazin-1-yl}propyl)benzamide

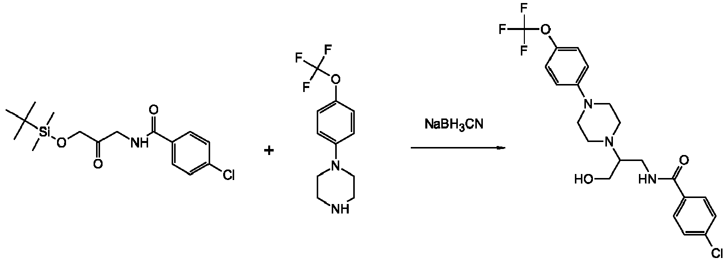

1-[4-(Trifluoromethoxy)phenyl]piperazine (345.89 mg, 1.4 mmol) was added to a solution of N-3-[(tert-butyldimethylsilyl)oxy]-2-oxopropyl-4-chlorobenzamide (400.24 mg, 1.17 mmol) in MeOH (10 mL) and the reaction mixture stirred at room temperature for 15 min, then sodium cyanoborohydride (73.56 mg, 1.17 mmol) was added and stirring continued for 48 h, then the solution was concentrated under reduced pressure to dryness. The residue was dissolved in CH₂Cl₂ (10 mL) and 10% HCl (10 mL) was added, and the mixture stirred at room temperature for 2 hours, CH₂Cl₂ was separated and the aqueous layer was washed CH₂Cl₂ (10 mL). Organic layers were combined, aqueous layer was triturated with solid NaHCO₃ to pH = 8. The crude product was extracted with CH₂Cl₂ (3 × 15 mL). The organic layers were combined, dried under Na₂SO₄ and concentrated under reduced pressure. The residue was purified by reverse preparative HPLC to afford 4-chloro-N-(3-hydroxy-2-4-[4-(trifluoromethoxy)phenyl]piperazin-1-ylpropyl)benzamide (29.0 mg, 63.34 µmol, 5.4% yield). LC-MS (ESI-pos): [M+H]⁺ = 458.2/460.2

### Synthesis of N-(4-chlorophenyl)-5-hydroxy-3-{4-[4-(trifluoromethoxy)phenyl]piperazin-1-yl}pentanamide Example 2 (not a compound of the present invention)

### Step 1A: The synthesis of ethyl (2E)-5-(prop-2-en-1-yloxy)pent-2-enoate

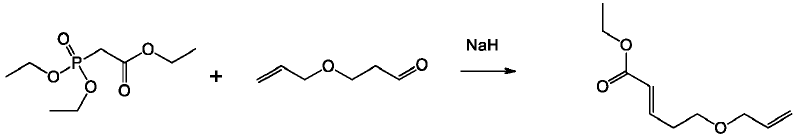

To a stirred suspension of sodium hydride (420.47 mg, 17.52 mmol) in THF (30 mL) was added ethyl 2-(diethyl phosphono)acetate (3.93 g, 17.52 mmol) at 0°C. The reaction mixture was stirred for 15 min, and a solution of the 3-(allyloxy)propanal (2.0 g, 17.52 mmol) in THF (5 mL) was then added. The mixture was stirred overnight at room temperature and then THF solution was evaporated *in vacuo* to afford ethyl (2E)-5-(prop-2-en-1-yloxy)pent-2-enoate (3.1 g, 16.83 mmol, 96% yield) as colorless oil. It was used in the next step without further purification.

### Step 1B: The synthesis of tert-butyl 4-[4-(trifluoromethoxy)phenyl]piperazine-1-carboxylate

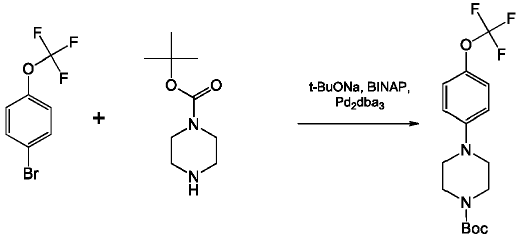

To a solution of 1-bromo-4-(trifluoromethoxy)benzene (24.1 g, 100.0 mmol) in toluene (500 mL) tert-butyl piperazine-1-carboxylate (16.76 g, 90.0 mmol), tris((4E)-1,5-diphenylpenta-1,4-dien-3-one) dipalladium (2.29 g, 2.5 mmol), 1-[2-(diphenylphosphanyl)naphthalen-1-yl]naphthalen-2-yldiphenylphosphane (3.11 g, 5.0 mmol) and sodium tert-butoxide (19.22 g, 200.0 mmol) were added consiquently. The mixture was refluxed under Ar atmosphere overnight. The solvent was evaporated to provide crude tert-butyl 4-[4-(trifluoromethoxy)phenyl]piperazine-1-carboxylate (63.0 g, 181.9 mmol, 181.9% yield) as a residue. It was directly used for next step without further purification.

### Step 2: Synthesis of ethyl 5-(prop-2-en-1-yloxy)-3-{4-[4-(trifluoromethoxy)phenyl]piperazin-1-yl}pentanoate

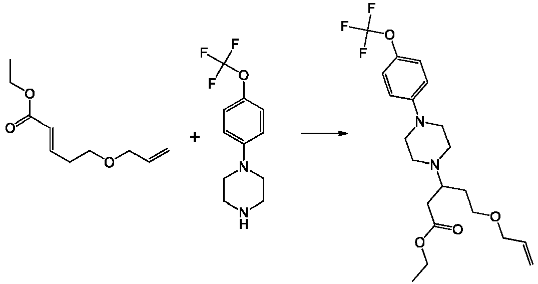

To a solution of ethyl (2E)-5-(prop-2-en-1-yloxy)pent-2-enoate (2.0 g, 10.86 mmol) in dry MeOH (10 mL) was added 1-[4-(trifluoromethoxy)phenyl]piperazine (2.67 g, 10.86 mmol) and the reaction mixture was stirred at room temperature overnight, then 6 hours at reflux. The reaction mixture was evaporated and crude product purified by flash chromatography on silica (eluent DCM/EtOAC = 1/1, Rf = 0.65) to afford ethyl 5-(prop-2-en-1-yloxy)-3-4-[4-(trifluoromethoxy)phenyl]piperazin-1-ylpentanoate (2.9 g, 6.74 mmol, 62.1% yield) . It was used in the next step without further purification.

### Step 3: The synthesis of 5-(prop-2-en-1-yloxy)-3-{4-[4-(trifluoromethoxy)phenyl]piperazin-1-yl}pentanoic acid

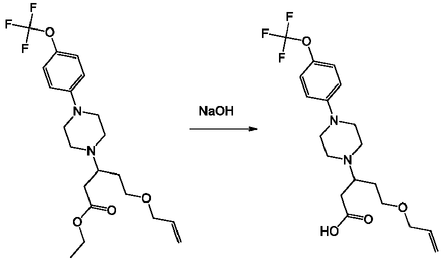

To a suspension of ethyl 5-(prop-2-en-1-yloxy)-3-4-[4-(trifluoromethoxy)phenyl]piperazin-1-ylpentanoate (2.9 g, 6.74 mmol) in MeOH and water (60 ml, 4/1) was added solution of sodium hydroxide (480.0 mg, 12.0 mmol) in water (3 mL) and reaction mixture stirred at room temperature 2 hours, then sodium hydrogen sulfate (1.52 g, 12.67 mmol) was added and reaction mixture stirred for 1 hour, and evaporated to dryness. The crude product was dissolved in MTBE (100 mL), washed with water (3x25), and evaporated to afford of 5-(prop-2-en-1-yloxy)-3-4-[4-(trifluoromethoxy)phenyl]piperazin-1-ylpentanoic acid (2.2 g, 5.47 mmol, 81.2% yield) as yellow oil, which was used in next without purification.

### Step 4: Synthesis of N-(4-chlorophenyl)-5-(prop-2-en-1-yloxy)-3-{4-[4-(trifluoromethoxy)phenyl]piperazin-1-yl}pentanamide

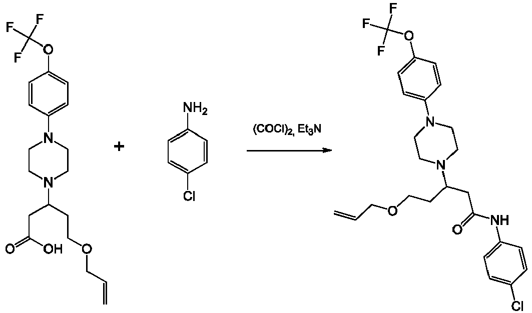

To a stirred solution of 5-(prop-2-en-1-yloxy)-3-4-[4-(trifluoromethoxy)phenyl]piperazin-1-ylpentanoic acid (2.2 g, 5.47 mmol) in DCM (30 mL) was added dropwise oxalyl chloride (693.93 mg, 5.47 mmol) and reaction mixture stirred at room temperature for 1 hour. After this time reaction mixture evaporated, residue was dissolved in DCM (30 mL) then 4-chloroaniline (697.46 mg, 5.47 mmol) and triethylamine (1.38 g, 13.67 mmol, 1.91 ml) were added and stirring continued at room temperature for 2 hours. Reaction mixture washed of water (3×30 mL) and evaporated. The crude product was purified by column chromatography (silica, eluent hexane/EtOAc = 1/1, R_{f} = 0.36) to afford crude N-(4-chlorophenyl)-5-(prop-2-en-1-yloxy)-3-4-[4-(trifluoromethoxy)phenyl]piperazin-1-ylpentanamide (1.21 g, 2.36 mmol, 43.2% yield) as a yellow oil. The crude material was further purified by reverse phase HPLC to afford pure title compound.

### Step 5: Synthesis of N-(4-chlorophenyl)-5-hydroxy-3-{4-(4-(trifluoromethoxy)phenyl]piperazin-1-yl}pentanamide

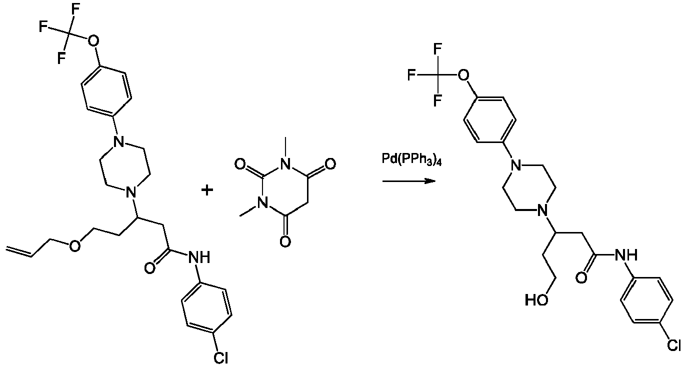

A mixture of N-(4-chlorophenyl)-5-(prop-2-en-1-yloxy)-3-4-[4-(trifluoromethoxy)phenyl]piperazin-1-ylpentanamide (511.0 mg, 998.12 µmol) , 1,3-dimethyl-1,3-diazinane-2,4,6-trione (314.48 mg, 2.01 mmol), tetrakis(triphenylphosphine)palladium(0) (57.76 mg, 49.81 µmol) and dry THF (4 mL) was heated at 90 °C in a sealed tube under argon atmosphere. After being stirred at the same temperature for 24 h, the reaction mixture was poured into sat. aq Na₂CO₃ and extracted with EtOAc twice. Organic layer was washed with brine, dried over MgSO₄ and evaporated in vacuo. The crude product was purified by reverse preparative HPLC to afford N-(4-chlorophenyl)-5-hydroxy-3-4-[4-(trifluoromethoxy)phenyl]piperazin-1-ylpentanamide (151.9 mg, 321.89 µmol, 32 % yield). LC-MS (ESI-pos): [M+H]⁺ = 472.2/474.2

### Starting material synthesis

In the examples below the p-trifluoromethoxyphenylaminoyl starting materials were synthesized from commercially available 1-bromo-4-(trifluoromethoxy)benzene and relevant amine by a method similarly to that described in Example 2, step 1B. The relevant amine may be protected by a cleavable protecting group such as e.g. a *tert*-butyloxycarbamoyl, a benzyloxycarbonyl group or similar. Alternatively, the p-trifluoromethoxyphenylaminoyl starting materials were synthesized from commercially available 1-bromo-4-(trifluoromethoxy)benzene and relevant non-protected amine by a method similarly to that described in Example 2, step 1B after which the starting materials were purified by standard methods known to a person skilled in the art, such as manual or automated silica chromatography.

### The Synthesis of N-(4-chlorophenyl)-4-hydroxy-3-{4-[4-(trifluoromethoxy)phenyl]-1,4-diazepan-1-yl}butanamide Example 3 (not a compound of the present invention)

### Step 1: Synthesis of 4-{4-[4-(trifluoromethoxy)phenyl]-1,4-diazepan-1-yl}oxolan-2-one

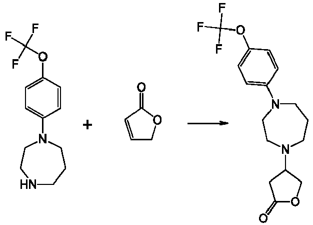

A mixture of 1-[4-(trifluoromethoxy)phenyl]-1,4-diazepane (5.2 g, 19.98 mmol) and 2,5-dihydrofuran-2-one (3.36 g, 39.96 mmol) in MeOH (12.5 mL) was stirred at room temperature overnight. The mixture was evaporated in *vacuo.* The crude product was purified by column chromatography on silica gel (CH₂Cl_{2/}EtOAc = 1/1, R_{f} = 0.12) to provide 4-4-[4-(trifluoromethoxy)phenyl]-1,4-diazepan-1-yloxolan-2-one (2.4 g, 6.97 mmol, 34.9% yield) as a yellow oil.

### Step 2: Synthesis of N-(4-chlorophenyl)-4-hydroxy-3-{4-(4-(trifluoromethoxy)phenyl]-1,4-diazepan-1-yl}butanamide

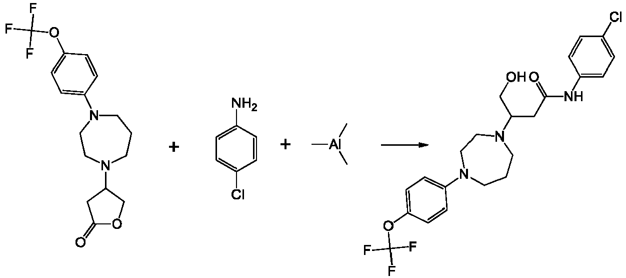

To a solution of 4-chloroaniline (254.87 mg, 2.0 mmol) in toluene (5 mL) was added trimethylaluminum (288.71 mg, 4.01 mmol). After stirring for 10 min, 4-4-[4-(trifluoromethoxy)phenyl]-1,4-diazepan-1-yloxolan-2-one (344.0 mg, 999.04 µmol) was added to a solution and the resulting mixture was heated to 80°C for 8 hours. After cooling to room temperature, the solvent was evaporated *in vacuo* and the residue was purified by reverse preparative HPLC to afford N-(4-chlorophenyl)-4-hydroxy-3-4- [4-(trifluoromethoxy)phenyl]-1,4-diazepan-1-ylbutanamide (38.6 mg, 81.8 µmol, 8% yield). LC-MS (ESI-pos): [M+H]⁺ = 472.0/474.0

### The Synthesis of N-(4-chlorophenyl)-4-hydroxy-3-15-[4-(trifluoromethoxy)phenyl]-1H,2H,3H,4H,5H,6H-pyrrolo[3,4-c]pyrrol-2-yl}butanamide Example 4

### Step 1: Synthesis of tert-butyl 5-[4-(trifluoromethoxy)phenyl]-1H, 2H, 3H, 4H, 5H, 6H-pyrrolo[3,4-c]pyrrole-2-carboxylate

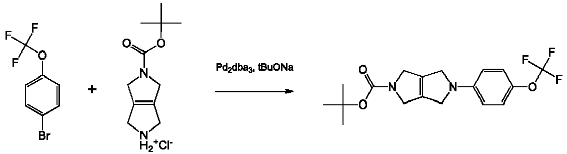

tert-Butyl 1H,2H,3H,4H,SH,6H-pyrrolo[3,4-c]pyrrole-2-carboxylate hydrochloride (2.0 g, 8.11 mmol), tris(1,5-diphenylpenta-1,4-dien-3-one) dipalladium (206.2 mg, 225.18 µmol), 1-[2-(diphenylphosphanyl)naphthalen-1-yl]naphthalen-2-yldiphenylphosphane (280.43 mg, 450.37 µmol) and sodium tert-butoxide (2.6 g, 27.02 mmol) were added consequently to a solution of 1-bromo-4-(trifluoromethoxy)benzene (2.17 g, 9.01 mmol) in toluene (70 mL) The mixture was degassed twice and refluxed under Ar atmosphere overnight. The solvent was evaporated to provide crude tert-butyl 5-[4-(trifluoromethoxy)phenyl]-1H,2H,3H,4H,5H,6H-pyrrolo[3,4-c]pyrrole-2-carboxylate (7.2 g, 19.44 mmol) as a residue. It was directly used for next step without further purification.

### Step 2: Synthesis of 2-[4-(trifluoromethoxy)phenyl]-1H,2H,3H,4H,5H,6H-pyrrolo[3,4-c]pyrrole

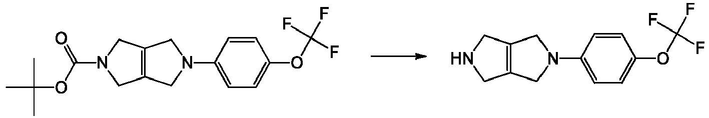

A mixture of crude tert-butyl 5-[4-(trifluoromethoxy)phenyl]-1H,2H,3H,4H,5H,6H-pyrrolo[3,4-c]pyrrole-2-carboxylate (3.34 g, 9.01 mmol) (obtained in previous step) in 45 mL of conc. HCl and 30 mL of DCM was stirred for 1.5 h at room temperature. After phase separation the DCM phase was discarded, and Na₂CO₃was added to the aqueous phase to pH ~9. Then 50 mL DCM were added then stirred for additional 0.5 h. The DCM phase was collected, dried over Na₂SO₄ and concentrated *in vacuo* to provide 2-[4-(trifluoromethoxy)phenyl]-1H,2H,3H,4H,5H,6H-pyrrolo[3,4-c]pyrrole (1.4 g, 5.18 mmol, 57.5% yield) as a white solid.

### Step 3: Synthesis of 4-[5-[4-(trifluoromethoxy)phenyl]-1H,2H,3H,4H,5H,6H-pyrrolo[3,4-c]pyrrol-2-yl}oxolan-2-one

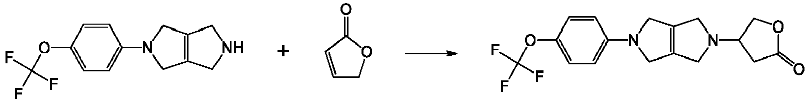

A mixture of 2-[4-(trifluoromethoxy)phenyl]-1H,2H,3H,4H,5H,6H-pyrrolo[3,4-c]pyrrole (1.4 g, 5.18 mmol) and 2,5-dihydrofuran-2-one (871.29 mg, 10.36 mmol) in MeOH (3.1 mL) was stirred at room temperature overnight The mixture was evaporated in *vacuo,* residue treated with water (20 mL), filtered, washed with water (2 × 20 mL), hexane (30 mL) and dried to afford 4-5-[4-(trifluoromethoxy)phenyl]-1H,2H,3H,4H,5H,6H-pyrrolo[3,4-c]pyrrol-2-yloxolan-2-one (1.6 g, 4.52 mmol, 87.1% yield) as a yellow solid.

### Step 4: The synthesis of N-(4-chlorophenyl)-4-hydroxy-3-[5-[4-(trifluoromethoxy)phenyl]-1H, 2H, 3H, 4H, 5H, 6H-pyrrolo[3,4-c]pyrrol-2-yl}butanamide

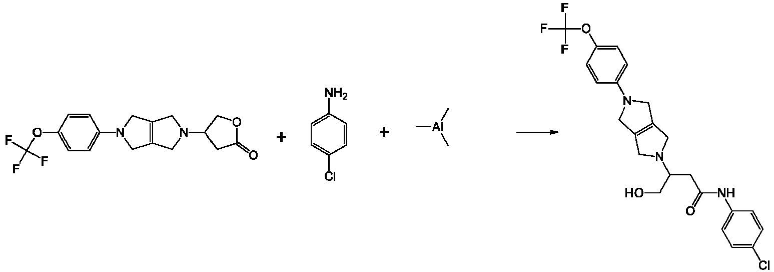

To a solution of 4-chloroaniline (254.67 mg, 2.0 mmol) in benzene (8 mL) was added trimethylaluminum (287.8 mg, 3.99 mmol). After stirring for 10 min, 4-5-[4-(trifluoromethoxy)phenyl]-1H,2H,3H,4H,5H,6H-pyrrolo[3,4-c]pyrrol-2-yloxolan-2-one (354.0 mg, 999.09 µmol) was added to a solution and the resulting mixture was heated to 50°C for 2.5 hours. After cooling to room temperature, water (3 mL) and EtOAc (15 mL) was added, filtered and the solvent was evaporated *in vacuo* , the residue was purified by reverse preparative HPLC to afford N-(4-chlorophenyl)-4-hydroxy-3-5-[4-(trifluoromethoxy)phenyl]-1H,2H,3H,4H,5H,6H-pyrrolo[3,4-c]pyrrol-2-ylbutanamide (186.0 mg, 385.98 µmol, 38% yield). LC-MS (ESI-pos): [M+H]⁺ = 482.2/484.0

### In a similar manner to the synthesis of Example 5 step 4 was synthesized (Example 29 to 34, 36-37, 42, 44)

| **Example** | **Compound** | **Analytical data** |
|---|---|---|
| **29** | **2-[1-(4-hydroxy-3-{5-[4-(trifluoromethoxy)phenyl]-1H,2H,3H,4H,5H,6H-pyrrolo[3,4-c]pyrrol-2-yl}butanoyl)pyrrolidin-3-yl]acetonitrile** | LC-MS (ESI): [M-H]⁻ / [M+H]⁺ = 463.2 / 465.2; Rt = 2.833 min |
| **30** | **N-(3-chlorophenyl)-4-hydroxy-3-{5-[4-(trifluoromethoxy)phenyl]-1H,2H,3H,4H,5H,6H-pyrrolo[3,4-c]pyrrol-2-yl}butanamide** | LC-MS (ESI): [M-H]⁻ / [M+H]⁺ = 480.2 / 482.2; Rt = 3.060 min |
| **31** | **4-hydroxy-N-(pyridin-3-yl)-3-{5-[4-(trifluoromethoxy)phenyl]-1H,2H,3H,4H,5H,6H-pyrrolo[3,4-c]pyrrol-2-yl}butanamide** | LC-MS (ESI): [M-H]⁻ / [M+H]⁺ = 447.2 / 449.2; Rt = 2.046 min |
| **32** | **4-hydroxy-N-(1,2-thiazol-5-yl)-3-{5-[4-(trifluoromethoxy)phenyl]-1H,2H,3H,4H,5H,6H-pyrrolo[3,4-c]pyrrol-2-yl}butanamide** | LC-MS (ESI): [M-H]⁻ / [M+H]⁺ = 453.2 / 455.2; Rt = 2.457 min |
| **33** | **N-[(dimethylcarbamoyl)methyl]-4-hydroxy-3-{5-[4-(trifluoromethoxy)phenyl]-1H,2H,3H,4H,5H,6H-pyrrolo[3,4-c]pyrrol-2-yl}butanamide** | LC-MS (ESI): [M-H]⁻ / [M+H]⁺ = 455.2 / 457.2; Rt = 2.259 min |
| **34** | **N-(2-chlorophenyl)-4-hydroxy-3-{5-[4-(trifluoromethoxy)phenyl]-1H,2H,3H,4H,5H,6H-pyrrolo[3,4-c]pyrrol-2-yl}butanamide** | LC-MS (ESI): [M-H]⁻ / [M+H]⁺ = 480.0 / 482.2; Rt = 3.166 min |
| **36** | **N-(4-cyanophenyl)-4-hydroxy-3-{5-[4-(trifluoromethoxy)phenyl]-1H,2H,3H,4H,5H,6H-pyrrolo[3,4-c]pyrrol-2-yl}butanamide** | LC-MS (ESI): [M-H]⁻ / [M+H]⁺ = 471.2 / 473.4; Rt = 2.656 min |
| **37** | **4-hydroxy-1-{1H,4H,SH,6H,7H-pyrazolo[4,3-c]pyridin-5-yl}-3-{5-[4-(trifluoromethoxy)phenyl]-1H,2H,3H,4H,5H,6H-pyrrolo[3,4-c]pyrrol-2-yl}butan-1-one** | LC-MS (ESI): [M-H]⁻ / [M+H]⁺ = 476.2 / 478.2; Rt = 1.954 min |
| **42** | **4-hydroxy-3-{5-[4-(trifluoromethoxy)phenyl]-1H,2H,3H,4H,5H,6H-pyrrolo[3,4-c]pyrrol-2-yl}-N-(2,4,6-trimethylphenyl)butanamide** | LC-MS (ESI): [M-H]⁻ / [M+H]⁺ = 488.2 / 490.2; Rt = 1.148 min |
| **44** | **4-hydroxy-1-{1H,4H,SH,6H,7H-pyrrolo[3,2-c]pyridin-5-yl}-3-{5-[4-(trifluoromethoxy)phenyl]-1H,2H,3H,4H,5H,6H-pyrrolo[3,4-c]pyrrol-2-yl}butan-1-one** | LC-MS (ESI): [M-H]⁻ / [M+H]⁺ = 476.2 / 477.2; Rt = 1.137 min |

### Synthesis of N-(4-chlorophenyl)-4-hydroxy-3-{2-[4-(trifluoromethoxy)phenyl]-6-oxa-2,9-diazaspiro[4.5]decan-9-yl}butanamide Example 5

### Step 1: Synthesis of tert-butyl 2-[4-(trifluoromethoxy)phenyl]-6-oxa-2,9-diazaspiro[4.5]decane-9-carboxylate

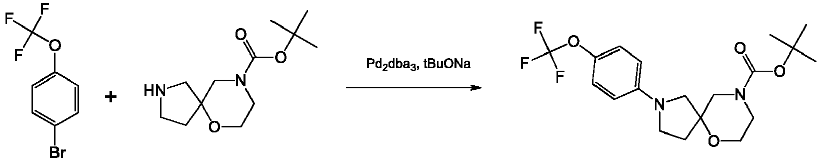

tert-Butyl 6-oxa-2,9-diazaspiro[4.5]decane-9-carboxylate (2.5 g, 10.32 mmol), tris(1,5-diphenylpenta-1,4-dien-3-one) dipalladium (262.43 mg, 286.59 µmol), 1-[2-(diphenylphosphanyl)naphthalen-1-yl]naphthalen-2-yldiphenylphosphane (356.9 mg, 573.17 µmol) and sodium tert-butoxide (2.2 g, 22.93 mmol) were added consequently to a solution of 1-bromo-4-(trifluoromethoxy)benzene (2.76 g, 11.46 mmol) in toluene (55 mL). The mixture was refluxed under Ar atmosphere overnight. The solvent was evaporated to provide crude tert-butyl 2-[4-(trifluoromethoxy)phenyl]-6-oxa-2,9-diazaspiro[4.5]decane-9-carboxylate (7.1 g, 17.64 mmol,) as a residue. It was used for next step without ANY purification.

### Step 2: Synthesis of 2-[4-(trifluoromethoxy)phenyl]-6-oxa-2,9-diazaspiro[4.5]decane

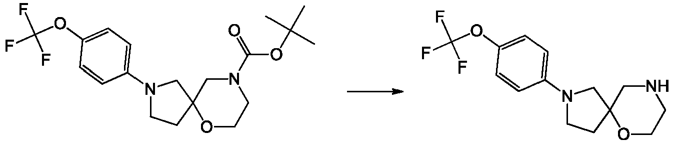

A solution of the crude tert-butyl 2-[4-(trifluoromethoxy)phenyl]-6-oxa-2,9-diazaspiro[4.5]decane-9-carboxylate (4.61 g, 11.46 mmol) (obtained in previous step) in 45 mL of conc. HCl and 30 mL of DCM was stirred for 1.5 h at room temperature. After phase separation the DCM phase was discarded, and the aqueous phase was evaporated in *vacuo* to dryness. The residue was dissolved in a mixture of 20 mL NaOH (2.0 M). Then DCM (50 mL) was add and the mixture was stirred for additional 0.5 h. The DCM phase was collected, dried over Na₂SO₄ and concentrated *in vacuo* to provide 2-[4-(trifluoromethoxy)phenyl]-6-oxa-2,9-diazaspiro[4.5]decane (2.3 g, 7.61 mmol, 66.4% yield) as yellow oil.

### Step 3: Synthesis of 4-{2-[4-(trifluoromethoxy)phenyl]-6-oxa-2,9-diazaspiro[4.5]decan-9-yl}oxolan-2-one

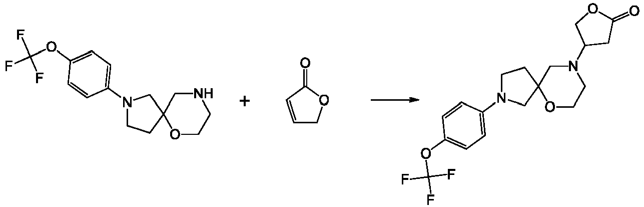

A mixture of 2-[4-(trifluoromethoxy)phenyl]-6-oxa-2,9-diazaspiro[4.5]decane (999.91 mg, 3.31 mmol) and 2,5-dihydrofuran-2-one (556.19 mg, 6.62 mmol) in MeOH (2.0 ml ) was stirred at room temperature overnight. The mixture was evaporated in *vacuo,* the residue was dissolved in MTBE (50 mL), washed with water (2 × 50 mL), brine (30 mL) and evaporated to afford 4-2-[4-(trifluoromethoxy)phenyl]-6-oxa-2,9-diazaspiro[4.5]decan-9-yloxolan-2-one (920.0 mg, 2.38 mmol, 72% yield) as a brown oil.

### Step 4: Synthesis of N-(4-chlorophenyl)-4-hydroxy-3-{2-(4-(trifluoromethoxy)phenyl]-6-oxa-2,9-diazaspiro[4.5]decan-9-yl}butanamide

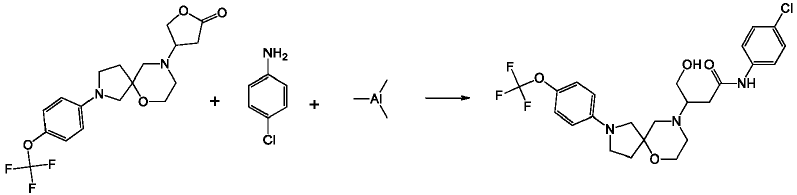

To a solution of 4-chloroaniline (255.57 mg, 2.0 mmol) in benzene (8 mL) was added trimethylaluminum (288.82 mg, 4.01 mmol, 2.0 ml). After stirring for 10 min, 4-2-[4-(trifluoromethoxy)phenyl]-6-oxa-2,9-diazaspiro[4.5]decan-9-yloxolan-2-one (387.0 mg, 1.0 mmol) was added to a solution and the resulting mixture was heated to 50°C for 2.5 hours. After cooling to room temperature, water (3 mL) and EtOAc (15 mL) was added, filtered and the solvent was evaporated *in vacuo* , the residue was purified by reverse preparative HPLC to afford N-(4-chlorophenyl)-4-hydroxy-3-2-[4-(trifluoromethoxy)phenyl]-6-oxa-2,9-diazaspiro[4.5]decan-9-ylbutanamide (184.0 mg, 358.02 µmol, 35.7% yield). LC-MS (ESI-pos): [M+H]⁺ = 514.2/516.0.

### In a similar manner to the synthesis of Example 5 step 4 was synthesized (Example 11 to 28, 35, 38 to 41, 43)

| **Example** | **Compound** | **LC-MS (ESI-pos): [M+H]⁺** |
|---|---|---|
| **11** | **N-[(dimethylcarbamoyl)methyl]-4-hydroxy-3-{2-[4-(trifluoromethoxy)phenyl]-6-oxa-2,9-diazaspiro[4.5]decan-9-yl}butanamide** | LC-MS (ESI): [M-H]⁻ / [M+H]⁺ = 487.2 / 489.2; Rt = 2.359 min |
| **12** | **3-(4-hydroxy-3-{2-[4-(trifluoromethoxy)phenyl]-6-oxa-2,9-diazaspiro[4.5]decan-9-yl}butanamido)pyrrolidine-1-carboxamide** | LC-MS (ESI): [M-H]⁻ / [M+H]⁺ = 514.4 / 516.4; Rt = 2.273 min |
| **13** | **N-[2-(dimethylcarbamoyl)ethyl]-4-hydroxy-3-{2-[4-(trifluoromethoxy)phenyl]-6-oxa-2,9-diazaspiro[4.5]decan-9-yl}butanamide** | LC-MS (ESI): [M-H]⁻ / [M+H]⁺ = 501.2 / 503.2; Rt = 2.396 min |
| **14** | **5-(4-hydroxy-3-{2-[4-(trifluoromethoxy)phenyl]-6-oxa-2,9-diazaspiro[4.5]decan-9-yl}butanamido)-1H-pyrazole-3-carboxamide** | LC-MS (ESI): [M-H]⁻ / [M+H]⁺ = 511.4 / 513.4; Rt = 2.297 min |
| **15** | **4-hydroxy-N-(2,2,2-trifluoroethyl)-3-{2-[4-(trifluoromethoxy)phenyl]-6-oxa-2,9-diazaspiro[4.5]decan-9-yl}butanamide** | LC-MS (ESI): [M-H]⁻ / [M+H]⁺ = 484.2 / 486.2; Rt = 1.161 min |
| **16** | **4-hydroxy-3-{2-[4-(trifluoromethoxy)phenyl]-6-oxa-2,9-diazaspiro[4.5]decan-9-yl}-N-[4-(trifluoromethyl)phenyl]butanamide** | LC-MS (ESI): [M-H]⁻ / [M+H]⁺ = 546.0 / 548.2; Rt = 3.855 min |
| **17** | ***N*-(5-chloropyridin-2-yl)-4-hydroxy-3-{2-[4-(trifluoromethoxy)phenyl]-6-oxa-2,9-diazaspiro[4.5]decan-9-yl}butanamide** | LC-MS (ESI): [M-H]⁻ / [M+H]⁺ = 513.2 / 515.2; Rt = 1.381min |
| **18** | **4-hydroxy-N-(3-methoxyphenyl)-3-{2-[4-(trifluoromethoxy)phenyl]-6-oxa-2,9-diazaspiro[4.5]decan-9-yl}butanamide** | LC-MS (ESI): [M-H]⁻ / [M+H]⁺ = 508.2 / 510.2; Rt = 2.319 min |
| **19** | ***N-*(4-acetamidophenyl)-4-hydroxy-3-{2-[4-(trifluoromethoxy)phenyl]-6-oxa-2,9-diazaspiro[4.5]decan-9-yl}butanamide** | LC-MS (ESI): [M-H]⁻ / [M+H]⁺ = 535.2 / 537.2; Rt = 2.796 min |
| **20** | ***N-*(3-acetamidophenyl)-4-hydroxy-3-{2-[4-(trifluoromethoxy)phenyl]-6-oxa-2,9-diazaspiro[4.5]decan-9-yl}butanamide** | LC-MS (ESI): [M-H]⁻ / [M+H]⁺ = 535.0 / 537.2; Rt = 2.843 min |
| **21** | **N-(4-cyanophenyl)-4-hydroxy-3-12-[4-(trifluoromethoxy)phenyl]-6-oxa-2,9-diazaspiro [4.5]decan-9-yl}butanamide** | LC-MS (ESI): [M-H]⁻ / [M+H]⁺ = 503.2 / 505.2; Rt = 3.049 min |
| **22** | ***N*-(2-chlorophenyl)-4-hydroxy-3-(2-[4-(trifluoromethoxy)phenyl]-6-oxa-2,9-diazaspiro[4.5]decan-9-yl}butanamide** | LC-MS (ESI): [M-H]⁻ / [M+H]⁺ = 512.2 / 514.2; Rt = 2.490 min |
| **23** | ***N*-(3-chlorophenyl)-4-hydroxy-3-{2-[4-(trifluoromethoxy)phenyl]-6-oxa-2,9-diazaspiro[4.5]decan-9-yl}butanamide** | LC-MS (ESI): [M-H]⁻ / [M+H]⁺ = 512.2 / 514.2; Rt = 3.229 min |
| **24** | ***N-*(4-fluorophenyl)-4-hydroxy-3-{2-[4-(trifluoromethoxy)phenyl]-6-oxa-2,9-diazaspiro[4.5]decan-9-yl}butanamide** | LC-MS (ESI): [M-H]⁻ / [M+H]⁺ = 496.4 / 498.4; Rt = 2.999 min |
| **25** | **1-(4-hydroxy-3-{2-[4-(trifluoromethoxy)phenyl]-6-oxa-2,9-diazaspiro[4.5]decan-9-yl}butanoyl)imidazolidin-4-one** | LC-MS (ESI): [M-H]⁻ / [M+H]⁺ = 471.2 / 473.2; Rt = 2.301 min |
| **26** | **4-hydroxy-N-(prop-2-en-1-yl)-3-12-[4-(trifluoromethoxy)phenyl]-6-oxa-2,9-diazaspiro[4.5]decan-9-yl}butanamide** | LC-MS (ESI): [M-H]⁻ / [M+H]⁺ = 442.0 / 444.2; Rt = 3.043 min |
| **27** | **4-hydroxy-1-(pyrazolidin-1-yl)-3-{2-[4-(trifluoromethoxy)phenyl]-6-oxa-2,9-diazaspiro[4.5]decan-9-yl}butan-1-one** | LC-MS (ESI): [M-H]⁻ / [M+H]⁺ = 457.2 / 459.2; Rt = 2.569 min |
| **28** | **4-hydroxy-1-(3-hydroxypyrrolidin-1-yl)-3-{2-[4-(trifluoromethoxy)phenyl]-6-oxa-2,9-diazaspiro[4.5]decan-9-yl}butan-1-one** | LC-MS (ESI): [M-H]⁻ / [M+H]⁺ = 473.2 / 474.2; Rt = 2.465 min |
| **35** | **4-hydroxy-N-(1H-pyrazol-1-yl)-3-{2-[4-(trifluoromethoxy)phenyl]-6-oxa-2,9-diazaspiro[4.5]decan-9-yl}butanamide** | LC-MS (ESI): [M-H]⁻ / [M+H]⁺ = 468.2 / 470.2; Rt = 3.059 min |
| **38** | **4-hydroxy-N-[(1r,4r)-4-cyanocyclohexyl]-3-{2-[4-(trifluoromethoxy)phenyl]-6-oxa-2,9-diazaspiro[4.5]decan-9-yl}butanamide** | LC-MS (ESI): [M-H]⁻ / [M+H]⁺ = 509.2 / 511.2; Rt = 2.260 min |
| **41** | **4-hydroxy-N-(pyridin-3-yl)-3-{2-[4-(trifluoromethoxy)phenyl]-6-oxa-2,9-diazaspiro[4.5]decan-9-yl}butanamide** | LC-MS (ESI): [M-H]⁻ / [M+H]⁺ = 479.2 / 481.2; Rt = 2.819 min |
| **43** | **4-hydroxy-N-(4-methanesulfonylphenyl)-3-{2-[4-(trifluoromethoxy)phenyl]-6-oxa-2,9-diazaspiro[4.5]decan-9-yl}butanamide** | LC-MS (ESI): [M-H]⁻ / [M+H]⁺ = 556.0 / 558.2; Rt = 3.059 min |

### Synthesis of N-(4-chlorophenyl)-4-hydroxy-3-(3-11-[4-(trifluoromethoxy)phenyl]azetidin-3-yl}piperidin-1-yl)butanamide Example 6

### Step 1: Synthesis of 3-{1-[4-(trifluoromethoxy)phenyl]azetidin-3-yl)piperidine

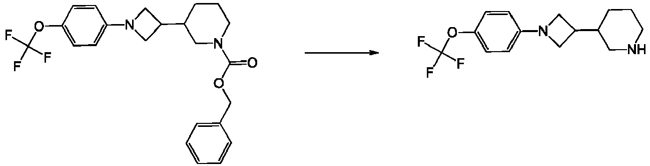

Benzyl 3-1-[4-(trifluoromethoxy)phenyl]azetidin-3-ylpiperidine-1-carboxylate (4.2 g, 9.67 mmol) was dissolved in MeOH (50 mL), 10% Pd/C (300 mg) was added and the reaction mixture was stirred overnight under H₂ atmosphere. The reaction mixture was filtered and evaporated under reduced pressure; the residue was treated with 10 N HCl (30 mL) and DCM (30 mL). Organic layer was diluted, aqueous layer was washed DCM (2x20 mL), and added 6N NaOH to pH ~10, the crude product was extracted with DCM (3 × 40 mL), organic layers were combined, dried over Na₂SO₄ and concentrated under reduced pressure to afford 3-1-[4-(trifluoromethoxy)phenyl]azetidin-3-ylpiperidine (800.0 mg, 2.66 mmol, 27.6% yield) as a brown oil.

### Step 2: Synthesis of 4-(3-{1-[4-(trifluoromethoxy)phenyl]azetidin-3-yl)piperidin-1-yl)oxolan-2-one

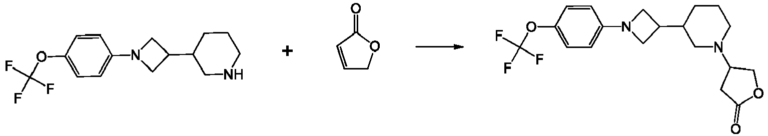

A mixture of 3-1-[4-(trifluoromethoxy)phenyl]azetidin-3-ylpiperidine (800.03 mg, 2.66 mmol) and 2,5-dihydrofuran-2-one (447.93 mg, 5.33 mmol) in MeOH (1.6 ml) was stirred at room temperature overnight The mixture was evaporated in *vacuo,the* residue was dissolved in MTBE (50 mL), washed with water (2x50 mL), brine (30 mL) and evaporated to afford 4-(3-1-[4-(trifluoromethoxy)phenyl]azetidin-3-ylpiperidin-1-yl)oxolan-2-one (600.0 mg, 1.56 mmol, 58.6% yield, purity -85%, mixture of diastereomers).

### Step 3: Synthesis of N-(4-chlorophenyl)-4-hydroxy-3-(3-{1-[4-(trifluoromethoxy)phenyl]azetidin-3-yl}piperidin-1-yl)butanamide

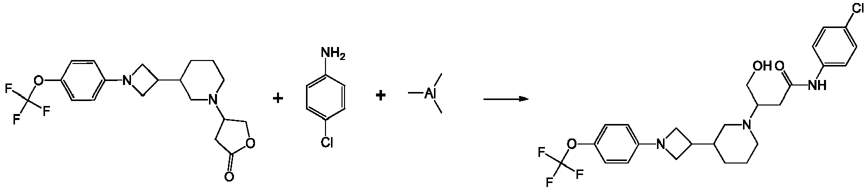

To a solution of 4-chloroaniline (398.69 mg, 3.13 mmol) in benzene (15 mL) was added trimethylaluminum ( g, mol) . After stirring for 10 min, 4-(3-1-[4-(trifluoromethoxy)phenyl]azetidin-3-ylpiperidin-1-yl)oxolan-2-one (600.0 mg, 1.56 mmol) was added to a solution and the resulting mixture was heated to 50°C for 2.5 hours. After cooling to room temperature, water (3 mL) and EtOAc (15 mL) was added, filtered and the solvent was evaporated *in vacuo,* the residue was purified by reverse preparative HPLC to afford N-(4-chlorophenyl)-4-hydroxy-3-(3-1-[4-(trifluoromethoxy)phenyl]azetidin-3-ylpiperidin-1-yl)butanamide (23.0 mg, 44.93 µmol, 3% yield). LC-MS (ESI-pos): [M+H]⁺ = 512.2/514.2

### Synthesis of N-(4-chlorophenyl)-4-hydroxy-3-(6-[4-(trifluoromethoxy)phenyl]-2,6-diazaspiro[3.4]octan-2-yl}butanamide Example 7

### Step 1: Synthesis of 4-{6-[4-(trifluoromethoxy)phenyl]-2,6-diazaspiro[3.4]octan-2-yl}oxolan-2-one

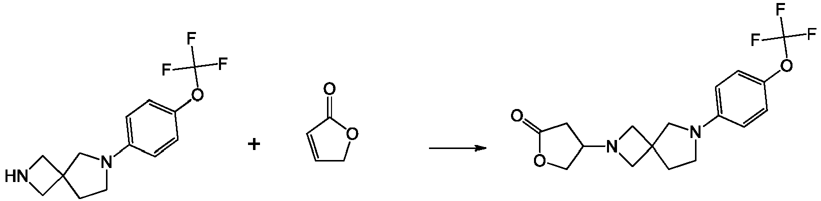

A mixture of 6-[4-(trifluoromethoxy)phenyl]-2,6-diazaspiro[3.4]octane (1.6 g, 5.88 mmol) and 2,5-dihydrofuran-2-one (1.24 g, 14.69 mmol) in 10 ml of EtOH was stirred at room temperature for 16 hours and concentrated under reduced pressure. A residue was dissolved in 50 ml of MTBE, washed with water (3 × 50 mL), brine (50 mL) and dried over sodium sulfate. The filtrate was concentrated under reduced pressure to afford 4-6-[4-(trifluoromethoxy)phenyl]-2,6-diazaspiro[3.4]octan-2-yloxolan-2-one (800.0 mg, 75.0% purity, 1.68 mmol, 28.7% yield) as a yellow solid which was used without purification.

### Step 2: Synthesis of N-(4-chlorophenyl)-4-hydroxy-3-{6-[4-(trifluoromethoxy)phenyl]-2,6-diazaspiro[3.4]octan-2-yl}butanamide

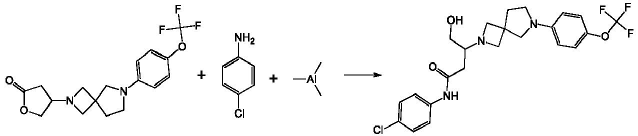

Trimethylaluminum (504.74 mg, 7.0 mmol, 3.5 ml) was added dropwise to a stirred solution of 4-chloroaniline (446.62 mg, 3.5 mmol) in 8 mL of benzene under argon. After 10 min of stirring 4-6-[4-(trifluoromethoxy)phenyl]-2,6-diazaspiro[3.4]octan-2-yloxolan-2-one (800.0 mg, 2.25 mmol) in 2 ml of benzene was added to the reaction dropwise and the mixture was stirred at 50 °C for 2 hours. The mixture was quenched with water (10 mL). The resulting slurry was diluted with EtOAc (40mL) and stirred rigorously for 15 min. Organic layer was separated, washed with brine, dried over sodium sulfate and concentrated under reduced pressure. A residue was purified by reverse preparative HPLC to afford N-(4-chlorophenyl)-4-hydroxy-3-6-[4-(trifluoromethoxy)phenyl]-2,6-diazaspiro[3.4]octan-2-ylbutanamide (150.0 mg, 95.0% purity, 294.48 µmol, 13.1% yield). LC-MS (ESI-pos): [M+H]⁺ = 484.2 / 486.2

### Synthesis of N-(4-chlorophenyl)-4-hydroxy-3-{1-[4-(trifluoromethoxy)phenyl]-octahydro-1H-pyrrolo[3,2-b]pyridin-4-yl}butanamide Example 8

### Step 1: Synthesis of 4-{1-[4-(trifluoromethoxy)phenyl]-octahydro-1H-pyrrolo[3,2-b]pyridin-4-yl}oxolan-2-one

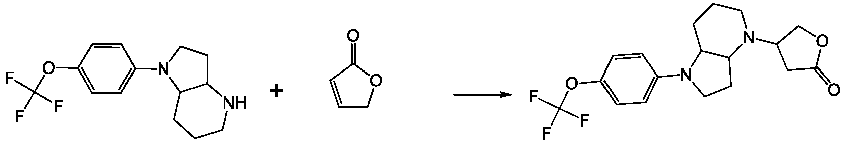

A mixture of 1-[4-(trifluoromethoxy)phenyl]-octahydro-1H-pyrrolo[3,2-b]pyridine (500.44 mg, 1.75 mmol) and 2,5-dihydrofuran-2-one (367.38 mg, 4.37 mmol, 310.0 µL) in 10 mL of EtOH was stirred at room temperature for 16 hours and concentrated under reduced pressure. A residue was dissolved in 80 mL of MTBE, washed with water (3 × 50 mL), brine (50 mL) and dried over sodium sulfate. The filtrate was concentrated under reduced pressure to afford 4-1-[4-(trifluoromethoxy)phenyl]-octahydro-1H-pyrrolo[3,2-b]pyridin-4-yloxolan-2-one (400.0 mg, 64.0% purity, 691.21 µmol, 39.5% yield) as a mixture of diastereomers which was used for the next step without purification.

### Step 2: Synthesis of N-(4-chlorophenyl)-4-hydroxy-3-{1-[4-(trifluoromethoxy)phenyl]-octahydro-1H-pyrrolo[3,2-b]pyridin-4-yl}butanamide

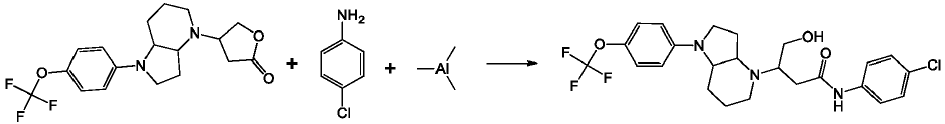

Trimethylaluminum (311.54 mg, 4.32 mmol, 2.16 mL) was added dropwise to a stirred solution of 4-chloroaniline (275.67 mg, 2.16 mmol) in 4 mL of benzene under argon. After 10 min of stirring 4-1-[4-(trifluoromethoxy)phenyl]-octahydro-1H-pyrrolo[3,2-b]pyridin-4-yloxolan-2-one (400.0 mg, 1.08 mmol) in 1 mL of benzene was added dropwise and the mixture was stirred at 50 °C for 2 hours. The mixture was quenched with water (10 mL). The resulting slurry was diluted with EtOAc (40 mL) and stirred rigorously for 15 min. Organic layer was separated, washed with brine, dried over sodium sulfate and concentrated under reduced pressure. The residue was purified by reverse preparative HPLC to afford N-(4-chlorophenyl)-4-hydroxy-3-1-[4-(trifluoromethoxy)phenyl]-octahydro-1H-pyrrolo[3,2-b]pyridin-4-ylbutanamide (77.0 mg, 154.64 µmol, 14.3% yield). LC-MS (ESI-pos): [M+H]⁺ = 498.2 / 500.2

### Synthesis of N-(4-chlorophenyl)-4-hydroxy-3-{5- [4-(trifluoromethoxy)phenyl] - octahydropyrrolo[3,4-c]pyrrol-2-yl}butanamide Example 9

### Step 1: Synthesis of tert-butyl 5-[4-(trifluoromethoxy)phenyl]-octahydropyrrolo[3,4-c]pyrrole-2-carboxylate

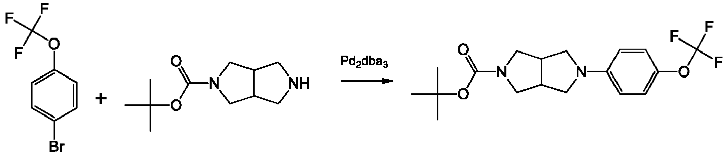

tert-butyl octahydropyrrolo[3,4-c]pyrrole-2-carboxylate (2.0 g, 9.42 mmol), tris(1,5-diphenylpenta-1,4-dien-3-one) dipalladium (239.64 mg, 261.69 µmol), 1-[2-(diphenylphosphanyl)naphthalen-1-yl]naphthalen-2-yldiphenylphosphane (325.9 mg, 523.39 µmol) and sodium tert-butoxide (2.01 g, 20.94 mmol) were added consequently to a solution of 1-bromo-4-(trifluoromethoxy)benzene (2.52 g, 10.47 mmol) in toluene (50 mL). The mixture was refluxed under argon overnight. The solvent was evaporated to provide crude tert-butyl 5-[4-(trifluoromethoxy)phenyl]-octahydropyrrolo[3,4-c]pyrrole-2-carboxylate (3.9g). It was used for next step without any purification.

### Step 2: Synthesis of 2-[4-(trifluoromethoxy)phenyl]-octahydropyrrolo[3,4-c]pyrrole

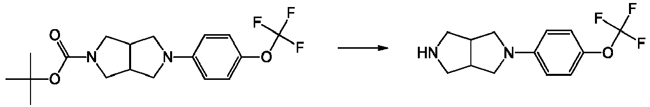

Crude tert-butyl 5 - [4-(trifluoromethoxy)phenyl] -octahydropyrrolo [3,4-c]pyrrole-2-carboxylate (3.9 g, 10.47 mmol) was dissolved in DCM (50 mL) and 50 mL of cone HCl was added slowly. The mixture was stirred at rt for 2 hours. Layers were separated; aqueous layer was washed with DCM (2 × 50 mL) and concentrated under reduced pressure. A residue was dissolved in 30 mL of water and basified with NaHCO₃. A resulting slurry was extracted with DCM (2 × 50 mL), organic phase was washed with brine, dried over Na₂SO₄ and concentrated under reduced pressure to afford 2-[4-(trifluoromethoxy)phenyl]-octahydropyrrolo[3,4-c]pyrrole (1.1 g, 3.84 mmol, 36.7% yield).

### Step 3: Synthesis of 4-{5-[4-(trifluoromethoxy)phenyl]-octahydropyrrolo[3,4-c]pyrrol-2-yl}oxolan-2-one

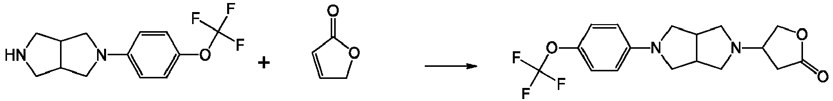

A mixture of 2-[4-(trifluoromethoxy)phenyl]-octahydropyrrolo[3,4-c]pyrrole (1.1 g, 4.04 mmol) and 2,5-dihydrofuran-2-one (1.02 g, 12.12 mmol) in 10 mL of EtOH was stirred at room temperature for 16 hours and concentrated under reduced pressure. A residue was dissolved in 80 mL of MTBE, washed with water (3 × 50 mL), brine (50 mL) and dried over sodium sulfate. The filtrate was concentrated under reduced pressure to afford 4-5-[4-(trifluoromethoxy)phenyl]-octahydropyrrolo[3,4-c]pyrrol-2-yloxolan-2-one (750.0 mg, 87.0% purity, 1.83 mmol, 45.3% yield) as a brown oil which was used without purification.

### Step 4: Synthesis of N-(4-chlorophenyl)-4-hydroxy-3-{5-(4-(trifluoromethoxy)phenyl]-octahydropyrrolo[3,4-c]pyrrol-2-yl}butanamide

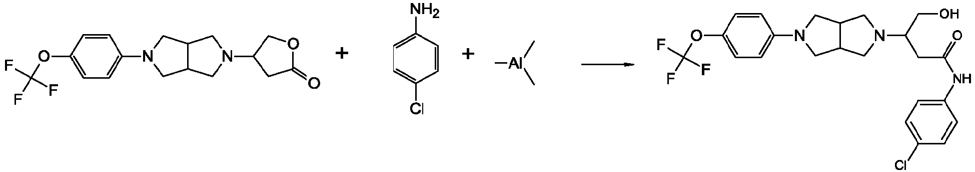

Trimethylaluminum (510.0 mg, 7.07 mmol, 3.54 ml) was added dropwise to a stirred solution of 4-chloroaniline (452.65 mg, 3.55 mmol) in 8 mL of benzene under argon. After 10 min of stirring 4-5-[4-(trifluoromethoxy)phenyl]-octahydropyrrolo[3,4-c]pyrrol-2-yloxolan-2-one (754.78 mg, 2.12 mmol) in 2 mL of benzene was added to the reaction dropwise and the mixture was stirred at 50 °C for 2 hours. The mixture was quenched with water (10 mL). The resulting slurry was diluted with EtOAc (40 mL) and stirred rigorously for 15 min. Organic layer was separated, washed with brine, dried over sodium sulfate and concentrated under reduced pressure. The residue was purified by reverse preparative HPLC to afford N-(4-chlorophenyl)-4-hydroxy-3-5-[4-(trifluoromethoxy)phenyl]-octahydropyrrolo[3,4-c]pyrrol-2-ylbutanamide (330.0 mg, 99.0% purity, 675.12 µmol, 31.9% yield). LC-MS (ESI-pos): [M+H]⁺ = 484.2 / 486.2

### Synthesis of N-(4-chlorophenyl)-4-hydroxy-3-{8-[4-(trifluoromethoxy)phenyl]-2,8-diazaspiro[5.5]undecan-2-yl}butanamide Example 10

### Step 1: Synthesis of 2-(4-(trifluoromethoxy)phenyl]-2,8-diazaspiro[5.5]undecane

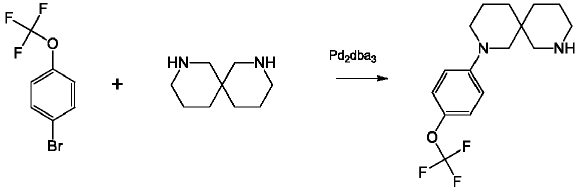

2,8-Diazaspiro[5.5]undecane (2.0 g, 12.97 mmol), tris(1,5-diphenylpenta-1,4-dien-3-one) dipalladium (296.84 mg, 324.17 µmol), 1-[2-(diphenylphosphanyl)naphthalen-1-yl]naphthalen-2-yldiphenylphosphane (403.7 mg, 648.33 µmol) and sodium tert-butoxide (2.49 g, 25.93 mmol) were added consequently to a solution of 1-bromo-4-(trifluoromethoxy)benzene (2.5 g, 10.37 mmol) in toluene (60 mL). The mixture was refluxed under Ar atmosphere overnight. The solvent was evaporated and the residue was treated a 45 mL of conc. HCl and 30 mL of DCM, then was stirred for 1.5 h at room temperature. After phase separation the DCM phase was discarded, and the aqueous phase was evaporated in *vacuo* to dryness. The residue was dissolved in 20 mL NaOH (2.0 M). Then DCM (50 mL) was added and the mixture was stirred for additional 0.5 h. The DCM phase was collected, dried over Na₂SO₄ and concentrated *in vacuo* to provide 2-[4-(trifluoromethoxy)phenyl]-2,8-diazaspiro[5.5]undecane (2.2 g, 7.0 mmol, 54% yield) as yellow oil. It was used for next step without any purification.

### Step 2: Synthesis of 4-{8-[4-(trifluoromethoxy)phenyl]-2,8-diazaspiro[5.5]undecan-2-yl}oxolan-2-one

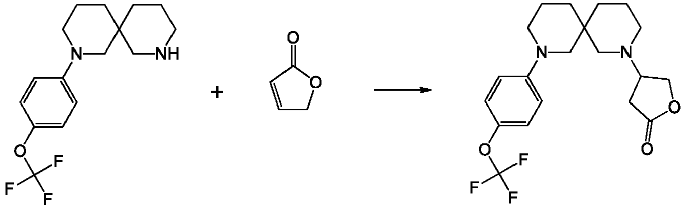

A mixture of 2-[4-(trifluoromethoxy)phenyl]-2,8-diazaspiro[5.5]undecane (1.1 g, 3.5 mmol) and 2,5-dihydrofuran-2-one (588.32 mg, 7.0 mmol) in MeOH (2.2 mL) was stirred at room temperature overnight. The mixture was concentrated in *vacuo,* residue dissolved in MTBE (50 mL), washed with water (2 × 50 mL), brine (30 mL) and concentrated under reduced pressure to afford 4-8-[4-(trifluoromethoxy)phenyl]-2,8-diazaspiro[5.5]undecan-2-yloxolan-2-one (950.0 mg, 2.38 mmol, 68.1% yield, purity 78%) as a brown oil.

### Step 3: Synthesis of N-(4-chlorophenyl)-4-hydroxy-3-{8-[4-(trifluoromethoxy)phenyl]-2,8-diazaspiro[5.5]undecan-2yl}butanamide

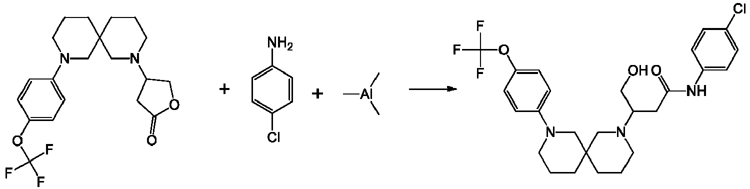

Trimethylaluminum (287.8 mg, 3.99 mmol) was added to a solution of 4-chloroaniline (254.66 mg, 2.0 mmol) in benzene (8 mL). After stirring for 10 min, 4-8-[4-(trifluoromethoxy)phenyl]-2,8-diazaspiro[5.5]undecan-2-yloxolan-2-one (398.0 mg, 998.95 µmol) was added to a solution and the resulting mixture was heated to 50°C for 2.5 hours. After cooling to room temperature, water (3 mL) and EtOAc (15 mL) was added, filtered and the solvent was evaporated *in vacuo* , the residue was purified by reverse preparative HPLC to afford N-(4-chlorophenyl)-4-hydroxy-3-8-[4-(trifluoromethoxy)phenyl]-2,8-diazaspiro[5.5]undecan-2-ylbutanamide (172.9 mg, 328.71 µmol, 17% yield). LC-MS (ESI-pos): [M+H]⁺ = 526.1 / 528.2.

## Claims

1. A compound of general formula I 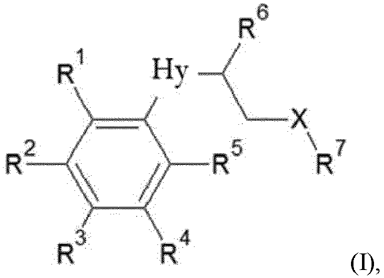 wherein
R¹-R⁵ are independently selected from the group consisting of hydrogen, halogen, CN, NO₂, CF₃, quaternary ammonium, COOH, COO-C₁₋₆ alkyl, OCF₃, SCF₃, SO₃-C₁₋₆ alkyl, provided that at least one of R¹-R⁵ is not hydrogen,
R⁶ is selected from C₁₋₃-R⁸, wherein R⁸ is selected from H, OH, SH, and NH₂,
R⁷ is selected from the group consisting of a) aryl substituted with one or more groups selected from hydrogen, halogen, CN, C₁₋₃ alkyl, NO₂, CONH₂, NHCO-C₁₋₃ alkyl, CF₃, tertiary ammonium, COOH, COO-C₁₋₆ alkyl, OC₁₋₃ alkyl, OCF₃, SCF₃, SO₂-C₁₋₃ alkyl, SO₃-C₁₋₃ alkyl, provided that at least one of said groups is not hydrogen; b) pyridyl optionally substituted with one or more groups selected from halogen, CN, C₁₋₃ alkyl, NO₂, CONH₂, NHCO-C₁₋₃ alkyl, CF₃, tertiary ammonium, COOH, COO-C₁₋₆ alkyl, OC₁₋₃ alkyl, OCF₃, SCF₃, SO₂-C₁₋₃ alkyl, SO₃-C₁₋₃ alkyl; c) pyrrolidinyl optionally substituted with one or more groups selected from halogen, CN, C₁₋₃ alkyl, NO₂, CONH₂, NHCO-C₁₋₃ alkyl, CF₃, tertiary ammonium, COOH, COO-C₁₋₆ alkyl, OC₁₋₃ alkyl, OCF₃, SCF₃, SO₂-C₁₋₃ alkyl, SO₃-C₁₋₃ alkyl; d) pyrazolyl optionally substituted with one or more groups selected from halogen, CN, C₁₋₃ alkyl, NO₂, CONH₂, NHCO-C₁₋₃ alkyl, CF₃, tertiary ammonium, COOH, COO-C₁₋₆ alkyl, OC₁₋₃ alkyl, OCF₃, SCF₃, SO₂-C₁₋₃ alkyl, SO₃-C₁₋₃ alkyl; e) thiazolyl optionally substituted with one or more groups selected from halogen, CN, C₁₋₃ alkyl, NO₂, CONH₂, NHCO-C₁₋₃ alkyl, CF₃, tertiary ammonium, COOH, COO-C₁₋₆ alkyl, OC₁₋₃ alkyl, OCF₃, SCF₃, SO₂-C₁₋₃ alkyl, SO₃-C₁₋₃ alkyl; f) C₅₋₇ cycloalkyl optionally substituted with one or more groups selected from halogen, CN, C₁₋₃ alkyl, NO₂, CONH₂, NHCO-C₁₋₃ alkyl, CF₃, tertiary ammonium, COOH, COO-C₁₋₆ alkyl, OC₁₋₃ alkyl, OCF₃, SCF₃, SO₂-C₁₋₃ alkyl, SO₃-C₁₋₃ alkyl; g) NHCONH₂; h) C₁₋₃ alkyl optionally substituted with a group selected from CONH₂, CON(CH₃)₂, CF₃, and C₂₋₄ alkene;
X is -CONH- or -NHCO-, or
X-R⁷ taken together is CONR'R", wherein R' and R" together with the nitrogen forms a monoheterocyclic ring or a biheterocyclic group selected from a) a monoheterocyclic ring having 4-7 ring atoms wherein 1-2 is selected from nitrogens and 0-2 from oxygens and the remaining ring atoms are carbon, and optionally substituted with a group selected from oxo, OH, C₁₋₃ alkyl-CN, or b) a biheterocyclic group having 7-10 ring atoms wherein 1-4 is selected from nitrogens and the remaining ring atoms are carbon, and optionally substituted with a group selected from oxo, OH, C₁₋₃ alkyl-CN;
Hy is a non-aromatic heterocyclic system selected from b) a biheterocyclic group having a first and a second heterocyclic ring which are fused, the biheterocyclic group having from 6 to 12 ring atoms wherein 2-4 ring atoms are N, 0-2 ring atoms is O, 0-2 ring atoms is S, and the remaining ring atoms are carbon, wherein the first heterocyclic ring is attached to the phenyl ring having substituents R¹-R⁵ of formula I, and the second heterocyclic ring is attached to the carbon atom linked to R⁶ and CH₂-X-R⁷ of formula I, c) a spiroheterocyclic group having a first and a second heterocyclic ring which are connected and share one carbon atom, the spiroheterocyclic group having from 6 to 12 ring atoms wherein 2-4 ring atoms are N, 0-2 ring atoms is O, 0-2 ring atoms is S, and the remaining ring atoms are carbon, wherein the first heterocyclic ring is attached to the phenyl ring having substituents R¹-R⁵ of formula I, and the second heterocyclic ring is attached to the carbon atom linked to R⁶ and CH₂-X-R⁷ of formula I, or d) a first and a second monoheterocyclic ring connected by a bond, wherein each monoheterocyclic ring is independently selected from a monoheterocyclic ring having 4-7 ring atoms, wherein 1-3 ring atoms are N, 0-1 ring atoms is O and 0-1 ring atoms is S, and the remaining ring atoms are carbon, wherein the first monoheterocyclic ring is attached to the phenyl ring having substituents R¹-R⁵ of formula I, and the second monoheterocyclic ring is attached to the carbon atom linked to R⁶ and CH₂-X-R⁷ of formula I; or a pharmaceutically acceptable salt or solvate thereof;
disclaiming 3-(4-hydroxy-3-{2-[4-(trifluoromethoxy)phenyl]-6-oxa-2,9-diazaspiro[4.5]decan-9-yl}butanamido)pyrrolidine-1-carboxamide.

2. The compound of claim 1 wherein R¹ is selected from halogen, CN, NO₂, CF₃, quaternary ammonium, COOH, COO-C₁₋₆ alkyl, OCF₃, SCF₃, SO₃-C₁₋₆ alkyl; and R²-R⁵ are hydrogen.

3. The compound of claim 1 or 2 wherein R² is selected from halogen, CN, NO₂, CF₃, quaternary ammonium, COOH, COO-C₁₋₆ alkyl, OCF₃, SCF₃, SO₃-C₁₋₆ alkyl; and R¹, R³-R⁵ are hydrogen.

4. The compound of any one of claims 1-3 wherein R³ is selected from halogen, CN, NO₂, CF₃, quaternary ammonium, COOH, COO-C₁₋₆ alkyl, OCF₃, SCF₃, SO₃-C₁₋₆ alkyl; and R¹, R², R⁴-R⁵ are hydrogen.

5. The compound of any one of claims 1-4 wherein R⁴ is selected from halogen, CN, NO₂, CF₃, quaternary ammonium, COOH, COO-C₁₋₆ alkyl, OCF₃, SCF₃, SO₃-C₁₋₆ alkyl; and R¹- R³, R⁵ are hydrogen.

6. The compound of any one of claims 1-5 wherein R⁵ is selected from halogen, CN, NO₂, CF₃, quaternary ammonium, COOH, COO-C₁₋₆ alkyl, OCF₃, SCF₃, SO₃-C₁₋₆ alkyl; and R¹-R⁴ are hydrogen.

7. The compound of any one of claims 1-6 wherein R⁶ is selected from C₁₋₃-R⁸, wherein R⁸ is OH.

8. The compound of any one of claims 1-7 wherein R⁷ is selected from the group consisting of a) a phenyl substituted with one or more groups selected from hydrogen, halogen, CN, C₁₋₃ alkyl, NO₂, CONH₂, NHCO-C₁₋₃ alkyl, CF₃, tertiary ammonium, COOH, COO-C₁₋₆ alkyl, OC₁₋₃ alkyl, OCF₃, SCF₃, SO₂-C₁₋₃ alkyl, SO₃-C₁₋₃ alkyl, provided that at least one of said groups is not hydrogen; typically, phenyl substituted with a group selected from halogen, CF₃, OCH₃, NHCOCH₃, CN, CH₃, and SO₂CH₃, b) a pyridyl optionally substituted with one or more halogen; c) a pyrrolidinyl substituted with one or more CONH₂; d) a pyrazolyl optionally substituted with one or more CONH₂; e) a thiazolyl; f) a cyclohexyl substituted with one or more CN alkyl; g) a NHCONH₂; and h) a C₁₋₂ alkyl substituted with a group selected from CON(CH₃)₂, CF₃, and C₂ alkene.

9. The compound of any one of claims 1-7 wherein X-R⁷ taken together is CONR'R", wherein R' and R" together with the nitrogen forms a monoheterocyclic ring or a biheterocyclic group selected from a) a monoheterocyclic group having 5 ring atoms wherein 1-2 is selected from nitrogens and the remaining ring atoms are carbon, and optionally substituted with a group selected from oxo, OH, CH₂-CN, or b) a biheterocyclic group having 9-10 ring atoms wherein 1-3 is selected from nitrogens and the remaining ring atoms are carbon.

10. The compound of any one of claims 1-9 wherein Hy is a non-aromatic cyclic system selected from b) wherein the biheterocyclic group has from 8 to 11 ring atoms wherein 1-2 is N, 0-1 is O and the remaining ring atoms are carbon.

11. The compound of any one of claims 1-9 wherein Hy is a non-aromatic cyclic system selected from c) wherein the spiroheterocyclic group has from 8 to 11 ring atoms wherein 2-3 ring atoms are N, 0-1 ring atoms is O, and the remaining ring atoms are carbon.

12. The compound of any one of claims 1-9 wherein Hy is a non-aromatic cyclic system selected from d) wherein the first and second monoheterocyclic ring is connected by a bond, and the first monoheterocyclic ring has from 4-6 ring atoms, wherein 1-2 is N and 0-1 is O, and the remaining ring atoms are carbon, and the second monoheterocyclic ring has from 4-6 ring atoms, wherein 1-2 is N and 0-1 is O, and the remaining ring atoms are carbon.

13. The compound of claim 1 selected from any one of
N (4-Chlorophenyl)-4-hydroxy-3- f 5-[4-(trifluoromethoxy)phenyl]-1H,2H,3H,4H,5H,6H-pyrrolo[3,4-c]pyrrol-2-yl}butanamide,
N (4-Chlorophenyl)-4-hydroxy-3- f 2-[4-(trifluoromethoxy)phenyl]-6-oxa-2,9-diazaspiro[4.5]decan-9-yl}butanamide,
*N*-(4-Chlorophenyl)-4-hydroxy-3-(3-{1-[4-(trifluoromethoxy)phenyl]azetidin-3-yl}piperidin-1-yl)butanamide,
*N*-(4-Chlorophenyl)-4-hydroxy-3-{6-[4-(trifluoromethoxy)phenyl]-2,6-diazaspiro[3.4]octan-2-yl}butanamide,
N (4-Chlorophenyl)-4-hydroxy-3- f 1-[4-(trifluoromethoxy)phenyl]-octahydro-1H-pyrrolo[3,2-b]pyridin-4-yl}butanamide,
N (4-Chlorophenyl)-4-hydroxy-3- f 5-[4-(trifluoromethoxy)phenyl]-octahydropyrrolo[3,4-c]pyrrol-2-yl}butanamide,
N (4-Chlorophenyl)-4-hydroxy-3- f 8-[4-(trifluoromethoxy)phenyl]-2,8-diazaspiro[5.5]undecan-2-yl}butanamide,
*N*-[(Dimethylcarbamoyl)methyl]-4-hydroxy-3-{2-[4-(trifluoromethoxy)phenyl]-6-oxa-2,9-diazaspiro[4.5]decan-9-yl}butanamide,
N [2-(Dimethylcarbamoyl)ethyl]-4-hydroxy-3- f 2-[4-(trifluoromethoxy)phenyl]-6-oxa-2,9-diazaspiro[4.5]decan-9-yl}butanamide,
5-(4-Hydroxy-3-{2-[4-(trifluoromethoxy)phenyl]-6-oxa-2,9-diazaspiro[4.5]decan-9-yl}butanamido)-1H-pyrazole-3-carboxamide,
4-Hydroxy-N (2,2,2-trifluoroethyl)-3- f 2-[4-(trifluoromethoxy)phenyl]-6-oxa-2,9-diazaspiro[4.5]decan-9-yl}butanamide,
4-Hydroxy-3-{2-[4-(trifluoromethoxy)phenyl]-6-oxa-2,9-diazaspiro[4.5]decan-9-yl}-N-[4-(trifluoromethyl)phenyl]butanamide,
*N*-(5-Chloropyridin-2-yl)-4-hydroxy-3-{2-[4-(trifluoromethoxy)phenyl]-6-oxa-2,9-diazaspiro[4.5]decan-9-yl}butanamide,
4-Hydroxy-N (3-methoxyphenyl)-3- f 2-[4-(trifluoromethoxy)phenyl]-6-oxa-2,9-diazaspiro[4.5]decan-9-yl}butanamide,
N (4-Acetamidophenyl)-4-hydroxy-3- f 2-[4-(trifluoromethoxy)phenyl]-6-oxa-2,9-diazaspiro[4.5]decan-9-yl}butanamide,
N (3-Acetamidophenyl)-4-hydroxy-3- f 2-[4-(trifluoromethoxy)phenyl]-6-oxa-2,9-diazaspiro[4.5]decan-9-yl}butanamide,
*N*-(4-Cyanophenyl)-4-hydroxy-3-{2-[4-(trifluoromethoxy)phenyl]-6-oxa-2,9-diazaspiro[4.5]decan-9-yl}butanamide,
*N*-(2-Chlorophenyl)-4-hydroxy-3-{2-[4-(trifluoromethoxy)phenyl]-6-oxa-2,9-diazaspiro[4.5]decan-9-yl}butanamide,
*N-*(3-Chlorophenyl)-4-hydroxy-3-{2-[4-(trifluoromethoxy)phenyl]-6-oxa-2,9-diazaspiro[4.5]decan-9-yl}butanamide,
*N-*(4-Fluorophenyl)-4-hydroxy-3-{2-[4-(trifluoromethoxy)phenyl]-6-oxa-2,9-diazaspiro[4.5]decan-9-yl}butanamide,
1-(4-Hydroxy-3-{2-[4-(trifluoromethoxy)phenyl]-6-oxa-2,9-diazaspiro[4.5]decan-9-yl }butanoyl)imidazolidin-4-one,
4-Hydroxy-*N-*(prop-2-en-1-yl)-3-{2-[4-(trifluoromethoxy)phenyl]-6-oxa-2,9-diazaspiro[4.5]decan-9-yl}butanamide,
4-Hydroxy-1-(pyrazolidin-1-yl)-3-{2-[4-(trifluoromethoxy)phenyl]-6-oxa-2,9-diazaspiro[4.5]decan-9-yl}butan-1-one,
4-Hydroxy-1-(3-hydroxypyrrolidin-1-yl)-3-{2-[4-(trifluoromethoxy)phenyl]-6-oxa-2,9-diazaspiro[4.5]decan-9-yl}butan-1-one,
2-[1-(4-Hydroxy-3-{5-[4-(trifluoromethoxy)phenyl]-1H,2H,3H,4H,5H,6H-pyrrolo[3,4-c]pyrrol-2-yl}butanoyl)pyrrolidin-3-yl]acetonitrile,
N-(3 -chlorophenyl)-4-hydroxy-3 - { 5-[4-(trifluoromethoxy)phenyl]-1H,2H,3H,4H,5H,6H-pyrrolo[3,4-c]pyrrol-2-yl}butanamide,
4-Hydroxy-jV-(pyridin-3-yl)-3-{5-[4-(trifluoromethoxy)phenyl]-1H,2H,3H,4H,5H,6H-pyrrolo[3,4-c]pyrrol-2-yl}butanamide,
4-Hydroxy-*N*-(1,2-thiazol-5-yl)-3-{5-[4-(trifluoromethoxy)phenyl]-1H,2H,3H,4H,5H,6H-pyrrolo[3,4-c]pyrrol-2-yl}butanamide,
*N*-[(Dimethylcarbamoyl)methyl]-4-hydroxy-3-{5-[4-(trifluoromethoxy)phenyl]-1H,2H,3H,4H,5H,6H-pyrrolo[3,4-c]pyrrol-2-yl}butanamide,
N (2-Chlorophenyl)-4-hydroxy-3- f 5-[4-(trifluoromethoxy)phenyl]-1H,2H,3H,4H,5H,6H-pyrrolo[3,4-c]pyrrol-2-yl}butanamide,
4-Hydroxy-N (1H-pyrazol-1-yl)-3- f 2-[4-(trifluoromethoxy)phenyl]-6-oxa-2,9-diazaspiro[4.5]decan-9-yl}butanamide,
*N-*(4-Cyanophenyl)-4-hydroxy-3-{5-[4-(trifluoromethoxy)phenyl]-1H,2H,3H,4H,5H,6H-pyrrolo[3,4-c]pyrrol-2-yl}butanamide,
4-Hydroxy-1-{1H,4H,5H,6H,7H-pyrazolo[4,3-c]pyridin-5-yl}-3-{5-[4-(trifluoromethoxy)phenyl]-1H,2H,3H,4H, SH,6H-pyrrolo[3,4-c]pyrrol-2-yl }butan-1-one,
4-Hydroxy-*N*-[(1r,4r)-4-cyanocyclohexyl]-3-{2-[4-(trifluoromethoxy)phenyl]-6-oxa-2,9-diazaspiro[4.5]decan-9-yl}butanamide,
N (Carbamoylamino)-4-hydroxy-3- f 2-[4-(trifluoromethoxy)phenyl]-6-oxa-2,9-diazaspiro[4.5]decan-9-yl}butanamide,
4-Hydroxy-jV-(pyridin-2-yl)-3-{2-[4-(trifluoromethoxy)phenyl]-6-oxa-2,9-diazaspiro[4.5]decan-9-yl}butanamide,
4-Hydroxy-*N*-(pyridin-3-yl)-3-{2-[4-(trifluoromethoxy)phenyl]-6-oxa-2,9-diazaspiro[4.5]decan-9-yl}butanamide,
4-Hydroxy-3-{5-[4-(trifluoromethoxy)phenyl]-1H,2H,3H,4H,5H,6H-pyrrolo[3,4-c]pyrrol-2-yl}-A-(2,4,6-trimethylphenyl)butanamide,
4-Hydroxy-*N*-(4-methanesulfonylphenyl)-3-{2-[4-(trifluoromethoxy)phenyl]-6-oxa-2,9-diazaspiro[4.5]decan-9-yl }butanamide,
4-Hydroxy-1-1H,4H,5H,6H,7H-pyrrolo[3,2-c]pyridin-5-yl}-3-{5-[4-(trifluoromethoxy)phenyl]-1H,2H,3H,4H, SH,6H-pyrrolo[3,4-c]pyrrol-2-yl }butan-1-one; or
a pharmaceutically acceptable salt or solvate thereof.

14. A compound of any one of claims 1-13 for use in a method for treating diabetes or pre-diabetes, such as diabetes type 2, in a subject in need thereof.

15. A pharmaceutical composition comprising the compound of any one of claims 1-13 and optionally a pharmaceutically acceptable additive.

## Patentansprüche

1. Eine Verbindung der allgemeinen Formel I 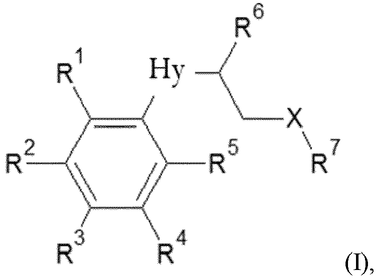 wobei
R¹-R⁵ unabhängig voneinander ausgewählt sind aus der Gruppe bestehend aus Wasserstoff, Halogen, CN, NO₂, CF₃, quartärem Ammonium, COOH, COO-C₁₋₆-Alkyl, OCF₃, SCF₃, SO₃-C₁₋₆ -Alkyl, unter der Voraussetzung, dass mindestens eines von R¹-R⁵ nicht Wasserstoff ist,
R⁶ ausgewählt ist aus C₁₋₃-R⁸, wobei R⁸ ausgewählt ist aus H, OH, SH und NH₂,
R⁷ ausgewählt ist aus der Gruppe bestehend aus a) Aryl, das mit einer oder mehreren Gruppen substituiert ist, die ausgewählt sind aus Wasserstoff, Halogen, CN, C₁₋₃-Alkyl, NO₂, CONH₂, NHCO-C₁₋₃-Alkyl, CF₃, tertiärem Ammonium, COOH, COO-C₁₋₆-Alkyl, OC₁₋₃-Alkyl, OCF₃, SCF₃, SO₂-C₁₋₃-Alkyl, SO₃-C₁₋₃-Alkyl, unter der Voraussetzung, dass mindestens eine der Gruppen nicht Wasserstoff ist; b) Pyridyl, wahlweise substituiert mit einer oder mehreren Gruppen, die ausgewählt sind aus Halogen, CN, C₁₋₃-Alkyl, NO₂, CONH₂, NHCO-C₁₋₃-Alkyl, CF₃, tertiärem Ammonium, COOH, COO-C₁₋₆-Alkyl, OC₁₋₃-Alkyl, OCF₃, SCF₃, SO₂-C₁₋₃-Alkyl, SO₃-C₁₋₃-Alkyl, c) Pyrrolidinyl, wahlweise substituiert mit einer oder mehreren Gruppen, die ausgewählt sind aus Halogen, CN, C₁₋₃-Alkyl, NO₂, CONH₂, NHCO-C₁₋₃-Alkyl, CF₃, tertiärem Ammonium, COOH, COO-C₁₋₆-Alkyl, OC₁₋₃-Alkyl, OCF₃, SCF₃, SO₂-C₁₋₃-Alkyl, SO₃-C₁₋₃-Alkyl, d) Pyrazolyl, wahlweise substituiert mit einer oder mehreren Gruppen, die ausgewählt sind aus Halogen, CN, C₁₋₃-Alkyl, NO₂, CONH₂, NHCO-C₁₋₃-Alkyl, CF₃, tertiärem Ammonium, COOH, COO-C₁₋₆ -Alkyl, OCF₃, SCF₃, SO₂-C₁₋₃-Alkyl, SO₃-C₁₋₃-Alkyl, e) Thiazolyl, wahlweise substituiert mit einer oder mehreren Gruppen, die ausgewählt sind aus Halogen, CN, C₁₋₃-Alkyl, NO₂, CONH₂, NHCO-C₁₋₃-Alkyl, CF₃, tertiärem Ammonium, COOH, COO-C₁₋₆-Alkyl, OC₁₋₃-Alkyl, OCF₃, SCF₃, SO₂-C₁₋₃ -Alkyl, SO₃-C₁₋₃-Alkyl; f) C₅₋₇-Cycloalkyl, wahlweise substituiert mit einer oder mehreren Gruppen, die ausgewählt sind aus Halogen, CN, C₁₋₃-Alkyl, NO₂, CONH₂, NHCO-C₁₋₃-Alkyl, CF₃, tertiärem Ammonium, COOH, COO-C₁₋₆-Alkyl, OC₁₋₃-Alkyl, OCF₃, SCF₃, SO₂-C₁₋₃-Alkyl, SO₃-C₁₋₃-Alkyl, g) NHCONH₂; h) C₁₋₃-Alkyl, wahlweise substituiert mit einer Gruppe, die ausgewählt ist aus CONH₂, CON(CH₃)₂, CF₃ und C₂₋₄-Alken;
X -CONH- oder -NHCO- ist, oder
X-R⁷ zusammengenommen CONR'R" ist, wobei R' und R" zusammen mit dem Stickstoff einen monoheterocyclischen Ring oder eine biheterocyclische Gruppe bilden, die ausgewählt ist aus a) einem monoheterocyclischen Ring mit 4-7 Ringatomen, wobei 1-2 ausgewählt ist aus Stickstoffatomen und 0-2 aus Sauerstoffatomen und die verbleibenden Ringatome Kohlenstoff sind und wahlweise mit einer Gruppe substituiert sind, die ausgewählt ist aus Oxo, OH, C₁₋₃-Alkyl-CN, oder b) einer biheterocyclischen Gruppe mit 7-10 Ringatomen, wobei 1-4 ausgewählt ist aus Stickstoffatomen und die verbleibenden Ringatome Kohlenstoff sind und wahlweise mit einer Gruppe substituiert sind, die ausgewählt ist aus Oxo, OH, C₁₋₃-Alkyl-CN;
Hy ein nicht-aromatisches heterocyclisches System ist, das ausgewählt ist aus b) einer biheterocyclischen Gruppe mit einem ersten und einem zweiten heterocyclischen Ring, die kondensiert sind, wobei die biheterocyclische Gruppe 6 bis 12 Ringatome aufweist, wobei 2-4 Ringatome N sind, 0-2 Ringatome O sind, 0-2 Ringatome S sind und die verbleibenden Ringatome Kohlenstoff sind, wobei der erste heterocyclische Ring an den Phenylring mit den Substituenten R¹-R⁵ der Formel I gebunden ist und der zweite heterocyclische Ring an das Kohlenstoffatom gebunden ist, das an R⁶ und CH₂-X-R⁷ der Formel I gebunden ist, c) einer spiroheterocyclischen Gruppe mit einem ersten und einem zweiten heterocyclischen Ring, die verbunden sind und sich ein Kohlenstoffatom teilen, wobei die spiroheterocyclische Gruppe 6 bis 12 Ringatome aufweist, wobei 2-4 Ringatome N sind, 0-2 Ringatome O sind, 0-2 Ringatome S sind und die verbleibenden Ringatome Kohlenstoff sind, wobei der erste heterocyclische Ring an den Phenylring mit den Substituenten R¹-R⁵ der Formel I gebunden ist und der zweite heterocyclische Ring an das Kohlenstoffatom gebunden ist, das an R⁶ und CH₂-X-R⁷ der Formel I gebunden ist, oder d) einem ersten und einem zweiten monoheterozyklischen Ring, die durch eine Bindung verbunden sind, wobei jeder monoheterozyklische Ring unabhängig ausgewählt ist aus einem monoheterozyklischen Ring mit 4-7 Ringatomen, wobei 1-3 Ringatome N sind, 0-1 Ringatome O sind und 0-1 Ringatome S sind und die verbleibenden Ringatome Kohlenstoff sind, wobei der erste monoheterozyklische Ring an den Phenylring mit den Substituenten R¹-R⁵ der Formel I gebunden ist und der zweite monoheterozyklische Ring an das Kohlenstoffatom gebunden ist, das an R⁶ und CH₂-X-R⁷ der Formel I gebunden ist; oder ein pharmazeutisch akzeptables Salz oder Solvat davon;
unter Ablehnung von 3-(4-Hydroxy-3-{2-[4-(trifluormethoxy)phenyl]-6-oxa-2,9-diazaspiro[4.5]decan-9-yl}butanamido)pyrrolidin-1-carboxamid.

2. Die Verbindung von Anspruch 1, wobei R¹ ausgewählt ist aus Halogen, CN, NO₂, CF₃, quartärem Ammonium, COOH, COO-C₁₋₆ -Alkyl, OCF₃, SCF₃, SO₃-C₁₋₆ -Alkyl; und R²-R⁵ Wasserstoff sind.

3. Die Verbindung von Anspruch 1 oder 2, wobei R² ausgewählt ist aus Halogen, CN, NO₂, CF₃, quaternärem Ammonium, COOH, COO-C₁₋₆ -Alkyl, OCF₃, SCF₃, SO₃-C₁₋₆ -Alkyl; und R¹, R³-R⁵ Wasserstoff sind.

4. Die Verbindung von einem der Ansprüche 1 bis 3, wobei R³ ausgewählt ist aus Halogen, CN, NO₂, CF₃, quartärem Ammonium, COOH, COO-C₁₋₆-Alkyl, OCF₃, SCF₃, SO₃-C₁₋₆ -Alkyl; und R¹, R², R⁴-R⁵ Wasserstoff sind.

5. Die Verbindung von einem der Ansprüche 1 bis 4, wobei R⁴ ausgewählt ist aus Halogen, CN, NO₂, CF₃, quartärem Ammonium, COOH, COO-C₁₋₆-Alkyl, OCF₃, SCF₃, SO₃-C₁₋₆ -Alkyl; und R¹- R³, R⁵ Wasserstoff sind.

6. Die Verbindung von einem der Ansprüche 1 bis 5, wobei R⁵ ausgewählt ist aus Halogen, CN, NO₂, CF₃, quartärem Ammonium, COOH, COO-C₁₋₆-Alkyl, OCF₃, SCF₃, SO₃-C₁₋₆ -Alkyl; und R¹-R⁴ Wasserstoff sind.

7. Die Verbindung von einem der Ansprüche 1 bis 6, wobei R⁶ ausgewählt ist aus C₁₋₃-R⁸, wobei R⁸ OH ist.

8. Die Verbindung von einem der Ansprüche 1 bis 7, wobei R⁷ ausgewählt ist aus der Gruppe bestehend aus a) einem Phenyl, das mit einer oder mehreren Gruppen substituiert ist, die ausgewählt sind aus Wasserstoff, Halogen, CN, C₁₋₃ -Alkyl, NO₂, CONH₂, NHCO-C₁₋₃ -Alkyl, CF₃, tertiärem Ammonium, COOH, COO-C₁₋₆ -Alkyl, OC₁₋₃-Alkyl, OCF₃, SCF₃, SO₂-C₁₋₃ -Alkyl, SO₃-C₁₋₃ -Alkyl, unter der Voraussetzung, dass mindestens eine der Gruppen nicht Wasserstoff ist; typischerweise Phenyl, das mit einer Gruppe substituiert ist, die ausgewählt ist aus Halogen, CF₃, OCH₃, NHCOCH₃, CN, CH₃ und SO₂CH₃, b) einem Pyridyl, das wahlweise mit einem oder mehreren Halogenen substituiert ist; c) einem Pyrrolidinyl, das mit einem oder mehreren CONH₂ substituiert ist; d) einem Pyrazolyl, das wahlweise mit einem oder mehreren CONH₂ substituiert ist; e) einem Thiazolyl; f) einem Cyclohexyl, das mit einem oder mehreren CN-Alkyl substituiert ist; g) einem NHCONH₂; und h) einem C₁₋₂ -Alkyl, das mit einer Gruppe substituiert ist, die aus CON(CH₃)₂, CF₃ und C₂ - Alken ausgewählt ist.

9. Die Verbindung von einem der Ansprüche 1 bis 7, wobei X-R⁷ zusammengenommen CONR'R" ist, wobei R' und R" zusammen mit dem Stickstoff einen monoheterocyclischen Ring oder eine biheterocyclische Gruppe bilden, die ausgewählt ist aus a) einer monoheterocyclischen Gruppe mit 5 Ringatomen, wobei 1-2 aus Stickstoff ausgewählt ist und die verbleibenden Ringatome Kohlenstoff sind, und wahlweise substituiert mit einer Gruppe, die ausgewählt ist aus Oxo, OH, CH₂-CN, oder b) einer biheterocyclischen Gruppe mit 9-10 Ringatomen, wobei 1-3 aus Stickstoff ausgewählt ist und die verbleibenden Ringatome Kohlenstoff sind.

10. Die Verbindung von einem der Ansprüche 1 bis 9, wobei Hy ein nicht-aromatisches zyklisches System ist, das aus b) ausgewählt ist, wobei die biheterozyklische Gruppe von 8 bis 11 Ringatome hat, wobei 1-2 N ist, 0-1 O ist und die verbleibenden Ringatome Kohlenstoff sind.

11. Die Verbindung von einem der Ansprüche 1 bis 9, wobei Hy ein nicht-aromatisches zyklisches System ist, das aus c) ausgewählt ist, wobei die spiroheterozyklische Gruppe von 8 bis 11 Ringatome aufweist, wobei 2-3 Ringatome N sind, 0-1 Ringatome O sind und die verbleibenden Ringatome Kohlenstoff sind.

12. Die Verbindung von einem der Ansprüche 1 bis 9, wobei Hy ein nicht-aromatisches cyclisches System ist, ausgewählt aus d) wobei der erste und zweite monoheterocyclische Ring durch eine Bindung verbunden ist und der erste monoheterocyclische Ring 4-6 Ringatome aufweist, wobei 1-2 N ist und 0-1 O ist und die verbleibenden Ringatome Kohlenstoff sind und der zweite monoheterocyclische Ring 4-6 Ringatome aufweist, wobei 1-2 N ist und 0-1 O ist und die verbleibenden Ringatome Kohlenstoff sind.

13. Die Verbindung von Anspruch 1, ausgewählt aus einem der folgenden:
N (4-Chlorphenyl)-4-hydroxy-3- f 5-[4-(trifluormethoxy)phenyl]-1H,2H,3H,4H,5H,6H-pyrrolo[3,4-c]pyrrol-2-yl}butanamid,
*N-*(4-Chlorphenyl)-4-hydroxy-3-{2-[4-(trifluormethoxy)phenyl]-6-oxa-2,9-diazaspiro[4.5]decan-9-yl}butanamid,
*N*-(4-Chlorphenyl)-4-hydroxy-3-(3-{1-[4-(trifluormethoxy)phenyl]azetidin-3-yl}piperidin-1-yl)butanamid,
N (4-Chlorphenyl)-4-hydroxy-3- f 6-[4-(trifluormethoxy)phenyl]-2,6-diazaspiro[3.4]octan-2-yl}butanamid,
N (4-Chlorphenyl)-4-hydroxy-3- f 1-[4-(trifluormethoxy)phenyl]-octahydro-1H-pyrrolo[3,2-b]pyridin-4-yl}butanamid,
N (4-Chlorphenyl)-4-hydroxy-3- f 5-[4-(trifluormethoxy)phenyl]-octahydropyrrolo[3,4-c]pyrrol-2-yl}butanamid,
*N*-(4-Chlorphenyl)-4-hydroxy-3-{8-[4-(trifluormethoxy)phenyl]-2,8-diazaspiro[5.5]undecan-2-yl}butanamid,
*N-*[(Dimethylcarbamoyl)methyl]-4-hydroxy-3-{2-[4-(trifluormethoxy)phenyl]-6-oxa-2,9-diazaspiro[4.5]decan-9-yl }butanamid,
N [2-(Dimethylcarbamoyl)ethyl]-4-hydroxy-3- f 2-[4-(trifluormethoxy)phenyl]-6-oxa-2,9-diazaspiro[4.5]decan-9-yl }butanamid,
5-(4-Hydroxy-3-{2-[4-(trifluormethoxy)phenyl]-6-oxa-2,9-diazaspiro[4.5]decan-9-yl}butanamido)-1H-pyrazol-3-carboxamid,
4-Hydroxy-N (2,2,2-trifluorethyl)-3- f 2-[4-(trifluormethoxy)phenyl]-6-oxa-2,9-diazaspiro[4.5]decan-9-yl}butanamid,
4-Hydroxy-3-{2-[4-(trifluormethoxy)phenyl]-6-oxa-2,9-diazaspiro[4.5]decan-9-yl}-N-[4-(trifluormethyl)phenyl]butanamid,
*N*-(5-Chlorpyridin-2-yl)-4-hydroxy-3-{2-[4-(trifluormethoxy)phenyl]-6-oxa-2,9-diazaspiro[4.5]decan-9-yl}butanamid,
4-Hydroxy-N (3-methoxyphenyl)-3- f 2-[4-(trifluormethoxy)phenyl]-6-oxa-2,9-diazaspiro[4.5]decan-9-yl}butanamid,
A-(4-Acetamidophenyl)-4-hydroxy-3-{2-[4-(trifluormethoxy)phenyl]-6-oxa-2,9-diazaspiro[4.5]decan-9-yl}butanamid,
A-(3-Acetamidophenyl)-4-hydroxy-3-{2-[4-(trifluormethoxy)phenyl]-6-oxa-2,9-diazaspiro[4.5]decan-9-yl}butanamid,
*N*-(4-Cyanophenyl)-4-hydroxy-3-{2-[4-(trifluormethoxy)phenyl]-6-oxa-2,9-diazaspiro[4.5]decan-9-yl}butanamid,
*N*-(2-Chlorphenyl)-4-hydroxy-3-{2-[4-(trifluormethoxy)phenyl]-6-oxa-2,9-diazaspiro[4.5]decan-9-yl}butanamid,
*N*-(3-Chlorphenyl)-4-hydroxy-3-{2-[4-(trifluormethoxy)phenyl]-6-oxa-2,9-diazaspiro[4.5]decan-9-yl}butanamid,
*N*-(4-Fluorphenyl)-4-hydroxy-3-{2-[4-(trifluormethoxy)phenyl]-6-oxa-2,9-diazaspiro[4.5]decan-9-yl}butanamid,
1- (4-Hydroxy-3-{2-[4-(trifluormethoxy)phenyl]-6-oxa-2,9-diazaspiro[4.5]decan-9-yl }butanoyl)imidazolidin-4-on,
4-Hydroxy-*N*-(prop-2-en-1-yl)-3-{2-[4-(trifluormethoxy)phenyl]-6-oxa-2,9-diazaspiro[4.5]decan-9-yl}butanamid,
4-Hydroxy-1-(pyrazolidin-1-yl)-3-{2-[4-(trifluormethoxy)phenyl]-6-oxa-2,9-diazaspiro[4.5]decan-9-yl}butan-1-on,
4-Hydroxy-1-(3-hydroxypyrrolidin-1-yl)-3-{2-[4-(trifluormethoxy)phenyl]-6-oxa-2,9-diazaspiro[4.5]decan-9-yl}butan-1-on,
2-[1-(4-Hydroxy-3-{5-[4-(trifluormethoxy)phenyl]-1H,2H,3H,4H,5H,6H-pyrrolo[3,4-c]pyrrol-2-yl}butanoyl)pyrrolidin-3-yl]acetonitril,
*N*-(3-Chlorphenyl)-4-hydroxy-3-{5-[4-(trifluormethoxy)phenyl]-1H,2H,3H,4H,5H,6H-pyrrolo[3,4-c]pyrrol-2-yl}butanamid,
4-Hydroxy-*N-*(pyridin-3-yl)-3-{5-[4-(trifluormethoxy)phenyl]-1H,2H,3H,4H,5H,6H-pyrrolo[3,4-c]pyrrol-2-yl}butanamid,
4-Hydroxy-*N*-(1,2-thiazol-5-yl)-3-{5-[4-(trifluormethoxy)phenyl]-1H,2H,3H,4H,5H,6H-pyrrolo[3,4-c]pyrrol-2-yl}butanamid,
*N*-[(Dimethylcarbamoyl)methyl]-4-hydroxy-3-{5-[4-(trifluormethoxy)phenyl]-1H,2H,3H,4H,5H,6H-pyrrolo[3,4-c]pyrrol-2-yl}butanamid,
*N*-(2-Chlorphenyl)-4-hydroxy-3-{5-[4-(trifluormethoxy)phenyl]-1H,2H,3H,4H,5H,6H-pyrrolo[3,4-c]pyrrol-2-yl}butanamid,
4-Hydroxy-*N-*(1H-pyrazol-1-yl)-3-{2-[4-(trifluormethoxy)phenyl]-6-oxa-2,9-diazaspiro[4.5]decan-9-yl}butanamid,
*N*-(4-Cy anophenyl)-4-hy droxy-3 - { 5 - [4-(trifluormethoxy)phenyl]-1H,2H,3H,4H,5H,6H-pyrrolo[3,4-c]pyrrol-2-yl}butanamid,
4-Hydroxy-1-{1H,4H,5H,6H,7H-pyrazolo[4,3-c]pyridin-5-yl}-3-{5-[4-(trifluormethoxy)phenyl]-1H,2H,3H,4H,5H,6H-pyrrolo[3,4-c]pyrrol-2-yl}butan-1-on,
4-Hydroxy-*N*[(1r,4r)-4-cyanocyclohexyl]-3-{2-[4-(trifluormethoxy)phenyl]-6-oxa-2,9-diazaspiro[4.5]decan-9-yl }butanamid,
*N*-(Carbamoylamino)-4-hydroxy-3-{2-[4-(trifluormethoxy)phenyl]-6-oxa-2,9-diazaspiro[4.5]decan-9-yl}butanamid,
4-Hydroxy-*N*-(pyridin-2-yl)-3-{2-[4-(trifluormethoxy)phenyl]-6-oxa-2,9-diazaspiro[4.5]decan-9-yl}butanamid,
4-Hydroxy-*N*-(pyridin-3-yl)-3-{2-[4-(trifluormethoxy)phenyl]-6-oxa-2,9-diazaspiro[4.5]decan-9-yl}butanamid,
4-Hydroxy-3-{5-[4-(trifluormethoxy)phenyl]-1H,2H,3H,4H,5H,6H-pyrrolo[3,4-c]pyrrol-2-yl}-*N*-(2,4,6-trimethylphenyl)butanamid,
4-Hydroxy-N (4-methansulfonylphenyl)-3- f 2-[4-(trifluormethoxy)phenyl]-6-oxa-2,9-diazaspiro[4.5]decan-9-yl}butanamid,
4-Hydroxy-1-{1H,4H,5H,6H,7H-pyrrolo[3,2-c]pyridin-5-yl}-3-{5-[4-(trifluormethoxy)phenyl]-1H,2H,3H,4H,5H,6H-pyrrolo[3,4-c]pyrrol-2-yl}butan-1-on; oder
ein pharmazeutisch akzeptables Salz oder Solvat davon.

14. Eine Verbindung von einem der Ansprüche 1 bis 13 zur Verwendung in einem Verfahren zur Behandlung von Diabetes oder Prädiabetes, wie etwa Diabetes Typ 2, bei einem Subjekt, das dies benötigt.

15. Eine pharmazeutische Zusammensetzung, die die Verbindung von einem der Ansprüche 1 bis 13 und wahlweise ein pharmazeutisch akzeptables Additiv umfasst.

## Revendications

1. Un composé de formule générale (I) 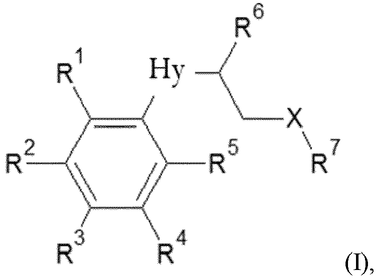 dans laquelle
R¹-R⁵ sont choisis indépendamment dans le groupe constitué par l'hydrogène, halogène, CN, NO₂, CF₃, ammonium quaternaire, COOH, alkyle en COO-C₁₋₆, OCF₃, SCF₃, alkyle en SO₃-C₁₋₆, à condition qu'au moins l'un de R¹-R⁵ ne soit pas l'hydrogène,
R⁶ est choisi parmi C₁₋₃-R⁸, dans lequel R⁸ est choisi parmi H, OH, SH et NH₂,
R⁷ est sélectionné dans le groupe constitué de a) aryle substitué par un ou plusieurs groupes choisis parmi hydrogène, halogène, CN, alkyle en C₁₋₃, NO₂, CONH₂, alkyle en NHCO-C₁₋₃, CF₃, ammonium tertiaire, COOH, alkyle en COO-C₁₋₆, alkyle en OC₁₋₃, OCF₃, SCF₃, alkyle en SO₂-C₁₋₃, alkyle en SO₃-C₁₋₃, à condition qu'au moins l'un desdits groupes ne soit pas hydrogène ; b) pyridyle éventuellement substitué par un ou plusieurs groupes choisis parmi halogène, CN, alkyle en C₁₋₃, NO₂, CONH₂, NHCO-alkyle en C₁₋₃, CF₃, ammonium tertiaire, COOH, COO-alkyle en C₁₋₆, OC-alkyle en C₁₋₃, OCF₃, SCF₃, alkyle en SO₂-C₁₋₃, alkyle en SO₃-C₁₋₃ ; c) pyrrolidinyle éventuellement substitué par un ou plusieurs groupes choisis parmi halogène, CN, alkyle en C₁₋₃, NO₂, CONH₂, NHCO-alkyle en C₁₋₃, CF₃, ammonium tertiaire, COOH, COO-alkyle en C₁₋₆, OC-alkyle en C₁₋₃, OCF₃, SCF₃, alkyle en SO₂-C₁₋₃, alkyle en SO₃-C₁₋₃ ; d) pyrazolyle éventuellement substitué par un ou plusieurs groupes choisis parmi halogène, CN, alkyle en C₁₋₃, NO₂, CONH₂, alkyle en NHCO-C₁₋₃, CF₃, ammonium tertiaire, COOH, alkyle en COO-C₁₋₆, alkyle en OC₁₋₃, OCF₃, SCF₃, alkyle en SO₂-C₁₋₃, alkyle en SO₃-C₁₋₃ ; e) thiazolyle éventuellement substitué par un ou plusieurs groupes choisis parmi halogène, CN, alkyle en C₁₋₃, NO₂, CONH₂, alkyle en NHCO-C₁₋₃, CF₃, ammonium tertiaire, COOH, alkyle en COO-C₁₋₆, alkyle en OC₁₋₃, OCF₃, SCF₃, alkyle en SO₂-C₁₋₃, alkyle en SO₃-C₁₋₃ ; f) cycloalkyle en C₅₋₇ éventuellement substitué par un ou plusieurs groupes choisis parmi halogène, CN, alkyle en C₁₋₃, NO₂, CONH₂, alkyle en NHCO-C₁₋₃, CF₃, ammonium tertiaire, COOH, alkyle en COO-C₁₋₆, alkyle en OC₁₋₃, OCF₃, SCF₃, alkyle en SO₂-C₁₋₃, alkyle en SO₃-C₁₋₃ ; g) NHCONH₂ ; h) alkyle en C₁₋₃ éventuellement substitué par un groupe choisi parmi CONH₂, CON(CH₃)₂, CF₃ et alcène en C₂₋₄ ;
X est -CONH- ou -NHCO-, ou
X-R⁷ pris ensemble est CONR'R", dans lequel R' et R" forment ensemble avec l'azote un cycle monohétérocyclique ou un groupe bihétérocyclique choisi parmi a) un cycle monohétérocyclique ayant 4-7 atomes cycliques dans lequel 1-2 est choisi parmi les azotes et 0-2 parmi les oxygènes et les atomes cycliques restants sont le carbone, et éventuellement substitué par un groupe choisi parmi oxo, OH, alkyle en C₁₋₃-CN, ou b) un groupe bihétérocyclique ayant 7-10 atomes cycliques dans lequel 1-4 est choisi parmi les azotes et les atomes cycliques restants sont le carbone, et éventuellement substitué par un groupe choisi parmi oxo, OH, alkyle en C₁₋₃-CN ;
Hy est un système hétérocyclique non aromatique choisi parmi b) un groupe bihétérocyclique ayant un premier et un second cycle hétérocyclique qui sont fusionnés, le groupe bihétérocyclique ayant de 6 à 12 atomes de cycle dans lequel 2 à 4 atomes de cycle sont N, 0 à 2 atomes de cycle est O, 0 à 2 atomes de cycle est S, et les atomes de cycle restants sont du carbone, dans lequel le premier cycle hétérocyclique est attaché au cycle phényle ayant les substituants R¹-R⁵ de formule I, et le second cycle hétérocyclique est attaché à l'atome de carbone lié à R⁶ et CH₂-X-R⁷ de formule I, c) un groupe spirohétérocyclique ayant un premier et un second cycle hétérocyclique qui sont connectés et partagent un atome de carbone, le groupe spirohétérocyclique ayant de 6 à 12 atomes de cycle dans lequel 2 à 4 atomes de cycle sont N, 0 à 2 atomes de cycle est O, 0 à 2 atomes de cycle est S, et les atomes de cycle restants sont du carbone, dans lequel le premier cycle hétérocyclique est attaché au cycle phényle ayant les substituants R¹-R⁵ de formule I, et le second cycle hétérocyclique est attaché à l'atome de carbone lié à R⁶ et CH₂-X-R⁷ de formule I, ou d) un premier et un second cycles monohétérocycliques reliés par une liaison, dans lesquels chaque cycle monohétérocyclique est choisi indépendamment parmi un cycle monohétérocyclique ayant de 4 à 7 atomes de cycle, dans lequel 1 à 3 atomes de cycle sont N, 0 à 1 atome de cycle est O et 0 à 1 atome de cycle est S, et les atomes de cycle restants sont du carbone, dans lequel le premier cycle monohétérocyclique est attaché au cycle phényle ayant les substituants R¹-R⁵ de formule I, et le second cycle monohétérocyclique est attaché à l'atome de carbone lié à R⁶ et CH₂-X-R⁷ de formule I ; ou un sel ou solvate pharmaceutiquement acceptable de celui-ci ;
excluant le 3-(4-hydroxy-3-{2-[4-(trifluorométhoxy)phényl]-6-oxa-2,9-diazaspiro[4.5]décan-9-yl}butanamido)pyrrolidine-1-carboxamide.

2. Le composé de la revendication 1, dans lequel R¹ est choisi parmi halogène, CN, NO₂, CF₃, ammonium quaternaire, COOH, alkyle en COO-C₁₋₆, OCF₃, SCF₃, alkyle en SO₃-C₁₋₆ ; et R²-R⁵ sont hydrogène.

3. Le composé de la revendication 1 ou 2, dans lequel R² est choisi parmi halogène, CN, NO₂, CF₃, ammonium quaternaire, COOH, alkyle en COO-C₁₋₆, OCF₃, SCF₃, alkyle en SO₃-C₁₋₆ ; et R¹, R³-R⁵ sont hydrogène.

4. Le composé de l'une quelconque des revendications 1 à 3, dans lequel R³ est choisi parmi halogène, CN, NO₂, CF₃, ammonium quaternaire, COOH, alkyle en COO-C₁₋₆, OCF₃, SCF₃, alkyle en SO₃-C₁₋₆ ; et R¹, R², R⁴ à R⁵ sont hydrogène.

5. Le composé de l'une quelconque des revendications 1 à 4, dans lequel R⁴ est choisi parmi halogène, CN, NO₂, CF₃, ammonium quaternaire, COOH, alkyle en COO-C₁₋₆, OCF₃, SCF₃, alkyle en SO₃-C₁₋₆ ; et R¹- R³, R⁵ sont hydrogène.

6. Le composé de l'une quelconque des revendications 1 à 5, dans lequel R⁵ est choisi parmi halogène, CN, NO₂, CF₃, ammonium quaternaire, COOH, alkyle en COO-C₁₋₆, OCF₃, SCF₃, alkyle en SO₃-C₁₋₆ ; et R¹-R⁴ sont hydrogène.

7. Le composé de l'une quelconque des revendications 1 à 6, dans lequel R⁶ est choisi parmi C₁₋₃-R⁸, dans lequel R⁸ est OH.

8. Le composé de l'une quelconque des revendications 1 à 7, dans lequel R⁷ est choisi dans le groupe constitué par a) un phényle substitué par un ou plusieurs groupes choisis parmi hydrogène, halogène, CN, alkyle en C₁₋₃, NO₂, CONH₂, alkyle en NHCO-C₁₋₃, CF₃, ammonium tertiaire, COOH, alkyle en COO-C₁₋₆, alkyle en OC₁₋₃, OCF₃, SCF₃, alkyle en SO₂-C₁₋₃, alkyle en SO₃-C₁₋₃, à condition qu'au moins l'un desdits groupes ne soit pas hydrogène ; typiquement, phényle substitué par un groupe choisi parmi halogène, CF₃, OCH₃, NHCOCH₃, CN, CH₃ et SO₂CH₃, b) un pyridyle éventuellement substitué par un ou plusieurs halogènes ; c) un pyrrolidinyle substitué par un ou plusieurs CONH₂ ; d) un pyrazolyle éventuellement substitué par un ou plusieurs CONH₂ ; e) un thiazolyle ; f) un cyclohexyle substitué par un ou plusieurs alkyle CN ; g) un NHCONH₂ ; et h) un alkyle en C₁₋₂ substitué par un groupe choisi parmi CON(CH₃)₂, CF₃ et alcène en C₂.

9. Le composé de l'une quelconque des revendications 1 à 7, dans lequel X-R⁷ pris ensemble est CONR'R", dans lequel R' et R" forment ensemble avec l'azote un cycle monohétérocyclique ou un groupe bihétérocyclique choisi parmi a) un groupe monohétérocyclique ayant 5 atomes de cycle, dans lequel 1-2 est choisi parmi des atomes d'azote et les atomes de cycle restants sont du carbone, et éventuellement substitué par un groupe choisi parmi oxo, OH, CH₂-CN, ou b) un groupe bihétérocyclique ayant 9-10 atomes de cycle, dans lequel 1-3 est choisi parmi des atomes d'azote et les atomes de cycle restants sont du carbone.

10. Le composé de l'une quelconque des revendications 1 à 9, dans lequel Hy est un système cyclique non aromatique choisi parmi b) dans lequel le groupe bihétérocyclique a de 8 à 11 atomes cycliques, où 1-2 est N, 0-1 est O et les atomes cycliques restants sont du carbone.

11. Le composé de l'une quelconque des revendications 1 à 9, dans lequel Hy est un système cyclique non aromatique choisi parmi c) dans lequel le groupe spirohétérocyclique a de 8 à 11 atomes cycliques, dans lequel 2 à 3 atomes cycliques sont N, 0 à 1 atome cyclique est O, et les atomes cycliques restants sont du carbone.

12. Le composé de l'une quelconque des revendications 1 à 9, dans lequel Hy est un système cyclique non aromatique choisi parmi d) dans lequel les premier et second cycles monohétérocycliques sont reliés par une liaison, et le premier cycle monohétérocyclique a de 4 à 6 atomes de cycle, dans lequel 1-2 est N et 0-1 est O, et les atomes de cycle restants sont du carbone, et le second cycle monohétérocyclique a de 4 à 6 atomes de cycle, dans lequel 1-2 est N et 0-1 est O, et les atomes de cycle restants sont du carbone.

13. Le composé de la revendication 1, choisi parmi l'un quelconque parmi
N (4-Chlorophényl)-4-hydroxy-3- f 5-[4-(trifluorométhoxy)phényl]-1H,2H,3H,4H,5H,6H-pyrrolo[3,4-c]pyrrol-2-yl}butanamide,
N (4-Chlorophényl)-4-hydroxy-3- f 2-[4-(trifluorométhoxy)phényl]-6-oxa-2,9-diazaspiro[4.5]décan-9-yl}butanamide,
*-* (4-Chlorophényl)-4-hydroxy-3-(3-{1-[4-(trifluorométhoxy)phényl]azétidin-3-yl}pipéridin-1-yl)butanamide,
N (4-Chlorophényl)-4-hydroxy-3- f 6-[4-(trifluorométhoxy)phényl]-2,6-diazaspiro[3.4]octan-2-yl}butanamide,
N (4-Chlorophényl)-4-hydroxy-3- f 1-[4-(trifluorométhoxy)phényl]-octahydro-1H-pyrrolo[3,2-b]pyridin-4-yl}butanamide,
N (4-Chlorophényl)-4-hydroxy-3- f 5-[4-(trifluorométhoxy)phényl]-octahydropyrrolo[3,4-c]pyrrol-2-yl}butanamide,
*N*-(4-Chlorophényl)-4-hydroxy-3-{8-[4-(trifluorométhoxy)phényl]-2,8-diazaspiro[5.5]undécan-2-yl}butanamide,
N [(Diméthylcarbamoyl)méthyl]-4-hydroxy-3- f 2-[4-(trifluorométhoxy)phényl]-6-oxa-2,9-diazaspiro[4.5]décan-9-yl}butanamide,
N [2-(Diméthylcarbamoyl)éthyl]-4-hydroxy-3- f 2-[4-(trifluorométhoxy)phényl]-6-oxa-2,9-diazaspiro[4.5]décan-9-yl}butanamide,
5-(4-Hydroxy-3-{2-[4-(trifluorométhoxy)phényl]-6-oxa-2,9-diazaspiro[4.5]décan-9-yl}butanamido)-1H-pyrazole-3-carboxamide,
4-Hydroxy-N- (2,2,2-trifluoroéthyl)-3-{2-[4-(trifluorométhoxy)phényl] -6-oxa-2,9-diazaspiro[4,5]décan-9-yl}butanamide,
4-Hydroxy-3-{2-[4-(trifluorométhoxy)phényl]-6-oxa-2,9-diazaspiro[4.5]décan-9-yl}-N-[4-(trifluorométhyl)phényl]butanamide,
N (5-Chloropyridin-2-yl)-4-hydroxy-3- f 2-[4-(trifluorométhoxy)phényl]-6-oxa-2,9-diazaspiro[4.5]décan-9-yl}butanamide,
4-Hydroxy-N- (3-méthoxyphényl)-3-{2-[4-(trifluorométhoxy)phényl] -6-oxa-2,9-diazaspiro[4.5]décan-9-yl}butanamide,
N (4-Acétamidophényl)-4-hydroxy-3- f 2-[4-(trifluorométhoxy)phényl]-6-oxa-2,9-diazaspiro[4.5]décan-9-yl}butanamide,
N (3-Acétamidophényl)-4-hydroxy-3- f 2-[4-(trifluorométhoxy)phényl]-6-oxa-2,9-diazaspiro[4.5]décan-9-yl}butanamide,
*N*-(4-Cyanophényl)-4-hydroxy-3-{2-[4-(trifluorométhoxy)phényl]-6-oxa-2,9-diazaspiro[4.5]décan-9-yl}butanamide,
N (2-Chlorophényl)-4-hydroxy-3- f 2-[4-(trifluorométhoxy)phényl]-6-oxa-2,9-diazaspiro[4.5]décan-9-yl}butanamide,
N (3-Chlorophényl)-4-hydroxy-3- f 2-[4-(trifluorométhoxy)phényl]-6-oxa-2,9-diazaspiro[4.5]décan-9-yl}butanamide,
N (4-Fluorophényl)-4-hydroxy-3- f 2-[4-(trifluorométhoxy)phényl]-6-oxa-2,9-diazaspiro[4.5]décan-9-yl}butanamide,
1- (4-Hydroxy-3-{2-[4-(trifluorométhoxy)phényl]-6-oxa-2,9-diazaspiro[4.5]décan-9-yl }butanoyl)imidazolidin-4-one,
4-Hydroxy-N- (prop-2-én-1-yl)-3-{2-[4-(trifluorométhoxy)phényl] -6-oxa-2,9-diazaspiro[4.5]décan-9-yl}butanamide,
4-Hydroxy-1-(pyrazolidin-1-yl)-3-{2-[4-(trifluorométhoxy)phényl]-6-oxa-2,9-diazaspiro[4.5]décan-9-yl}butan-1-one,
4-Hydroxy-1-(3-hydroxypyrrolidin-1-yl)-3-{2-[4-(trifluorométhoxy)phényl]-6-oxa-2,9-diazaspiro[4.5]décan-9-yl }butan-1-one,
2-[1-(4-Hydroxy-3-{5-[4-(trifluorométhoxy)phényl]-1H,2H,3H,4H,5H,6H-pyrrolo[3,4-c]pyrrol-2-yl }butanoyl)pyrrolidin-3-yl] acétonitrile,
N-(3 -chlorophényl)-4-hydroxy-3 - { 5 - [4-(trifluorométhoxy)phényl] - 1H,2H,3H,4H,5H,6H-pyrrolo[3,4-c]pyrrol-2-yl}butanamide,
4-Hydroxy-N-(pyridin-3-yl)-3-{5-[4-(trifluorométhoxy)phényl]-1H,2H,3H,4H,5H,6H-pyrrolo[3,4-c]pyrrol-2-yl}butanamide,
4-Hydroxy-N-(1,2-thiazol-5-yl)-3-{5-[4-(trifluorométhoxy(phényl]-1H,2H,3H,4H,5H,6H-pyrrolo[3,4-c]pyrrol-2-yl}butanamide,
*N*-[(Diméthylcarbamoyl)méthyl]-4-hydroxy-3-{5-[4-(trifluorométhoxy)phényl]-1H,2H,3H,4H,5H,6H-pyrrolo[3,4-c]pyrrol-2-yl}butanamide,
*N*-(2-Chlorophényl)-4-hydroxy-3-{5-[4-(trifluorométhoxy)phényl]-1H,2H,3H,4H,5H,6H-pyrrolo[3,4-c]pyrrol-2-yl}butanamide,
4-Hydroxy-N-(1H-pyrazol-1-yl)-3- f 2-[4-(trifluorométhoxy)phényl] -6-oxa-2,9-diazaspiro[4.5]décan-9-yl}butanamide,
*N-*(4-Cyanophényl)-4-hydroxy-3-{5-[4-(trifluorométhoxy)phényl]-1H,2H,3H,4H,5H,6H-pyrrolo[3,4-c]pyrrol-2-yl}butanamide,
4-Hydroxy-1-{1H,4H,SH,6H,7H-pyrazolo[4,3-c]pyridin-5-yl}-3-{5-[4-(trifluorométhoxy)phényl]-1H,2H,3H,4H,5H,6H-pyrrolo[3,4-c]pyrrol-2-yl}butan-1-one,
4-Hydroxy-N-[(1r,4r) -4-cyanocyclohexyl]-3-{2-[4-(trifluorométhoxy)phényl]-6-oxa-2,9-diazaspiro[4.5]décan-9-yl}butanamide,
*N*-(Carbamoylamino)-4-hydroxy-3-{2-[4-(trifluorométhoxy)phényl]-6-oxa-2,9-diazaspiro[4.5]décan-9-yl}butanamide,
4-Hydroxy-N- (pyridin-2-yl)-3-{2-[4-(trifluorométhoxy)phényl] -6-oxa-2,9-diazaspiro[4.5]décan-9-yl}butanamide,
4-Hydroxy-N- (pyridin-3-yl)-3-{2-[4-(trifluorométhoxy)phényl] -6-oxa-2,9-diazaspiro[4.5]décan-9-yl}butanamide,
4-Hydroxy-3-{5-[4-(trifluorométhoxy)phényl]-1H,2H,3H,4H,5H,6H-pyrrolo[3,4-c]pyrrol-2-yl}-*N*-(2,4,6-triméthylphényl)butanamide,
4-Hydroxy-*N*-(4-méthanesulfonylphényl)-3-{2-[4-(trifluorométhoxy)phényl] -6-oxa-2,9-diazaspiro[4.5]décan-9-yl}butanamide,
4-Hydroxy-1-{1H,4H,5H,6H,7H-pyrrolo[3,2-c]pyridin-5-yl}-3-{5-[4-(trifluorométhoxy)phényl]-1H,2H,3H,4H,5H,6H-pyrrolo[3,4-c]pyrrol-2-yl}butan-1-one ; ou
un sel ou un solvat pharmaceutiquement acceptable de celui-ci.

14. Un composé de l'une quelconque des revendications 1 à 13 pour une utilisation dans une méthode de traitement du diabète ou du pré-diabète, tel que le diabète de type 2, chez un sujet en ayant besoin.

15. Une composition pharmaceutique comprenant le composé de l'une quelconque des revendications 1 à 13 et éventuellement un additif pharmaceutiquement acceptable.
